# EUROPEAN PATENT SPECIFICATION

(11) **EP 2 440 546 B1**
(45) Date of publication and mention of the grant of the patent: **28.12.2022**
(21) Application number: 10728369.9
(22) Date of filing: 14.06.2010
(51) Int. Cl.: C07D 215/46, C07D 215/42, A61P 39/06, A61P 35/00, A61P 31/18, C07D 213/74, A61P 17/00, A61P 3/10, C07D 401/12, C07D 403/12, C07D 241/44, C07D 215/38

(54) **COMPOUNDS USEFUL FOR TREATING PREMATURE AGING AND IN PARTICULAR PROGERIA**
VERBINDUNGEN ZUR BEHANDLUNG VORZEITIGER ALTERUNG UND INSBESONDERES PROGERIA
COMPOSES UTILES POUR LE TRAITEMENT DE VIEILLISSEMENT PRÉMATURÉ ET EN PARTICULIER PROGERIA

(30) Priority: 12.06.2009 EP 09162630; 12.06.2009 US 186552 P; 12.06.2009 EP 09305540; 12.06.2009 US 186544 P
(43) Date of publication of application: 18.04.2012
(73) Proprietor: ABIVAX, 75008 Paris (FR); Centre National de la Recherche Scientifique (C.N.R.S.), 75794 Paris Cedex 16 (FR); Institut Curie, 75248 Paris Cedex 05 (FR); UNIVERSITE DE MONTPELLIER, 34090 Montpellier (FR)
(72) Inventor: TAZI, Jamal, F-34380 Clapiers (FR); MAHUTEAU, Florence, F-78470 Saint Remy Les Chevreuse (FR); NAJMAN, Romain, F-94240 L'Hay-les-Roses (FR); SCHERRER, Didier, 34170 Castelnau le lez (FR); SANTO, Julien, 34090 Montpellier (FR)
(74) Representative: Nony
(86) International application number: PCT/IB2010/052652
(87) International publication number: WO 2010/143170

(56) References cited:
- WO-A2-2008/101935
- VULLIAMY ET AL.: "Mutations in the telomerase component NHP2 cause the premature ageing syndrome dyskeratosis congenita", PROCEEDINGS OF THE NATIONAL ACADEMY OF SCIENCES OF THE UNITED STATES OF AMERICA, vol. 105, no. 23, 10 June 2008 (2008-06-10), pages 8073-8078, XP002612935,
- "Progeria: A new kind of laminopathy", , 25 September 2003 (2003-09-25), XP002612936, Retrieved from the Internet: URL:http://www.med.uni-magdeburg.de/proger ia/Docs/Progeria%20Symposium%20Magdeburg.p df [retrieved on 2003-09-25]

## Description

### FIELD OF THE INVENTION

The present invention is generally dedicated to the use of compounds for the manufacture of compositions useful to treat diseases related to premature aging. The compounds and compositions containing them and compositions according to the invention may in particular be used to inhibit, prevent and/or treat Progeria.

### BACKGROUND OF THE INVENTION

The present invention focuses on premature aging. Said premature aging may be encountered in patients suffering from various diseases and in particular from the Hutchinson-Gilford progeria syndrome (HGPS) and from the HIV infection.

Hutchinson-Gilford progeria syndrome (HGPS) is a rare genetic disorder phenotypically characterized by many features of premature aging. It is clinically characterized by postnatal growth retardation, midface hypoplasia, micrognathia, premature atherosclerosis, absence of subcutaneous fat, alopecia and generalized osteodysplasia (Khalifa, 1989 - Hutchinson-Gilford progeria syndrome: report of a Libyan family and evidence of autosomal recessive inheritance. Clin. Genet. 35, 125-132.). At birth, the appearance of patients is generally normal, but by 1 year of age patients show severe growth retardation, balding and sclerodermatous skin changes. They average about 1m in height and usually weigh less than 15 kg even as teenagers. The age at death ranges from 7 to 28 years, with a median of 13.4 years. Over 80% of deaths are due to heart attacks or congestive heart failure.

Premature aging syndrome has been observed in patients suffering from HIV infections. One mechanical pathway underlying said premature aging could be associated, as for the HGPS and as exposed beneath, with an aberrant splicing of the nuclear lamin A gene. Indeed it has recently been hypothesized that protease inhibitors against HIValso block the transformation of prelamin A into lamin A as it turned out in HGPS.

Most of the patients suffering from premature aging carry a heterozygous silent mutation that activates the use of a cryptic 5' splice site in exon 11 of LMNA pre-mRNA. This aberrant splicing event leads to the production of a truncated protein (progerin) with a dominant negative effect which is responsible for the observed phenotype (De Sandre-Giovannoli et al., 2003 - Lamin A truncation in Hutchinson-Gilford progeria. Science 300, 2055 / Pendas et al., 2002a - Defective prelamin A processing and muscular and adipocyte alterations in Zmpste24 metalloproteinase-deficient mice. Nat. Genet.31, 94-99.).

Most of the premature aging syndromes in particular associated withHutchinson-Gilford progeria and HIV infection are due to a recurrent, de novo point mutation in *LMNA* exon 11: c.1824C>T. This mutation is localized in the part of the gene specifically encoding lamin A (De Sandre-Giovannoli *et al.,* 2003 / De Sandre-Giovannoli and Levy, 2006 - Altered splicing in prelamin A-associated premature aging phenotypes. Prog. Mol. Subcell. Biol. 44, 199-232). Its predicted effect is a silent amino acid change at codon 608 (p.G608G). In fact, this sequence variation is not silent as it occurs in a probable exon splicing enhancer. As a result, a cryptic splice site is activated in transcripts issued from the mutated allele, which is located 5 nucleotides upstream of the mutation.

So far, therapeutic approaches have been mainly focused on progerin which is attached to a lipid anchor (a farnesyl lipid anchor). This lipid anchor is attached to progerin by a specific cellular enzyme, protein farnesyltransferase. Experiments in mouse models suggest that farnesyltransferase inhibitors (FTIs) may have beneficial effects in humans with progeria (Fong et al., 2006 - A protein farnesyltransferase inhibitor ameliorates disease in a mouse model of progeria. Science 311, 1621-1623). More recently, Nicolas Levy's team has used a combination of a statin and an aminobisphosphonate to prevent the fixation of the fatty acid to the progerin, and thus reduce its toxicity (Varela et al., 2008 - Combined treatment with statins and aminobisphosphonates extends longevity in a mouse model of human premature aging. Nat. Med. 14, 767-772.).

In WO2006/081444 has been reported a method for reducing at least one cellular defect in a cell from a subject susceptible to a disease or condition characterized by farnesylation on an abnormally farnesylated form of a lamin, comprising administering to the cell a therapeutically effective dose of farnesylstransferase inhibitor.

It has been recently reported in WO2008/003864 the use of a hydroxymethylglutaryl-coenzyme A (HMG-CoA) reductase inhibtor and a farnesyl-pyrophosphate synthase inhibitor, or one of their associated physiologically acceptable salts, in the preparation of a composition, for use in the treatment of human or animal, pathological situations related to the accumulation and/or the persistence of prenylated proteins in cells, such as during progeria, restrictive dermopathy or physiological aging.

In WO 2008/115870 substituted quinoline are described, which are useful for treating cancer.

In US 2008/0161353 other substituted quinoline are disclosed as agents to treat neurological conditions.

In WO2008/101935 compounds for treating genetic diseases resulting from at least one mutation inducing the occurrence of an early termination codon are described.

Vulliamy et al. (Proceedings of the National Academy of Sciences of the United States of America, vol. 105, no.23, 2008, pages 8073-8078) describes the relationship between dyskeratosis congenita, a premature aging syndrome, and bi-allellic mutations in the telomerase component NHP2.The document "Progeria: A new kind of laminopathy" (2003) is a document containing the information disclosed during the First European Symposium in Magdeburg (Germany) on progeria which is part of the family of the laminopathies, all disorders related to an alteration (mutations) of the lamina proteins.

### SUMMARY OF THE INVENTION

It has now been found that derivatives of formula (I) as defined in formula (I) hereinafter are able to interfere with the usage of the cryptic splice and demonstrate efficient inhibition of aberrant splicing leading to progerin production as illustrated in the experimental data herein after and, on the basis of such activity, the compounds are useful in the treatment of premature aging and in particular of progeria.

The present invention therefore relates to compounds of formula (I) as defined below for use as agents for preventing, inhibiting or treating pathological conditions linked with premature aging.

The present invention further relates to some particular derivatives as such, as defined below.

The present invention also provides pharmaceutical compositions comprising at least one of said particular compounds.

### DETAILED DESCRIPTION OF THE INVENTION

The present invention is based on a novel approach based on the inhibition of aberrant splicing leading to progerin production.

The truncated Lamin A protein lacking the last 150 base pairs of exon 11 also called "progerin", acting as a dominant negative mutant, is predicted to be responsible for the characteristic manifestations seen in HGPS patients. Given that similar alteration of lamin A/C splicing was observed in aged individuals, it is proposed here that therapeutic molecules that interfere with the usage of the cryptic splice site will prevent side effects associated with accumulation of progerin during physiological aging. In other words, the compounds according to the present invention prevent usage of the cryptic 5' splice site in exon 11 of LMNA, allowing overcoming deleterious effect associated with progerin.

According to a first aspect, a subject-matter of the present invention relates to a compound of formula (I): 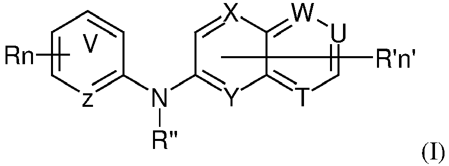 wherein: 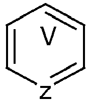
means an aromatic ring wherein V is C or N and when V is N, V is in ortho, meta or para of Z, i.e. forms respectively a pyridazine, a pyrimidine or a pyrazine group,
R independently represent a hydrogen atom, a halogen atom or a group chosen among a -CN group, a hydroxyl group, a -COOR₁ group, a (C₁-C₃)fluoroalkyl group, a (C₁-C₃)fluoroalkoxy group, a -NO₂ group, a -NR₁R₂ group, a (C₁-C₄)alkoxy group, a phenoxy group and a (C₁-C₃)alkyl group, said alkyl being optionally mono-substituted by a hydroxyl group,
R₁ and R₂ are independently a hydrogen atom or a (C₁-C₃)alkyl group,
n is 1, 2 or 3,
n' is 1 or 2,
R' is a hydrogen atom, a halogen atom or a group chosen among a (C₁-C₃)alkyl group, , a hydroxyl group, a -COOR₁ group, a -NO₂ group, a -NR₁R₂ group, a morpholinyl or a morpholino group, a N-methylpiperazinyl group, a (C₁-C₃)fluoroalkyl group, a (C₁-C₄)alkoxy group and a -CN group,
R" is a hydrogen atom or a (C₁-C₄)alkyl group,
- Z is N, V is C, Y is N, X is C, T is C, U is C and W is C,
- Z is C, V is C, Y is N, X is C, T is C, U is C and W is C,
- Z is N, V is C, Y is C, X is N, T is C, U is C and W is C,
- Z is N, V is C, Y is C, X is C, T is C, U is C and W is N,
- Z is N, V is N and is in para of Z, Y is N, X is C, T is C, U is C and W is C
- Z is C, V is N and is in para of Z, Y is C, X is N, T is C, U is C and W is C,
- Z is C, V is N and is in meta of Z and is in para of the bond linked to NR", Y is N, X is C, T is C, U is C and W is C,
- Z is C, V is N and is in meta of Z and is in para of the bond linked to NR", Y is C, X is N, T is C, U is C and W is C,
- Z is C, V is C, Y is C, X is N, T is C, U is C and W is C,
- Z is C, V is C, Y is N, X is N, T is C, U is C and W is C,
- Z is N, V is N and is in meta of Z and in ortho of the bond linked to NR", Y is N, X is C, T is C, U is C and W is C,
- Z is N, V is N and is in para of Z, Y is C, X is C, T is C, U is C and W is N,
- Z is N, V is N and is in para of Z, Y is C, X is N, T is C, U is C and W is C,
- Z is N, V is C, Y is N, X is N, T is C, U is C and W is C,
- Z is N, V is N and is in meta of Z and is in ortho of the bond linked to NR", Y is N, X is N, T is C, U is C and W is C,
- Z is C, V is C, Y is C, X is C, T is N, U is C and W is C, or
- Z is N, V is C, Y is C, X is C, T is N, U is C and W is C,
or anyone of its pharmaceutically acceptable salt,
for use as an agent for preventing, inhibiting or treating pathological conditions linked with premature aging.

The present text describes a compound of formula (Ia-1) 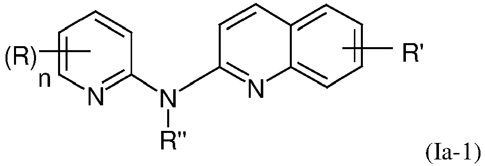 wherein:
R independently represent a hydrogen atom, a halogen atom, or a group chosen among a (C₁-C₃)alkyl group, a -CN group, a -COOH group, a (C₁-C₃)fluoroalkyl group, a (C₁-C₃)fluoroalkoxy group, a -NO₂ group and a (C₁-C₃)alkoxy group,
R" is as defined above and is advantageously a hydrogen atom,
n is 1 or 2, and advantageously 1, and
R' is a hydrogen atom or a group chosen among a (C₁-C₃)alkyl group, a halogen atom, a hydroxy group, a -COOH group and a -CN group,
or one of its pharmaceutically acceptable salt,
for use as an agent for preventing, inhibiting or treating pathological conditions linked with premature aging.

The present text also describes more particularly compounds of formula (Ia'-1) 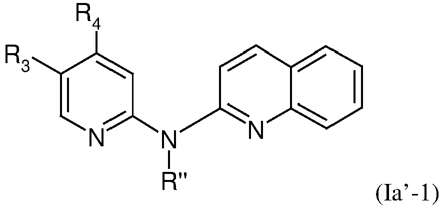 wherein:
at least one of R₃ and R₄ is a hydrogen atom and the other is a hydrogen atom, a -COOH group or a (C₁-C₃)alkyl group, and
R" is as defined above and is advantageously a hydrogen atom,
or one of its pharmaceutically acceptable salt,
for use as an agent for preventing, inhibiting or treating pathological conditions linked with premature aging.

The present text also decribes a compound of formula (Ib-1) 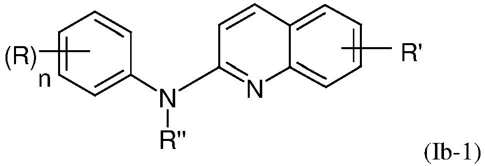 wherein:
R independently represent a hydrogen atom, a halogen atom, or a group chosen among a (C₁-C₃)alkyl group, a -CN group, a -COOH group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group, n is 1 or 2, and advantageously 1,
R' is a hydrogen atom or a group chosen among a (C₁-C₃)alkyl group, a halogen atom, a hydroxy group, a -COOH group and a -CN group, and
R" is as defined above and is advantageously a hydrogen atom,
or one of its pharmaceutically acceptable salt,
for use as an agent for preventing, inhibiting or treating pathological conditions linked with premature aging.

The present text also describes compounds of formula (Ib'-1) 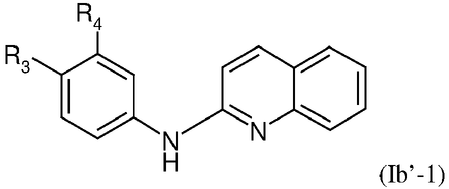 wherein:
at least one of R₃ and R₄ is a hydrogen atom and the other is a (C₁-C₃)fluoroalkoxy group or a (C₁-C₄)alkoxy group,
or one of its pharmaceutically acceptable salt,
for use as an agent for preventing, inhibiting or treating pathological conditions linked with premature aging.

The present text also describes a compound of formula (Ic-1) 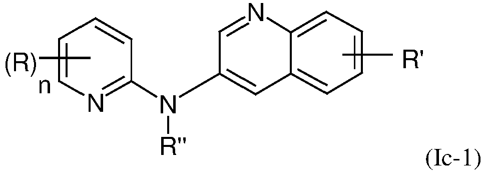 wherein:
R independently represent a hydrogen atom, a halogen atom, or a group chosen among a (C₁-C₃)alkyl group a -CN group, a -COOH group, a (C₁-C₃)fluoroalkyl group, a -NO₂ group and a (C₁-C₃)alkoxy group,
n is 1 or 2, and advantageously 1,
R' is a hydrogen atom or a (C₁-C₃)alkyl group, and in particular is a hydrogen atom, and
R" is as defined above and is advantageously a hydrogen atom,
or one of its pharmaceutically acceptable salt,
for use as an agent for preventing, inhibiting or treating pathological conditions linked with premature aging.

The present text further describesa compound of formula (Ic-1) as defined above, as such
wherein:
R, R' R" and n are as defined above, and
wherein R and R' are not simultaneously a hydrogen atom,
or one of its pharmaceutically acceptable salt.

The present text also describes compounds of formula (Ic'-1): 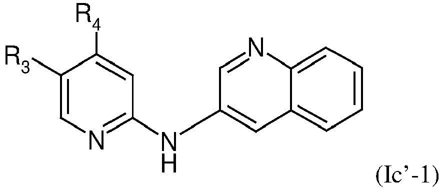 wherein:
at least one of R₃ and R₄ is a hydrogen atom and the other is a (C₁-C₃)alkyl group,
or one of its pharmaceutically acceptable salt,
for use as an agent for preventing, inhibiting or treating pathological conditions linked with premature aging.

The present text also describes a compound of formula (Id-1): 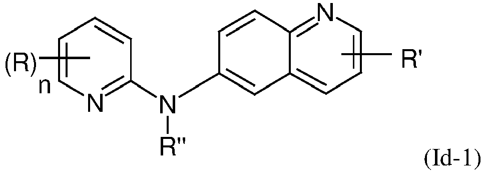 wherein:
R independently represent a hydrogen atom, a halogen atom, or a group chosen among a (C₁-C₃)alkyl group, a -CN group, a -COOH group, a (C₁-C₃)fluoroalkyl group, a (C₁-C₃)fluoroalkoxy group and a -NO₂ group,
n is 1 or 2, and advantageously 1,
R' is a hydrogen atom or a (C₁-C₃)alkyl group, and in particular is a hydrogen atom, and
R" is as defined above and is advantageously a hydrogen atom,
or one of its pharmaceutically acceptable salt,
for use as an agent for preventing, inhibiting or treating pathological conditions linked with premature aging.

The compound of formula (Id-1) as such and as defined above are also described in the present text, with the proviso that when R' is a hydrogen atom, R is different from a -NO₂ group, or one of its pharmaceutically acceptable salt.

The compounds of formulae (Ia-1), (Ib-1), (Ic-1) and (Id-1) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

According to one aspect, the present invention relates to a compound of formula (I) as defined above, wherein Z is N, V is C, Y is N, X is C, T is C, U is C and W is C, for use as an agent for preventing, inhibiting or treating pathological conditions linked with premature aging.

According to another aspect, the present invention relates to a compound of formula (I) as defined above, wherein Z is C, V is C, Y is N, X is C, T is C, U is C and W is C, for use as an agent for preventing, inhibiting or treating pathological conditions linked with premature aging.

According to another aspect, the present invention relates to a compound of formula (I) as defined above, wherein Z is N, V is C, Y is C, X is N, T is C, U is C and W is C, for use as an agent for preventing, inhibiting or treating pathological conditions linked with premature aging.

According to another aspect, the present invention relates to a compound of formula (I) as defined above, wherein Z is N, V is C, Y is C, X is C, T is C, U is C and W is N, for use as an agent for preventing, inhibiting or treating pathological conditions linked with premature aging.

According to another aspect, the present invention relates to a compound of formula (I) as defined above, wherein Z is N, V is N and is in para of Z, Y is N, X is C, T is C, U is C and W is C, for use as an agent for preventing, inhibiting or treating pathological conditions linked with premature aging.

According to another aspect, the present invention relates to a compound of formula (I) as defined above, wherein Z is C, V is C, Y is C, X is N, T is C, U is C and W is C, for use as an agent for preventing, inhibiting or treating pathological conditions linked with premature aging.

According to another aspect, the present invention relates to a compound of formula (I) as defined above, wherein Z is C, V is C, Y is N, X is N, T is C, U is C and W is C, for use as an agent for preventing, inhibiting or treating pathological conditions linked with premature aging.

According to another aspect, the present invention relates to a compound of formula (I) as defined above, wherein Z is N, V is N and is in meta of Z and in ortho of the bond linked to NR", Y is N, X is C, T is C, U is C and W is C, for use as an agent for preventing, inhibiting or treating pathological conditions linked with premature aging.

According to another aspect, the present invention relates to a compound of formula (I) as defined above, wherein Z is N, V is C, Y is N, X is N, T is C, U is C and W is C, for use as an agent for preventing, inhibiting or treating pathological conditions linked with premature aging.

According to another aspect, the present invention relates to a compound of formula (I) as defined above, wherein Z is N, V is N and is in meta of Z and is in ortho of the bond linked to NR", Y is N, X is N, T is C, U is C and W is C, for use as an agent for preventing, inhibiting or treating pathological conditions linked with premature aging.

According to another aspect, the present invention relates to a compound of formula (I) as defined above, wherein Z is N, V is C, Y is C, X is C, T is N, U is C and W is C, for use as an agent for preventing, inhibiting or treating pathological conditions linked with premature aging.

The compounds of the invention may exist in the form of free bases or of addition salts with pharmaceutically acceptable acids.

Suitable physiologically acceptable acid addition salts of compounds of formula (I) include hydrochloride, hydrobromide, tartrate, fumarate, citrate, trifluoroacetate, ascorbate, triflate, mesylate, tosylate, formate, acetate and malate.

The compounds of formula (I) and or salts thereof may form solvates (e.g. hydrates).

In the context of the present invention, the term:
- "halogen" is understood to mean chlorine, fluorine, bromine, or iodine, and in particular denotes chlorine, fluorine or bromine,
- "(C₁-C₃)alkyl" as used herein respectively refers to C₁-C₃ normal, secondary or tertiary saturated hydrocarbon. Examples are, but are not limited to, methyl, ethyl, 1-propyl, 2-propyl,
- "(C₁-C₃)alkoxy" as used herein respectively refers to O-(C₁-C₃)alkyl moiety, wherein alkyl is as defined above. Examples are, but are not limited to, methoxy, ethoxy, 1-propoxy, 2-propoxy,
- "fluoroalkyl group" and "fluoroalkoxy group" refers respectively to alkyl group and alkoxy group as above-defined, said groups being substituted by at least one fluorine atom. Examples are perfluoroalkyl groups, such as trifluoromethyl or perfluoropropyl, and
- "patient" may extend to humans or mammals, such as cats or dogs.

According to one embodiment, the present invention relates to a compound of formula (I) as defined above for use as an agent for preventing, inhibiting or treating pathological conditions linked with premature aging, wherein T is C, and Z, V, Y, X, U and W are as defined above.

According to another embodiment, the present invention relates to a compound of formula (I) as defined above for use as an agent for preventing, inhibiting or treating pathological conditions linked with premature aging, wherein W is C, and Z, V, Y, X, U and T are as defined above.

According to another embodiment, the present invention relates to a compound of formula (I) as defined above for use as an agent for preventing, inhibiting or treating pathological conditions linked with premature aging, wherein Z is N, V is C, U is C, T is C and W, Y and X are as defined above.

According to one preferred aspect, the present invention relates to a compound of formula (I) as defined above, wherein Z is N, V is C, Y is N, X is C, T is C, U is C and W is C, for use as an agent for preventing, inhibiting or treating pathological conditions linked with premature aging.

According to another preferred aspect, the present invention relates to a compound of formula (I) as defined above, wherein Z is C, V is C, Y is N, X is C, T is C, U is C and W is C, for use as an agent for preventing, inhibiting or treating pathological conditions linked with premature aging.

According to another preferred aspect, the present invention relates to a compound of formula (I) as defined above, wherein Z is N, V is C, Y is C, X is N, T is C, U is C and W is C, for use as an agent for preventing, inhibiting or treating pathological conditions linked with premature aging.

According to another preferred aspect, the present invention relates to a compound of formula (I) as defined above, wherein Z is N, V is C, Y is C, X is C, T is C, U is C and W is N, for use as an agent for preventing, inhibiting or treating pathological conditions linked with premature aging.

According to another preferred aspect, the present invention relates to a compound of formula (I) as defined above, wherein Z is N, V is N and is in para of Z, Y is N, X is C, T is C, U is C and W is C, for use as an agent for preventing, inhibiting or treating pathological conditions linked with premature aging.

According to another aspect, the present invention relates to a compound of formula (I) as defined above, wherein Z is C, V is C, Y is C, X is N, T is C, U is C and W is C, for use as an agent for preventing, inhibiting or treating pathological conditions linked with premature aging.

According to another preferred aspect, the present invention relates to a compound of formula (I) as defined above, wherein Z is C, V is C, Y is N, X is N, T is C, U is C and W is C, for use as an agent for preventing, inhibiting or treating pathological conditions linked with premature aging.

According to another preferred aspect, the present invention relates to a compound of formula (I) as defined above, wherein Z is N, V is N and is in meta of Z and is in ortho of the bond linked to NR", Y is N, X is C, T is C, U is C and W is C, for use as an agent for preventing, inhibiting or treating pathological conditions linked with premature aging.

According to another preferred aspect, the present invention relates to a compound of formula (I) as defined above, wherein Z is N, V is C, Y is N, X is N, T is C, U is C and W is C, for use as an agent for preventing, inhibiting or treating pathological conditions linked with premature aging.

According to another preferred aspect, the present invention relates to a compound of formula (I) as defined above, wherein Z is N, V is N and is in meta of Z and is in ortho of the bond linked to NR", Y is N, X is N, T is C, U is C and W is C, for use as an agent for preventing, inhibiting or treating pathological conditions linked with premature aging.

According to another preferred aspect, the present invention relates to a compound of formula (I) as defined above, wherein Z is N, V is C, Y is C, X is C, T is N, U is C and W is C, for use as an agent for preventing, inhibiting or treating pathological conditions linked with premature aging.

According to a particular embodiment, an additional subject-matter of the present invention is a compound of formula (Ia) 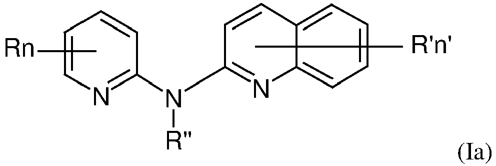 wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a -NO₂ group, a -NR₁R₂ group and a (C₁-C₃)alkoxy group,
R" is as defined above and is advantageously a hydrogen atom,
n is as defined above and is advantageously 1,
n' is as defined above and is advantageously 1,
R' is a hydrogen atom, a halogen atom or a group chosen among a (C₁-C₃)alkyl group, a -NO₂ group, a (C₁-C₃)alkoxy group and a -NR₁R₂ group,
R₁ and R₂ are a hydrogen atom or a (C₁-C₃)alkyl group,
or one of its pharmaceutically acceptable salt,
for use as an agent for preventing, inhibiting or treating pathological conditions linked with premature aging.

According to another particular embodiment, an additional subject-matter of the present invention is a compound of formula (Ib) 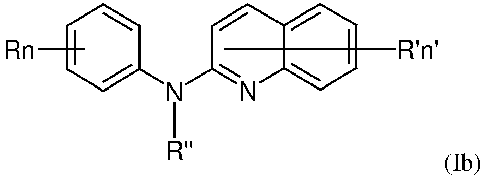 wherein :
R independently represent a hydrogen atom, a halogen atom or a group chosen among a (C₁-C₃)alkyl group, a -NR₁R₂ group, a (C₁-C₃)fluoroalkoxy group, a -NO₂ group, a phenoxy group and a (C₁-C₄)alkoxy group,
R₁ and R₂ are independently a hydrogen atom or a (C₁-C₃)alkyl group,
R" is as defined above and is advantageously a hydrogen atom,
n is as defined above and is preferably 1 or 2,
n' is as defined above and is preferably 1,
R' is a hydrogen atom, a halogen atom or a group chosen among a (C₁-C₃)alkyl group and a (C₁-C₄)alkoxy group,
or one of its pharmaceutically acceptable salt,
for use as an agent for preventing, inhibiting or treating pathological conditions linked with premature aging.

According to another particular embodiment, an additional subject-matter of the present invention is a compound of formula (Ic) 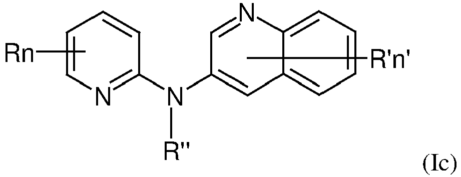 wherein:
R independently represent a hydrogen atom or a group chosen among a (C₁-C₃)alkyl group, a (C₁-C₃)fluoroalkyl group, a -NR₁R₂ group, a -COOR₁ group, a -NO₂ group and a (C₁-C₃)alkoxy group,
R" is as defined above and is advantageously a hydrogen atom,
n is as defined above and is advantageously 1,
n' is as defined above and is advantageously 1,
R' is a hydrogen atom,
R₁ and R₂ are independently a hydrogen atom or a (C₁-C₃)alkyl group,
or one of its pharmaceutically acceptable salt,
for use as an agent for preventing, inhibiting or treating pathological conditions linked with premature aging.

According to another particular embodiment, an additional subject-matter of the present invention is a compound of formula (Id) 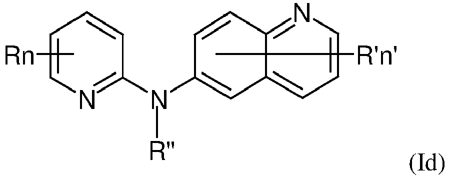 wherein:
R independently represent a hydrogen atom or a group chosen among a (C₁-C₃)alkyl group, a (C₁-C₃)fluoroalkyl group and a (C₁-C₃)alkoxy group,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Ie) 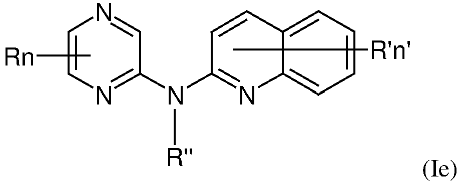 wherein:
R represents a hydrogen atom,
R" is as defined above and is advantageously a hydrogen atom,
n is as defined above and is advantageously 1,
n' is as defined above and is advantageously 1,
R' is a hydrogen atom, a halogen atom or a group chosen among a (C₁-C₃)alkyl group and a (C₁-C₃)alkoxy group,
or one of its pharmaceutically acceptable salt,
for use as an agent for preventing, inhibiting or treating pathological conditions linked with premature aging.

According to another particular embodiment, an additional subject-matter of the present invention is a compound of formula (If) 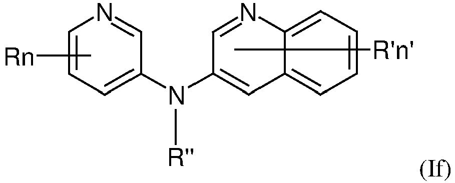 wherein:
R represents a hydrogen atom,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Ig) 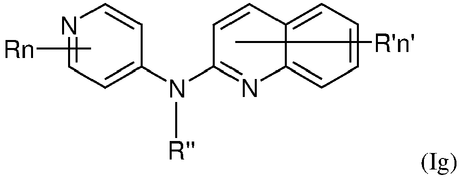 wherein:
R represents a hydrogen atom,
R" is as defined above and is advantageously a hydrogen atom,
n is as defined above and is advantageously 1,
n' is as defined above and is advantageously 1,
R' is a hydrogen atom or a halogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Ih) 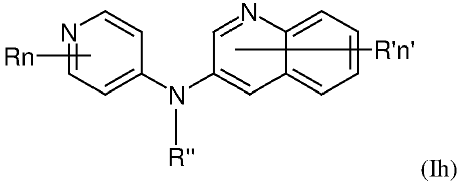 wherein:
R represents a hydrogen atom,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Ii) 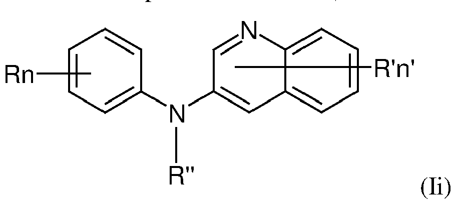 wherein:
R independently represent a hydrogen atom or a group chosen among a (C₁-C₃)fluoroalkoxy group and a (C₁-C₃)alkoxy group,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Ij) 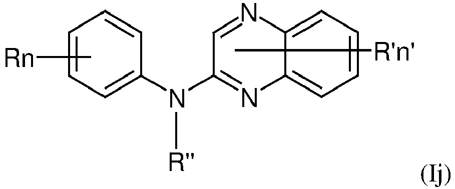 wherein:
R independently represent a hydrogen atom or a group chosen among a (C₁-C₃)fluoroalkoxy group and a (C₁-C₃)alkyl group,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Ik) 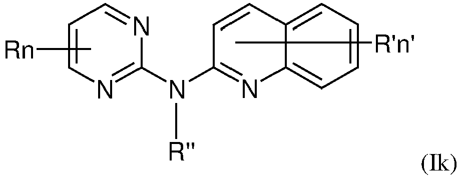 wherein:
R represents a hydrogen atom,
R" is as defined above and is advantageously a hydrogen atom,
n is as defined above and is advantageously 1,
n' is as defined above and is advantageously 1,
R' is a hydrogen atom, a halogen atom or a (C₁-C₃)alkyl group,
or one of its pharmaceutically acceptable salt,
for use as an agent for preventing, inhibiting or treating pathological conditions linked with premature aging.

According to another particular embodiment, an additional subject-matter of the present invention is a compound of formula (Il) 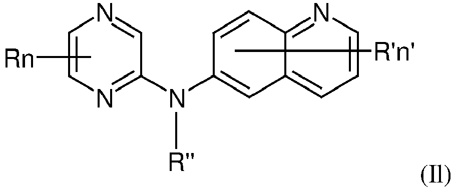 wherein:
R represents a hydrogen atom,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Im) 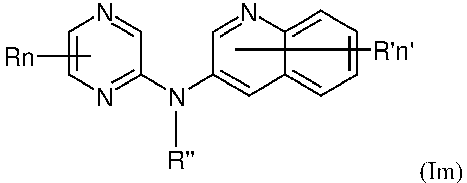 wherein:
R represents a hydrogen atom,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Io) 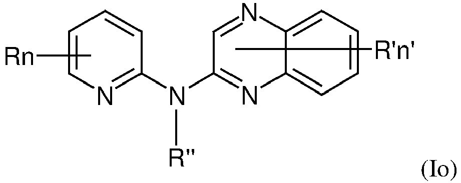 wherein:
R independently represent a hydrogen atom or a halogen atom or a group chosen among, a -NO₂ group, a -CN group and a (C₁-C₃)alkyl group, said alkyl being optionally mono-substituted by a hydroxyl group,
R" is as defined above and is advantageously a hydrogen atom,
n is as defined above and is advantageously 1,
n' is as defined above and is advantageously 1,
R' is a hydrogen atom, a halogen atom or a (C₁-C₃)fluoroalkyl group,
or one of its pharmaceutically acceptable salt,
for use as an agent for preventing, inhibiting or treating pathological conditions linked with premature aging.

According to another particular embodiment, an additional subject-matter of the present invention is a compound of formula (Ip) 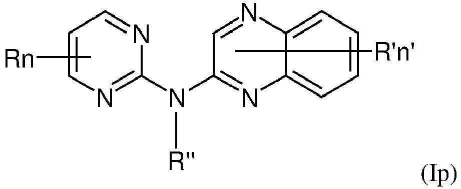 wherein:
R represents a hydrogen atom,
R" is as defined above and is advantageously a hydrogen atom,
n is as defined above and is advantageously 1,
n' is as defined above and is advantageously 1,
R' is a hydrogen atom,
or one of its pharmaceutically acceptable salt,
for use as an agent for preventing, inhibiting or treating pathological conditions linked with premature aging.

According to another particular embodiment, an additional subject-matter of the present invention is a compound of formula (Iq) 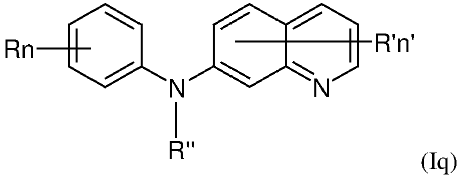 wherein:
R independently represent a hydrogen atom, a (C₁-C₃)alkoxy group or a (C₁-C₃)fluoroalkoxy group,
R" is as defined above and is advantageously a hydrogen atom,
n is as defined above and is advantageously 1,
n' is as defined above and is advantageously 1,
R' is a hydrogen atom or a group chosen among a -NR₁R₂ group, a N-methylpiperazinyl group, a (C₁-C₃)alkoxy group and a morpholino group,
R₁ and R₂ are independently a hydrogen atom or a (C₁-C₃)alkyl group,
or one of its pharmaceutically acceptable salt,
for use as an agent for preventing, inhibiting or treating pathological conditions linked with premature aging.

According to another particular embodiment, an additional subject-matter of the present invention is a compound of formula (Ir) 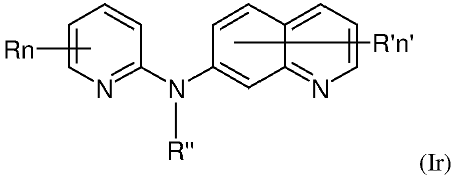 wherein:
R independently represent a hydrogen atom or a (C₁-C₃)alkyl group,
R" is as defined above and is advantageously a hydrogen atom,
n is as defined above and is advantageously 1,
n' is as defined above and is advantageously 1,
R' is a hydrogen atom or a group chosen among a -NR₁R₂ group, a morpholino group and a (C₁-C₃)alkoxy group,
R₁ and R₂ are independently a hydrogen atom or a (C₁-C₃)alkyl group,
or one of its pharmaceutically acceptable salt,
for use as an agent for preventing, inhibiting or treating pathological conditions linked with premature aging.

Among the previous defined families of compounds of formulae (Ia) to (Ir), some are more particularly preferred for their use as an agent for preventing, inhibiting or treating pathological conditions linked with premature aging. These preferred compounds particularly belong to formulae (Ia), (Ib), (Ic), (Id), (Ie), (Ii), (Ij), (Ik), (Io), (Ip) and (Ir), as defined above or one of its pharmaceutically acceptable salts.

Accordingly the present invention further relates to a compound chosen among compounds of formulae (Ia), (Ib), (Ic), (Id), (Ie), (Ii), (Ij), (Ik), (Io), (Ip), (Ir) and their pharmaceutically acceptable salts for use as an agent for preventing, inhibiting or treating pathological conditions linked with premature aging.

Furthermore, among such compounds particularly preferred for their use as described above, some of them, i.e. compounds of formulae (Ia), (Ib), (Ic), (Ie), (Ii), (Ij), (Ik), and (Io) are more particularly preferred for their use, as described below:
Thus, according to a more particular embodiment, the present invention particularly focuses on a compound of formula (Ia)
wherein:
R independently represent a hydrogen atom, a halogen atom or a group chosen among a (C₁-C₃)alkyl group, a -CN group, a -COOR₁ group, a (C₁-C₃)fluoroalkyl group, a -NO₂ group and a (C₁-C₃)alkoxy group,
R₁ is a hydrogen atom or a (C₁-C₃)alkyl group,
R" is as defined above and more preferably is a hydrogen atom,
n is as defined above and more preferably is 1,
n' is as defined above and more preferably is 1,
R' is a hydrogen atom, a halogen atom or a (C₁-C₃)alkyl group,
or one of its pharmaceutically acceptable salt,
for use as an agent for preventing, inhibiting or treating pathological conditions linked with premature aging.

The present text also describes compounds of formula (la'), 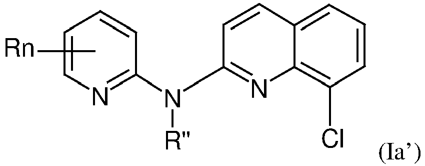 wherein,
R independently represent a hydrogen atom, a -COOR₁ group or a (C₁-C₃) alkyl group,
R₁ is as defined above,
R" is a hydrogen atom,
n is 1 or 2,
or one of its pharmaceutically acceptable salt,
for use as an agent for preventing, inhibiting or treating pathological conditions linked with premature aging.

According to another more particular embodiment, the present invention particularly focuses on a compound of formula (Ib)
wherein :
R independently represent a hydrogen atom, a halogen atom or a group chosen among a (C₁-C₃)alkyl group, a (C₁-C₃)fluoroalkoxy group and a phenoxy group,
R₁ is a hydrogen atom or a (C₁-C₃)alkyl group,
R" is as defined above and more preferably is a hydrogen atom,
n is as defined above and more preferably is 1,
n' is as defined above,
R' is a hydrogen atom,
or one of its pharmaceutically acceptable salt,
for use as an agent for preventing, inhibiting or treating pathological conditions linked with premature aging.

According to another more particular embodiment, the present invention particularly focuses on a compound of formula (Ic)
wherein:
R independently represent a hydrogen atom or a group chosen among a (C₁-C₃)alkyl group, a -NO₂ group and a (C₁-C₃)alkoxy group,
R" is as defined above and more preferably is a hydrogen atom,
n is as defined above and more preferably is 1,
n' is as defined above,
R' is a hydrogen atom,
or one of its pharmaceutically acceptable salt,
for use as an agent for preventing, inhibiting or treating pathological conditions linked with premature aging.

According to another more particular embodiment, the present invention particularly focuses on a compound of formula (Ie) wherein:
R represents a hydrogen atom,
R" is as defined above and more preferably is a hydrogen atom,
n is as defined above and more preferably is 1,
n' is as defined above,
R' is a hydrogen atom or a halogen atom,
or one of its pharmaceutically acceptable salt,
for use as an agent for preventing, inhibiting or treating pathological conditions linked with premature aging.

According to another more particular embodiment, the present invention particularly focuses on a compound of formula (Ii)
wherein:
R independently represent a hydrogen atom or a (C₁-C₃)alkoxy group,
R" is as defined above and is advantageously a hydrogen atom,
n is as defined above and is advantageously 1,
n' is as defined above,
R' is a hydrogen atom,
or one of its pharmaceutically acceptable salt,
for use as an agent for preventing, inhibiting or treating pathological conditions linked with premature aging.

According to another more particular embodiment, the present invention particularly focuses on a compound of formula (Ij)
wherein:
R independently represent a hydrogen atom or a group chosen among a (C₁-C₃)fluoroalkoxy group and a (C₁-C₃)alkyl group,
R" is as defined above and more preferably is a hydrogen atom,
n is as defined above and more preferably is 2,
n' is as defined above,
R' is a hydrogen atom,
or one of its pharmaceutically acceptable salt,
for use as an agent for preventing, inhibiting or treating pathological conditions linked with premature aging.

According to another more particular embodiment, the present invention particularly focuses on a compound of formula (Ik)
wherein:
R represents a hydrogen atom,
R" is as defined above and more preferably is a hydrogen atom,
n is as defined above and more preferably is 1,
n' is as defined above,
R' is a hydrogen atom,
or one of its pharmaceutically acceptable salt,
for use as an agent for preventing, inhibiting or treating pathological conditions linked with premature aging.

According to another more particular embodiment, the present invention particularly focuses on a compound of formula (Io)
wherein:
R independently represent a hydrogen atom or a group chosen among a (C₁-C₃)alkyl group and a -CN group,
R" is as defined above and more preferably is a hydrogen atom,
n is as defined above and more preferably is 1,
n' is as defined above,
R' is a hydrogen atom,
or one of its pharmaceutically acceptable salt,
for use as an agent for preventing, inhibiting or treating pathological conditions linked with premature aging.

In a particular embodiment, the present invention relates to a compound of formula (Ib), (Ie) or (Ij) as defined above or one of its pharmaceutically acceptable salts, for use as an agent for preventing, inhibiting or treating pathological conditions linked with premature aging.

According to a preferred embodiment of the present invention, the compound for use as an agent for preventing, inhibiting or treating pathological conditions linked with premature aging, is chosen from:
- (1) (8-Chloro-quinolin-2-yl)-pyridin-2-yl-amine
- (2) 2-(Quinolin-2-ylamino)-isonicotinic acid
- (3) (4-Methyl-pyridin-2-yl)-quinolin-2-yl-amine
- (4) Pyridin-2-yl-quinolin-2-yl-amine
- (5) 2-(8-Chloro-quinolin-2-ylamino)-isonicotinic acid
- (6) (8-Chloro-quinolin-2-yl)-(4-methyl-pyridin-2-yl)-amine
- (7) 6-(Quinolin-2-ylamino)-nicotinonitrile
- (8) Quinolin-2-yl-(4-trifluoromethoxy-phenyl)-amine
- (9) Pyridin-2-yl-quinolin-3-yl-amine
- (10) (3-Methoxy-pyridin-2-yl)-quinolin-3-yl-amine
- (11) Quinolin-3-yl-(5-trifluoromethyl-pyridin-2-yl)-amine
- (12) (5-Nitro-pyridin-2-yl)-quinolin-3-yl-amine,
- (13) (5-Methyl-pyridin-2-yl)-quinolin-3-yl-amine
- (14) 2-(Quinolin-3-ylamino)-isonicotinic acid
- (15) Quinolin-6-yl-(5-trifluoromethyl-pyridin-2-yl)-amine
- (16) (6-Methyl-pyridin-2-yl)-quinolin-6-yl-amine
- (17) N-(6-methylpyridin-2-yl)quinolin-2-amine
- (18) 8-chloro-N-(6-methylpyridin-2-yl)quinolin-2-amine
- (19) 4-methyl-N-(pyridin-2-yl)quinolin-2-amine
- (20) 4-methyl-N-(4-methylpyridin-2-yl)quinolin-2-amine
- (21) 3-methyl-N-(4-methylpyridin-2-yl)quinolin-2-amine
- (22) 3-methyl-N-(pyridin-2-yl)quinolin-2-amine
- (23) 6-((4-methylquinolin-2-yl)amino)nicotinonitrile
- (24) 6-((3-methylquinolin-2-yl)amino)nicotinonitrile
- (25) 6-chloro-N-(4-methylpyridin-2-yl)quinolin-2-amine
- (26) 6-chloro-N-(6-methylpyridin-2-yl)quinolin-2-amine
- (27) 4-methyl-N-(5-nitropyridin-2-yl)quinolin-2-amine
- (28) N-(3-nitropyridin-2-yl)quinolin-2-amine
- (29) 8-chloro-N-(3-nitropyridin-2-yl)quinolin-2-amine
- (30) 2-((4-methylquinolin-2-yl)amino)nicotinonitrile
- (31) N-(3-methylpyridin-2-yl)quinolin-2-amine
- (32) N-(5-methylpyridin-2-yl)quinolin-2-amine
- (33) 2-(quinolin-2-ylamino)isonicotinonitrile
- (34) N-(5-(trifluoromethyl)pyridin-2-yl)quinolin-2-amine
- (35) 8-chloro-N-(3-methylpyridin-2-yl)quinolin-2-amine
- (36) 8-chloro-N-(5-methylpyridin-2-yl)quinolin-2-amine
- (37) 8-chloro-N-(5-(trifluoromethyl)pyridin-2-yl)quinolin-2-amine
- (38) N-(3-methoxypyridin-2-yl)quinolin-2-amine
- (39) N-(5-nitropyridin-2-yl)quinolin-2-amine
- (40) 6-((8-chloroquinolin-2-yl)amino)nicotinonitrile
- (41) N-(5-fluoropyridin-2-yl)quinolin-2-amine
- (42) N-(6-(trifluoromethyl)pyridin-2-yl)quinolin-2-amine
- (43) 8-chloro-N-(5-fluoropyridin-2-yl)quinolin-2-amine
- (44) 2-((8-chloroquinolin-2-yl)amino)nicotinic acid
- (45) 4-methyl-N-(6-methylpyridin-2-yl)quinolin-2-amine
- (46) 3-methyl-N-(6-methylpyridin-2-yl)quinolin-2-amine
- (47) 5-cyano-2-(quinolin-2-ylamino)pyridin-1-ium chloride
- (48) 2-((8-chloroquinolin-2-yl)amino)-4-methylpyridin-1-ium chloride
- (49) 8-chloro-N-(4-ethylpyridin-2-yl)quinolin-2-amine
- (50) 8-chloro-N-(6-ethylpyridin-2-yl)quinolin-2-amine
- (51) 8-chloro-N-(4,6-dimethylpyridin-2-yl)quinolin-2-amine
- (52) 6-((8-chloroquinolin-2-yl)amino)-2-methylnicotinonitrile
- (53) 8-chloro-N-(4-chloropyridin-2-yl)quinolin-2-amine
- (54) 8-methyl-N-(4-methylpyridin-2-yl)quinolin-2-amine
- (55) N-(5-bromo-4-methylpyridin-2-yl)-8-chloroquinolin-2-amine
- (56) 8-chloro-N-(3-ethyl-6-methylpyridin-2-yl)quinolin-2-amine
- (57) 8-fluoro-N-(4-methylpyridin-2-yl)quinolin-2-amine
- (58) 8-bromo-N-(4-methylpyridin-2-yl)quinolin-2-amine
- (59) methyl 6-(quinolin-2-ylamino)nicotinate
- (60) methyl 6-[(8-chloroquinolin-2-yl)amino]pyridine-3-carboxylate
- (61) methyl 6-[(3-methylquinolin-2-yl)amino]pyridine-3-carboxylate
- (62) methyl 2-[(8-chloroquinolin-2-yl)amino]pyridine-3-carboxylate
- (63) 8-methoxy-N-(4-methylpyridin-2-yl)quinolin-2-amine
- (64) N-(4-methylpyridin-2-yl)-5-nitroquinolin-2-amine
- (65) 2-N-(4-methylpyridin-2-yl)quinoline-2,8-diamine
- (66) N-(4-methylpyridin-2-yl)-5-aminoquinolin-2-amine
- (67) methyl 6-[(4-methylquinolin-2-yl)amino]pyridine-3-carboxylate
- (68) 8-chloro-N-[4-(trifluoromethyl)pyridin-2-yl]quinolin-2-amine
- (69) 2-[(8-chloroquinolin-2-yl)amino]pyridin-3-ol
- (70) 8-chloro-N-[6-(trifluoromethyl)pyridin-2-yl]quinolin-2-amine
- (71) 6-chloro-N-(5-fluoropyridin-2-yl)quinolin-2-amine
- (72) N-(6-ethylpyridin-2-yl)-3-methylquinolin-2-amine
- (73) N-(5-fluoropyridin-2-yl)-3-methylquinolin-2-amine
- (74) 3-methyl-N-[5-(trifluoromethyl)pyridin-2-yl]quinolin-2-amine
- (75) 4-N-(8-chloroquinolin-2-yl)-1-N,1-N-dimethylbenzene-1,4-diamine
- (76) N-(4-methoxyphenyl)quinolin-2-amine
- (77) 8-chloro-N-(4-methoxyphenyl)quinolin-2-amine
- (78) 4-methyl-N-[4-(trifluoromethoxy)phenyl]quinolin-2-amine
- (79) N-(4-methoxyphenyl)-3-methylquinolin-2-amine
- (80) 3-methyl-N-[4-(trifluoromethoxy)phenyl]quinolin-2-amine
- (81) 1-N,1-N-dimethyl-4-N-(3-methylquinolin-2-yl)benzene-1,4-diamine
- (82) N-[2-methyl-4-(trifluoromethoxy)phenyl]quinolin-2-amine
- (83) N-[3-(trifluoromethoxy)phenyl]quinolin-2-amine
- (84) N-[2-(trifluoromethoxy)phenyl]quinolin-2-amine
- (85) N-(4-nitrophenyl)quinolin-2-amine
- (86) N-(3-fluorophenyl)quinolin-2-amine
- (87) 8-chloro-N-[3-(trifluoromethoxy)phenyl]quinolin-2-amine
- (88) 8-chloro-N-(3-fluorophenyl)quinolin-2-amine
- (89) 2-{[4-(tnfluoromethoxy)phenyl]amino}quinolin-1-ium chloride
- (90) 8-chloro-N-[4-(trifluoromethoxy)phenyl]quinolin-2-amine
- (91) 3-methyl-N-[2-methyl-4-(trifluoromethoxy)phenyl]quinolin-2-amine
- (92) 3-methyl-N-[3-(trifluoromethoxy)phenyl]quinolin-2-amine
- (93) 3-methyl-N-[2-(trifluoromethoxy)phenyl]quinolin-2-amine
- (94) 8-chloro-N-[2-methyl-4-(trifluoromethoxy)phenyl]quinolin-2-amine
- (95) 3-methyl-2-{[4-(trifluoromethoxy)phenyl]amino}quinolin-1-ium chloride
- (96) 6-chloro-N-(4-(trifluoromethoxy)phenyl)quinolin-2-amine
- (97) 4-methyl-2-{[4-(trifluoromethoxy)phenyl] amino}quinolin-1-ium chloride
- (98) 8-bromo-N-[4-(trifluoromethoxy)phenyl]quinolin-2-amine
- (99) 8-fluoro-N-[4-(trifluoromethoxy)phenyl]quinolin-2-amine
- (100) 8-methyl-N-[4-(trifluoromethoxy)phenyl]quinolin-2-amine
- (101) N-(4-butoxyphenyl)-8-chloroquinolin-2-amine
- (102) N-(4-phenoxyphenyl)quinolin-2-amine
- (103) 8-methoxy-N-[4-(trifluoromethoxy)phenyl]quinolin-2-amine
- (104) 8-chloro-N-[3-chloro-4-(trifluoromethoxy)phenyl]quinolin-2-amine
- (105) N-(6-methylpyridin-2-yl)quinolin-3-amine
- (106) N-(3-nitropyridin-2-yl)quinolin-3-amine
- (107) N-(5-methylpyridin-2-yl)quinolin-6-amine
- (108) N-(3-methoxypyridin-2-yl)quinolin-6-amine
- (109) 6-chloro-N-(pyrazin-2-yl)quinolin-2-amine
- (110) 8-bromo-N-(pyrazin-2-yl)quinolin-2-amine
- (111) 8-methyl-N-(pyrazin-2-yl)quinolin-2-amine
- (112) 8-chloro-N-(pyrazin-2-yl)quinolin-2-amine
- (113) N-(pyrazin-2-yl)quinolin-2-amine
- (114) 4-methyl-N-(pyrazin-2-yl)quinolin-2-amine
- (115) 3-methyl-N-(pyrazin-2-yl)quinolin-2-amine
- (116) 8-fluoro-N-(pyrazin-2-yl)quinolin-2-amine
- (117) 8-methoxy-N-(pyrazin-2-yl)quinolin-2-amine
- (118) N-(pyridin-3-yl)quinolin-3-amine
- (119) 8-chloro-N-(pyridin-4-yl)quinolin-2-amine
- (120) N-(pyridin-4-yl)quinolin-2-amine
- (121) N-(pyridin-4-yl)quinolin-3-amine
- (122) N-[4-(trifluoromethoxy)phenyl]quinolin-3-amine
- (123) N-(4-methoxyphenyl)quinolin-3-amine
- (124) N-[4-(trifluoromethoxy)phenyl]quinoxalin-2-amine
- (125) N-[2-methyl-4-(trifluoromethoxy)phenyl]quinoxalin-2-amine
- (126) N-[3-(trifluoromethoxy)phenyl]quinoxalin-2-amine
- (127) N-[2-(trifluoromethoxy)phenyl]quinoxalin-2-amine
- (128) N-(pyrimidin-2-yl)quinolin-2-amine
- (129) 8-chloro-N-(pyrimidin-2-yl)quinolin-2-amine
- (130) 4-methyl-N-(pyrimidin-2-yl)quinolin-2-amine
- (131) N-(pyrazin-2-yl)quinolin-6-amine
- (132) N-(pyrazin-2-yl)quinolin-3-amine
- (133) 6-methyl-N-(naphthalen-2-yl)pyridin-2-amine
- (134) N-(naphthalen-2-yl)pyridin-2-amine
- (135) N-(pyridin-2-yl)quinoxalin-2-amine
- (136) N-(4-methylpyridin-2-yl)quinoxalin-2-amine
- (137) 6-(quinoxalin-2-ylamino)pyridine-3-carbonitrile
- (138) N-(6-methylpyridin-2-yl)quinoxalin-2-amine
- (139) N-(4-methylpyridin-2-yl)-3-(trifluoromethyl)quinoxalin-2-amine
- (140) N-(3,5-dichloro-4-methylpyridin-2-yl)quinoxalin-2-amine
- (141) N-(4-methyl-3-nitropyridin-2-yl)quinoxalin-2-amine
- (142) N-(pyrimidin-2-yl)quinoxalin-2-amine
- (143) 4-N,4-N-dimethyl-7-N-[4-(trifluoromethoxy)phenyl]quinoline-4,7-diamine
- (144) 4-(morpholin-4-yl)-N-[4-(trifluoromethoxy)phenyl]quinolin-7-amine
- (145) 4-methoxy-N-(pyridin-2-yl)quinolin-7-amine
- (146) 4-methoxy-N-(4-methylpyridin-2-yl)quinolin-7-amine
- (147) 4-N,4-N-dimethyl-7-N-(4-methylpyridin-2-yl)quinoline-4,7-diamine
- (150) N-(4-methylpyridin-2-yl)-8-nitroquinolin-2-amine
- (151) 6-chloro-N-(6-ethylpyridin-2-yl)quinolin-2-amine
- (152) 6-chloro-N-(5-methylpyridin-2-yl)quinolin-2-amine
- (153) 6-chloro-N-[5-(trifluoromethyl)pyridin-2-yl]quinolin-2-amine
- (154) N2-(8-chloroquinolin-2-yl)-4-methylpyridine-2,3-diamine
- (155) N-(4-butoxyphenyl)-3-methylquinolin-2-amine
- (156) 4-N-(6-chloroquinolin-2-yl)-1-N,1-N-dimethylbenzene-1,4-diamine
- (157) 8-chloro-N-(3-chloro-4-methoxyphenyl)quinolin-2-amine
- (158) N1-(8-chloroquinolin-2-yl)-4-(trifluoromethoxy)benzene-1,2-diamine
- (159) N-(3-aminopyridin-2-yl)quinolin-3-amine
- (160) 6-chloro-N-(4-methylpyridin-2-yl)quinoxalin-2-amine
- (161) N-(4-ethylpyridin-2-yl)quinoxalin-2-amine
- (162) N-(5-bromo-4-methylpyridin-2-yl)quinoxalin-2-amine
- (163) N-(4,6-dimethylpyridin-2-yl)quinoxalin-2-amine
- (164) [2-(quinoxalin-2-ylamino)pyridin-4-yl]methanol
- (165) N-(4-methyl-5-nitropyridin-2-yl)quinoxalin-2-amine
- (166) N-(4-methoxyphenyl)-4-(4-methylpiperazin-1-yl)quinolin-7-amine
- (167) 4-methoxy-N-[4-(trifluoromethoxy)phenyl]quinolin-7-amine
- (168) N-(4-methylpyridin-2-yl)-4-(morpholin-4-yl)quinolin-7-amine
- and their pharmaceutically acceptable salts.

Among said compounds, compounds (2), (3), (4), (5), (7), (8), (9), (10), (13), (15), (16), (17), (18), (25), (26), (28), (31), (32), (33), (34), (35), (36), (38), (39), (41), (42), (45), (59), (61), (82), (83), (86), (102), (105), (106), (107), (108), (109), (113), (120), (123), (125), (128), (135), (136), (137), (138), (142), (145), (146) and (147) are of particular interest.

The present invention therefore extends to compounds (2), (3), (4), (5), (7), (8), (9), (10), (13), (15), (16), (17), (18), (25), (26), (28), (31), (32), (33), (34), (35), (36), (38), (39), (41), (42), (45), (59), (61), (82), (83), (86), (102), (105), (106), (107), (108), (109), (113), (120), (123), (125), (128), (135), (136), (137), (138), (142), (145), (146) and (147) or one of its pharmaceutically acceptable salts for use as an agent for preventing, inhibiting or treating pathological conditions linked with premature aging.

Some of said preceding compounds are new and form part of the present invention: (10) to (16), (105) to (108), (118), (119), (121), (124) to (130), (132), (135) to (141) (145) to (147), (159) to (165) and (168) or one of its pharmaceutically acceptable salts such as hydrochloride, hydrobromide, tartrate, fumarate, citrate, trifluoroacetate, ascorbate, triflate, mesylate, tosylate, formate, acetate and malate.

The compounds of formulae (I), (Ia), (Ib), (Ic), (Id), (Ie), (If), (Ig), (Ih), (Ii), (Ij), (Ik), (Il), (Im), (Io), (Ip), (Iq), (Ir) and (lee) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

Among the compounds of formula (I), some of them are new and form part of the invention, as well as their pharmaceutically acceptable salts, such as hydrochloride, hydrobromide, tartrate, fumarate, citrate, trifluoroacetate, ascorbate, triflate, mesylate, tosylate, formate, acetate and malate.

According to a particular embodiment, the present invention encompasses compounds of formula (Ig)
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group, and a (C₁-C₃)alkoxy group,
n is 1 or 2,
n' is 1 or 2,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
R₁ and R₂ are independently a hydrogen atom or a (C₁-C₃)alkyl group,
with the proviso that R and R' are not simultaneously a hydrogen atom,
and when n and n' are 1 and R is a hydrogen atom then R' is not a -COOH group,
or anyone of its pharmaceutically acceptable salt.

For a sake of simplification, the following compounds and their corresponding definitions are called "new compounds".

According to another particular embodiment, the present invention encompasses compounds of formula (Ic), as such, 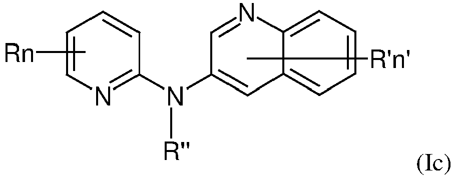 wherein:
R independently represent a hydrogen atom, a halogen atom or a group chosen among a (C₁-C₃)alkyl group, a (C₁-C₃)fluoroalkyl group, a -CN group, a hydroxyl group, a -COOR₁ group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
n' is 1 or 2,
R" is as defined in formula (Ic),
R' is a hydrogen atom or a group chosen among a (C₁-C₃)alkyl group, a -NO₂ group, a -NR₁R₂ group and a (C₁-C₃)alkoxy group,
with the proviso that
R and R' are not simultaneously a hydrogen atom,
R is not a bromine atom when R' is a hydrogen atom,
or one of its pharmaceutically acceptable salt.

Still according to this particular embodiment, the present invention more particularly focuses on compounds of formula (Ic), as such,
wherein:
R is a hydrogen atom, a (C₁-C₃)fluoroalkyl group, a (C₁-C₃)alkyl group, a (C₁-C3)alkoxy group, a -NO₂ group or a -COOR₁ group,
n, R", n' and R₁ are as defined in formula (Ic),
R' is a hydrogen atom or a group chosen among a (C₁-C₃)alkyl group, a -NO₂ group, a -NR₁R₂ group and a (C₁-C₃)alkoxy group, and is preferably a hydrogen atom,
or one of its pharmaceutically acceptable salt.

According to another particular embodiment, the present invention encompasses compounds of formula (Id), as such, 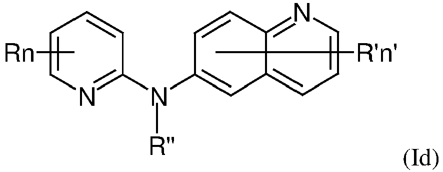 wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group and a -NR₁R₂ group,
R₁ and R₂ are independently a hydrogen atom or a (C₁-C₃)alkyl group,
n is 1 or 2, and advantageously 1,
n' is 1 or 2,
R" is as defined in formula (I) and is advantageously a hydrogen atom,
R' is a hydrogen atom or a group chosen among a (C₁-C₃)alkyl group, a -NO₂ group, a -NR₁R₂ group and a (C₁-C₃)alkoxy group,
with the proviso that when R' is a hydrogen atom, R is different from a -NO₂ group, a -NH2 group or a -COOH group,
or one of its pharmaceutically acceptable salt.

Still according to this particular embodiment, the present invention more particularly focuses on compounds of formula (Id), as such, wherein,
R is a hydrogen atom, a (C₁-C₃)alkyl group, a (C₁-C₃)alkoxy group or a (C₁-C₃)fluoroalkyl group,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group, and advantageously a hydrogen atom,
R" is as defined in formula (I) and is advantageously a hydrogen atom,
n is 1 or 2, and advantageously 1,
n' is 1 or 2,
or one of its pharmaceutically acceptable salt.

According to another particular embodiment, the present invention encompasses compounds of formula (Ii'), as such, 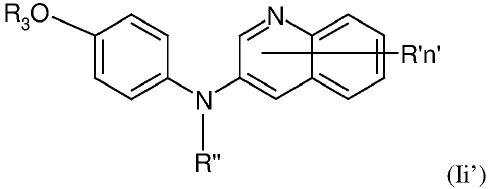 wherein:
R3 is a (C₁-C₃)fluoroalkyl group or a (C₁-C₃)alkyl group,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined above and is advantageously a hydrogen atom,
n' is as defined above and is advantageously 1,
with the proviso that
when R' is a hydrogen atom, R₃ is not a methyl group or a trifluoromethyl group
or one of its pharmaceutically acceptable salt.

According to another particular embodiment, the present invention encompasses compounds of formula (Ij'), as such, 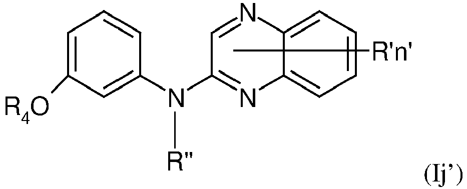 wherein:
R4 is a (C₁-C₃)fluoroalkyl group or a (C₁-C₃)alkyl group,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined above and is advantageously a hydrogen atom,
n' is as defined above and is advantageously 1,
with the proviso that
when R' is a hydrogen atom, R4 is not a methyl group
or one of its pharmaceutically acceptable salt.

According to another particular embodiment, the present invention encompasses compounds of formula (Ij"), as such, 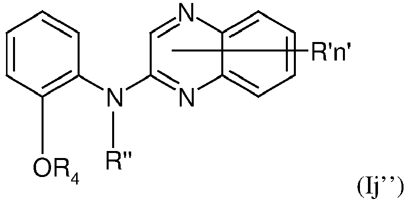 wherein:
R4 is a (C₁-C₃)fluoroalkyl group or a (C₁-C₃)alkyl group,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined above and is advantageously a hydrogen atom,
n' is as defined above and is advantageously 1,
or one of its pharmaceutically acceptable salt.

According to another particular embodiment, the present invention encompasses compounds of formula (Ij‴), as such, 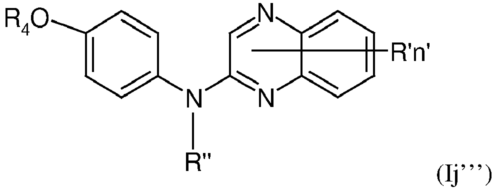 wherein:
R4 is a (C₁-C₃)fluoroalkyl group or a (C₁-C₃)alkyl group,
R' is a hydrogen atom or a group chosen among a (C₁-C₃)alkyl group, a halogen atom, a hydroxyl group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined above and is advantageously a hydrogen atom,
n' is as defined above and is advantageously 1,
with the proviso that
when R' is a chlorine atom or a hydrogen atom, R₄ is not an ethyl group or a methyl group,
when R' is a methyl group or a tertio-butyl group, R4 is not a methyl group,
or one of its pharmaceutically acceptable salt.

According to another particular embodiment, the present invention encompasses compounds of formula (Ik), as such, 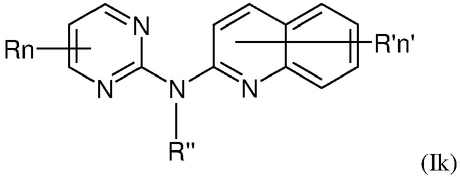 wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy,
R₁ and R₂ are independently a hydrogen atom or a (C₁-C₃)alkyl group,
n is 1 or 2, and is advantageously 1,
n' is 1 or 2,
R" is as defined in formula (Ik),
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
or one of its pharmaceutically acceptable salt.

Still according to this particular embodiment, the present invention more particularly focuses on compounds of formula (Ik), as such,
wherein:
R is a hydrogen atom,
R" is as defined in formula (Ik),
R' is a hydrogen atom, a halogen atom or a (C₁-C₃) alkyl group,
n is 1 or 2, and is advantageously 1,
n' is 1 or 2,
or one of its pharmaceutically acceptable salt.

According to another particular embodiment, the present invention encompasses compounds of formula (Io), as such, 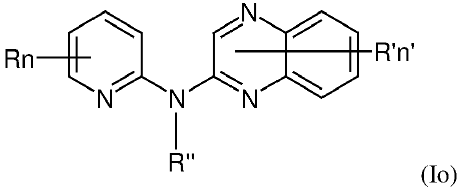 wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1, 2 or 3,
n' is 1 or 2,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
with the proviso that
when R is a hydrogen atom and n' is 1, R' is not a hydroxyl group,
or one of its pharmaceutically acceptable salt.

Still according to this particular embodiment, the present invention more particularly focuses on compounds of formula (Io), as such,
wherein:
R is a hydrogen atom, a (C₁-C₃)alkyl group or a -CN group,
n is 1, 2 or 3,
n' is 1 or 2,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group, and preferably is a hydrogen atom or a (C₁-C₃)alkyl group,
R₁ is as defined in formula (Io),
R" is a hydrogen atom or a (C₁-C₄)alkyl group,
or one of its pharmaceutically acceptable salt.

According to another particular embodiment, the present invention encompasses compounds of formula (Ip), as such, 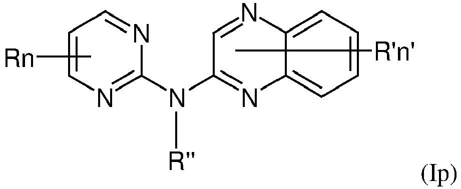 wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
n' is 1 or 2,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
with the proviso that
R and R' are not simultaneously a hydrogen atom,
when n and n' are 2 then R and R' are not simultaneously a methyl group.
or one of its pharmaceutically acceptable salt.

According to another particular embodiment, the present invention encompasses compounds of formula (Ir), as such, 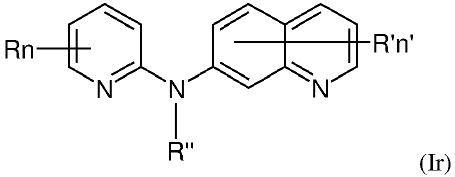 wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
n' is 1 or 2,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
or one of its pharmaceutically acceptable salt.

Still according to this particular embodiment, the present invention more particularly focuses on compounds of formula (Ir), as such,
wherein:
R is a hydrogen atom or a (C₁-C₃)alkyl group,
R' is a (C₁-C₃)alkoxy group or a -NR₁R₂ group,
R" is a hydrogen atom or a (C₁-C₄)alkyl group,
n and n' are 1,
R₁ and R₂ are independently a hydrogen atom or a (C₁-C₃)alkyl group,
or one of its pharmaceutically acceptable salt.

Among said compounds as such, compounds (10)-(16), (105)-(108), (118), (119), (121), (124)-(130), (132), (135)-(141), (145)-(147), (159)-(165), and (168) and their pharmaceutically acceptable salts are of particular interest.

The present invention therefore extends to compounds (10)-(16), (105)-(108), (118), (119), (121), (124)-(130), (132), (135)-(141), (145)-(147), (159)-(165) and (168) and their pharmaceutically acceptable salts, as such.

The present invention therefore extends more preferably to compounds (10)-(16), (105)-(108), (118), (119), (121), (124)-(130), (132), (135)-(141), (145)-(147), (159)-(165) and (168) and their pharmaceutically acceptable salts, such as hydrochloride, hydrobromide, tartrate, fumarate, citrate, trifluoroacetate, ascorbate, triflate, mesylate, tosylate, formate, acetate and malate.

The new compounds of the present invention, i.e. compounds of formula (Ic), (Id), (Ie), (Ik), (Ii'), (Ij'), (Ij"), (Ij‴), (Io), (Ip) and (Ir) and the specific compounds as listed above, are not only useful as agent for inhibiting, preventing or treating premature aging but can also be used as agent for inhibiting, preventing or treating AIDS or cancer, and more particularly colorectal cancer, pancreatic cancer, lung cancer including non-small cell lung cancer, breast cancer, bladder cancer, gall bladder cancer, liver cancer, thyroid cancer, melanoma, uterine/cervical cancer, oesophageal cancer, kidney cancer, ovarian cancer, prostate cancer, head and neck cancer and stomach cancer, etc.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

The compounds of general formula (I) can be prepared according to scheme 1 below.

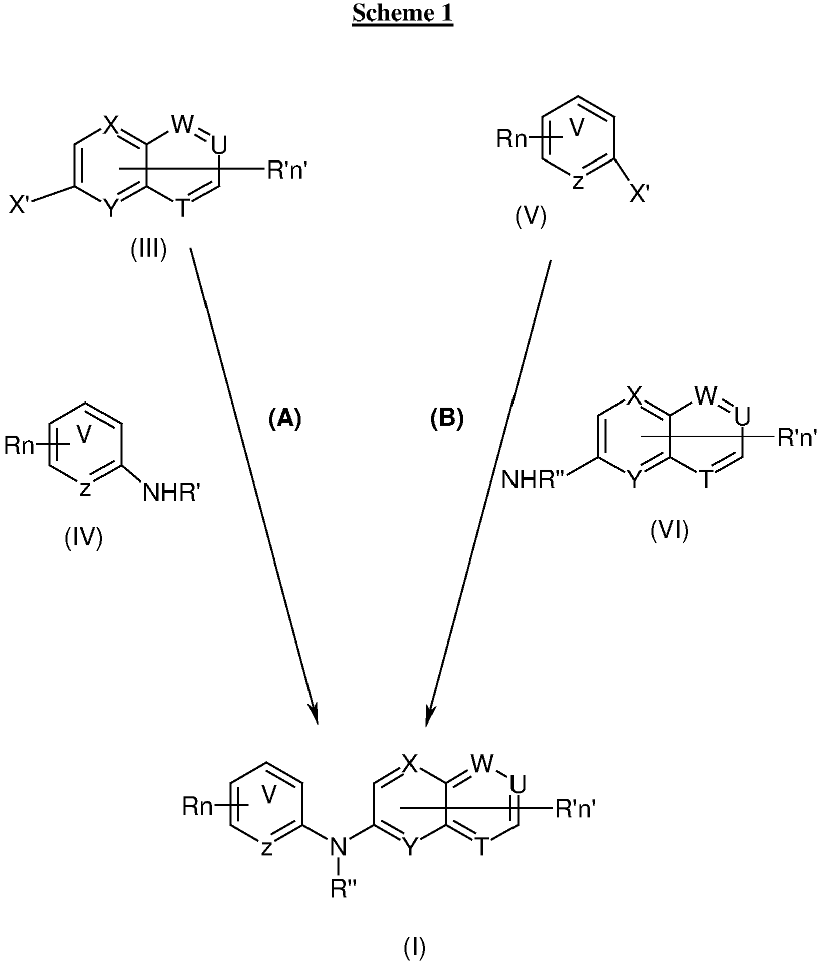

As appears in said scheme two routes are available for recovering a compound of formula (I) according to the present invention.

The synthesis is based on a coupling reaction alternatively starting from a halogeno-bicycle of formula (III), wherein X, Y, W, T, U, n', R' and R" are as defined above and X' is a chlorine atom or a bromine atom or from a chloro-monocycle of formula (V), wherein Z, V, n and R are as defined above and X' is a chlorine atom or a bromine atom.

According to route (A), the compound of formula (III) is placed in a protic solvent such as tert-butanol. The compound of formula (IV) is then added in a molar ratio ranging from 1 to 1.5 with respect to the compound of formula (III) in presence of an inorganic base, such as Cs₂CO₃ or K₂CO₃ in a molar ratio ranging from 1 and 2, in the presence of a diphosphine, such as Xantphos (4,5-Bis(diphenylphosphino)-9,9-dimethylxanthene) or X-Phos (2-Dicyclohexylphosphino-2',4',6'-triisopropylbiphenyl) in an amount ranging from 2mol% to 10mol% relative to the total amount of compound of formula (III), and in the presence of a catalyst, such as Pd(OAc)2 or Pd2dba3 in an amount ranging from 2mol% to 10mol% relative to the total amount of compound of formula (III). The reaction mixture can then be heated at a temperature ranging from 80 to 120°C, for example at 90°C and stirred for a time ranging form 15 to 25 hours, for example during 20 hours under inert gas and for example argon. The reaction mixture can be concentrated under reduced pressure.

According to route (B) the compound of formula (V) is placed in a protic solvent such as tert-butanol. The compound of formula (VI) is then added in a molar ratio ranging from 1 to 1.5 with respect to the compound of formula (V) in presence of an inorganic base, such as Cs₂CO₃ or K₂CO₃ in a molar ratio ranging from 1 to 2, in the presence of a diphosphine, such as Xantphos (4,5-Bis(diphenylphosphino)-9,9-dimethylxanthene) or X-Phos (2-Dicyclohexylphosphino-2',4',6'-triisopropylbiphenyl) in an amount ranging from 2 mol% to 10 mol% relative to the total amount of compound of formula (V), and in the presence of a catalyst, such as Pd(OAc)2 or Pd2dba3 in an amount ranging from 2 mol% to 10 mol% relative to the total amount of compound of formula (V). The reaction mixture can then be heated at a temperature ranging from 80 to 120°C, for example at 90°C and stirred for a time ranging form 15 to 25 hours, for example during 20 hours under inert gas and for example argon. The reaction mixture can be concentrated under reduced pressure.

The starting compounds of formula (III), (IV), (V) and (VI) are commercially available or can be prepared according to methods known to the person skilled in the art.

The chemical structures and spectroscopic data of some compounds of formula (I) of the invention are illustrated respectively in the following Table I and Table II.

**Table I**

| 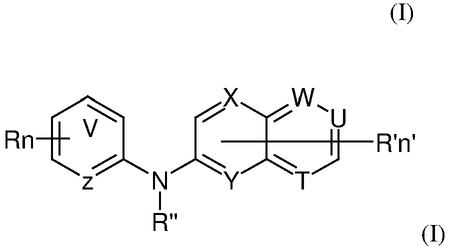 | |
|---|---|
| **Formula (Ia) (invention)** | |
| 1 | 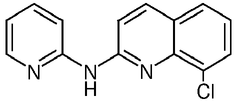 |
| 2 | 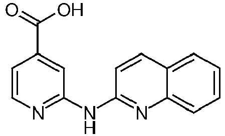 |
| 3 | 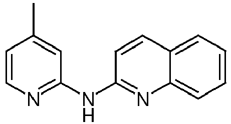 |
| 4 | 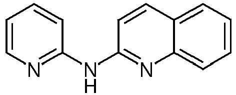 |
| 5 | 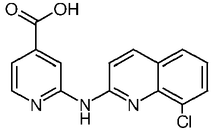 |
| 6 | 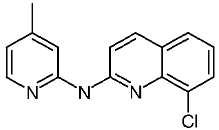 |
| 7 | 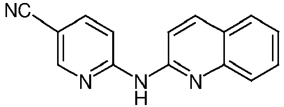 |
| 17 | 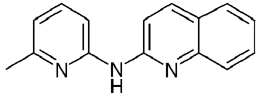 |
| 18 | 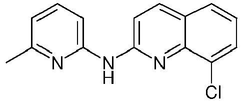 |
| 19 | 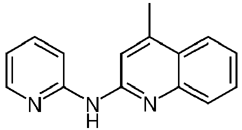 |
| 20 | 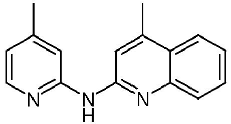 |
| 21 | 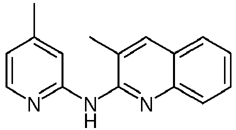 |
| 22 | 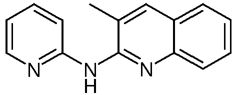 |
| 23 | 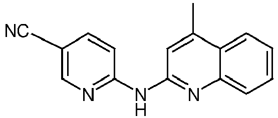 |
| 24 | 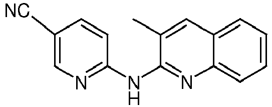 |
| 25 | 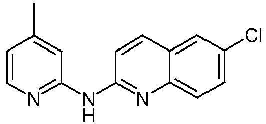 |
| 26 | 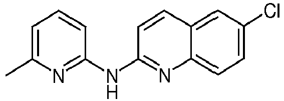 |
| 27 | 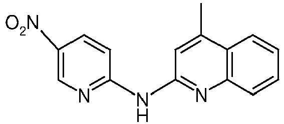 |
| 28 | 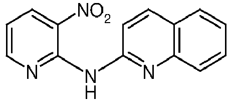 |
| 29 | 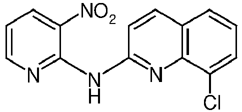 |
| 30 | 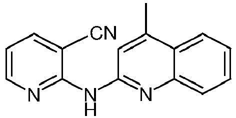 |
| 31 | 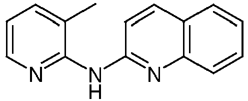 |
| 32 | 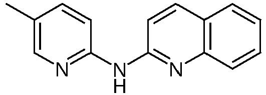 |
| 33 | 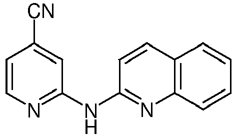 |
| 34 | 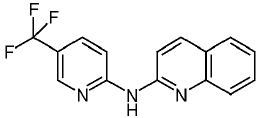 |
| 35 | 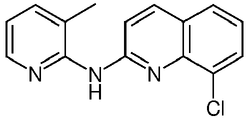 |
| 36 | 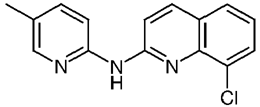 |
| 37 | 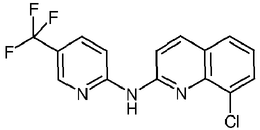 |
| 38 | 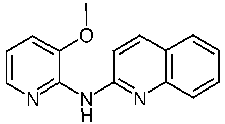 |
| 39 | 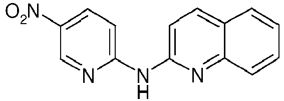 |
| 40 | 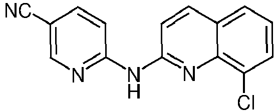 |
| 41 | 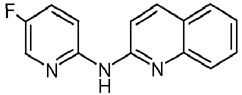 |
| 42 | 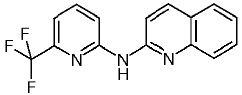 |
| 43 | 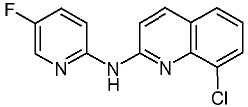 |
| 44 | 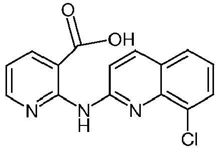 |
| 45 | 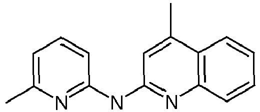 |
| 46 | 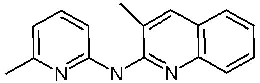 |
| 47 | 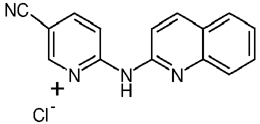 |
| 48 | 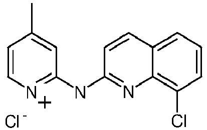 |
| 49 | 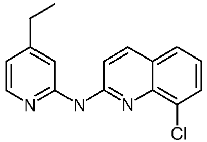 |
| 50 | 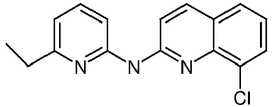 |
| 51 | 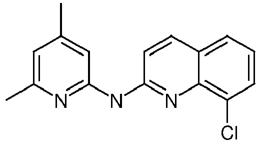 |
| 52 | 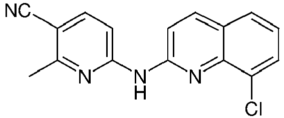 |
| 53 | 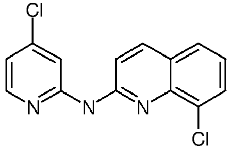 |
| 54 | 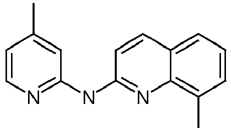 |
| 55 | 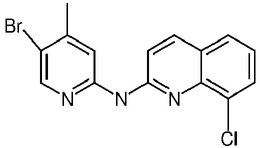 |
| 56 | 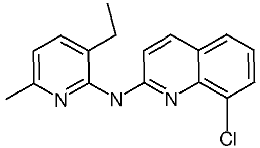 |
| 57 | 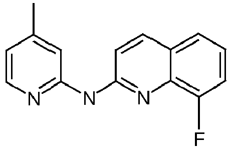 |
| 58 | 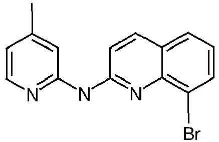 |
| 59 | 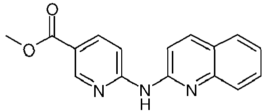 |
| 60 | 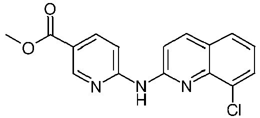 |
| 61 | 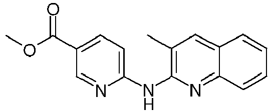 |
| 62 | 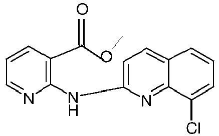 |
| 63 | 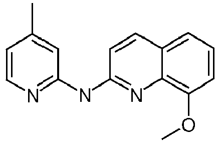 |
| 64 | 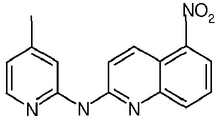 |
| 65 | 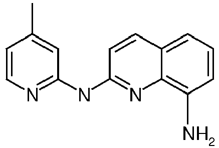 |
| 66 | 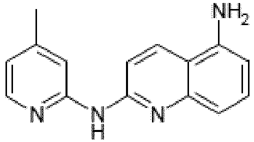 |
| 67 | 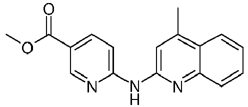 |
| 68 | 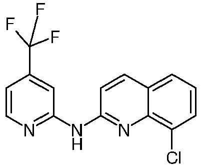 |
| 69 | 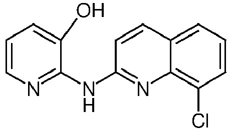 |
| 70 | 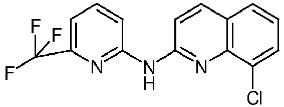 |
| 71 | 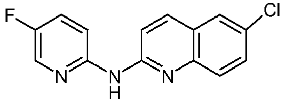 |
| 72 | 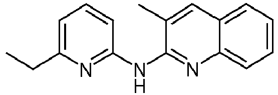 |
| 73 | 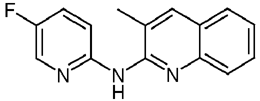 |
| 74 | 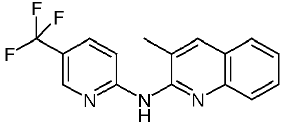 |
| 150 | 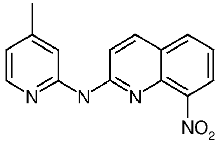 |
| 151 | 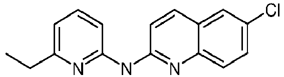 |
| 152 | 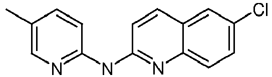 |
| 153 | 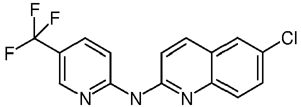 |
| 154 | 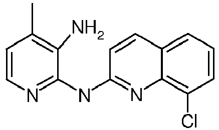 |

| **Formula (Ib) (invention)** | |
|---|---|
| 8 | 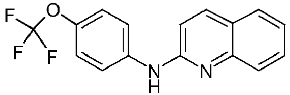 |
| 75 | 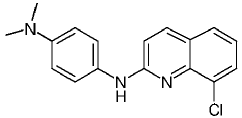 |
| 76 | 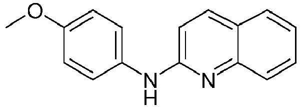 |
| 77 | 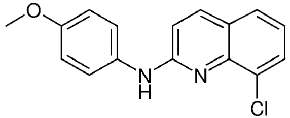 |
| 78 | 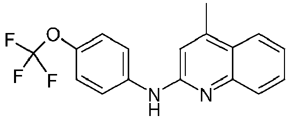 |
| 79 | 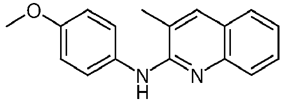 |
| 80 | 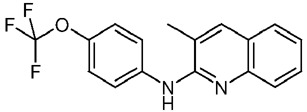 |
| 81 | 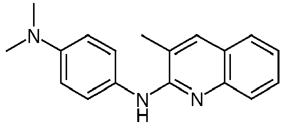 |
| 82 | 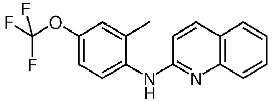 |
| 83 | 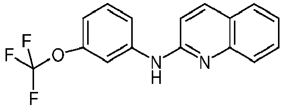 |
| 84 | 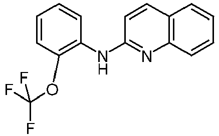 |
| 85 | 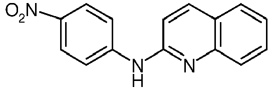 |
| 86 | 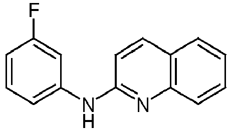 |
| 87 | 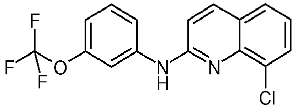 |
| 88 | 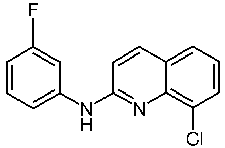 |
| 89 | 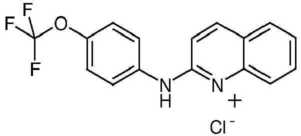 |
| 90 | 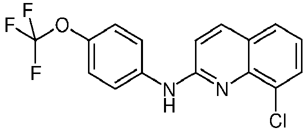 |
| 91 | 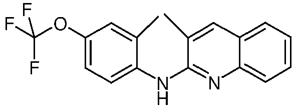 |
| 92 | 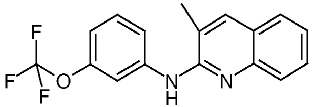 |
| 93 | 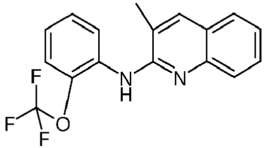 |
| 94 | 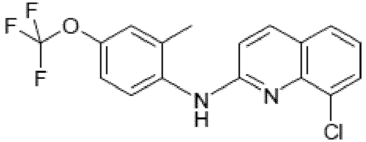 |
| 95 | 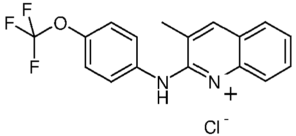 |
| 96 | 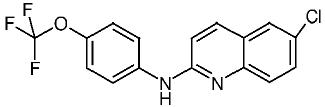 |
| 97 | 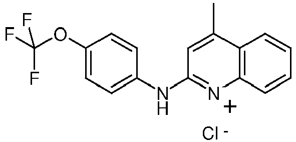 |
| 98 | 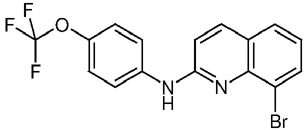 |
| 99 | 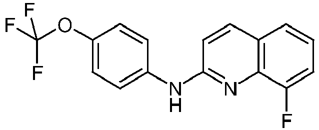 |
| 100 | 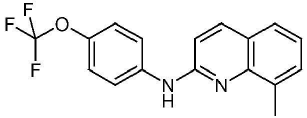 |
| 101 | 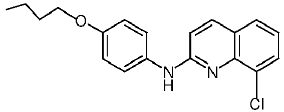 |
| 102 | 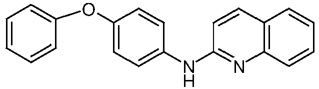 |
| 103 | 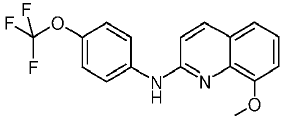 |
| 104 | 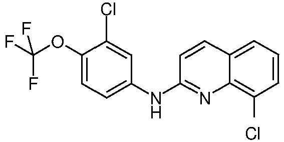 |
| 155 | 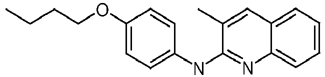 |
| 156 | 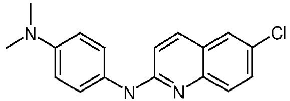 |
| 157 | 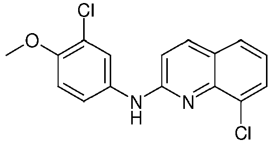 |
| 158 | 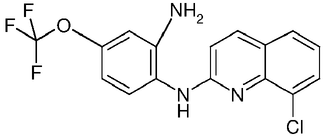 |

| **Formula (Ic) (invention)** | |
|---|---|
| 9 | 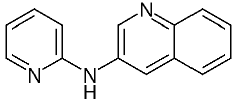 |
| 10 | 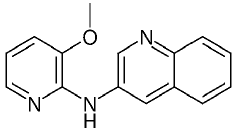 |
| 11 | 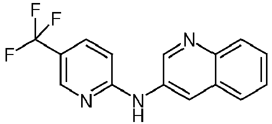 |
| 12 | 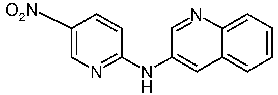 |
| 13 | 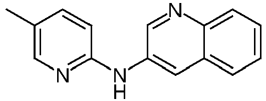 |
| 14 | 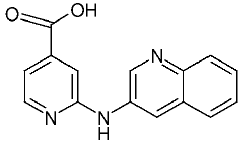 |
| 105 | 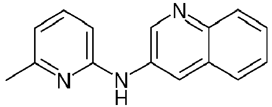 |
| 106 | 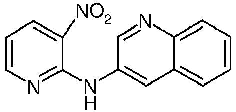 |
| 159 | 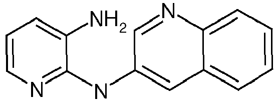 |

| **Formula (Id) (invention)** | |
|---|---|
| 15 | 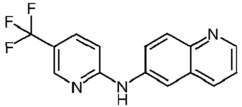 |
| 16 | 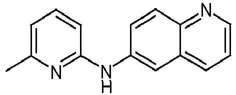 |
| 107 | 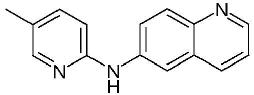 |
| 108 | 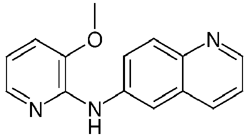 |

| **Formula (Ie) (invention)** | |
|---|---|
| 109 | 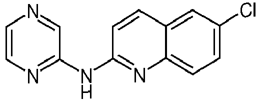 |
| 110 | 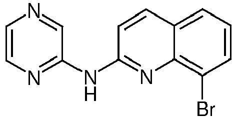 |
| 111 | 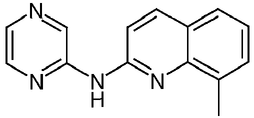 |
| 112 | 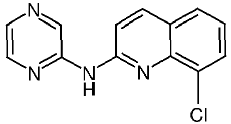 |
| 113 | 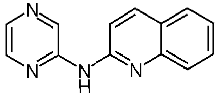 |
| 114 | 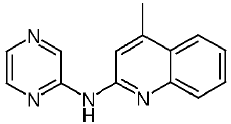 |
| 115 | 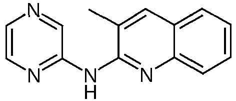 |
| 116 | 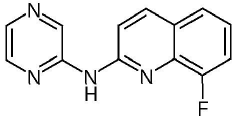 |
| 117 | 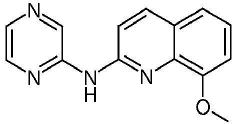 |

| **Formula (If) (invention)** | |
|---|---|
| 118 | 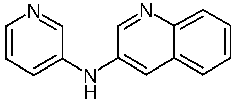 |

| **Formula (Ig) (invention)** | |
|---|---|
| 119 | 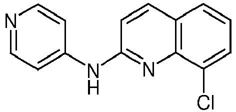 |
| 120 | 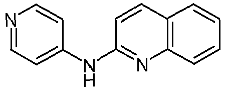 |

| **Formula (Ih) (invention)** | |
|---|---|
| 121 | 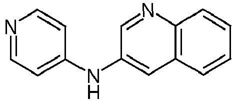 |

| **Formula (Ii) (invention)** | |
|---|---|
| 122 | 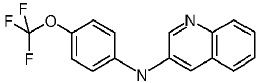 |
| 123 | 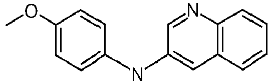 |

| **Formula (Ij) (invention)** | |
|---|---|
| 124 | 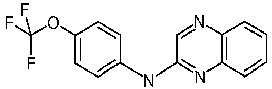 |
| 125 | 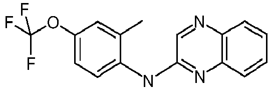 |
| 126 | 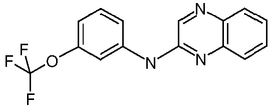 |
| 127 | 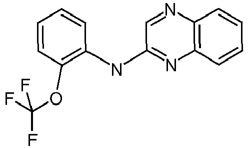 |

| **Formula (Ik) (invention)** | |
|---|---|
| 128 | 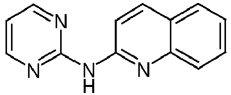 |
| 129 | 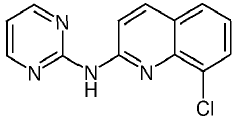 |
| 130 | 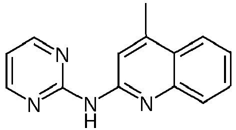 |

| **Formula (Il) (invention)** | |
|---|---|
| 131 | 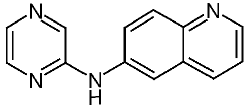 |

| **Formula (Im) (invention)** | |
|---|---|
| 132 | 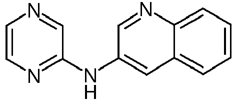 |

| **Formula (Io) (invention)** | |
|---|---|
| 135 | 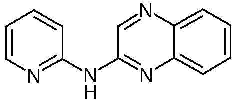 |
| 136 | 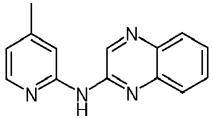 |
| 137 | 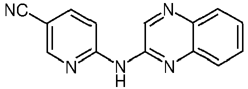 |
| 138 | 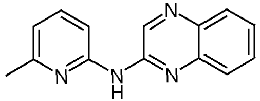 |
| 139 | 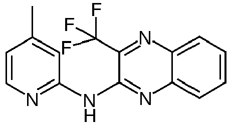 |
| 140 | 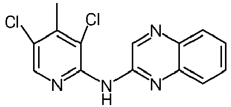 |
| 141 | 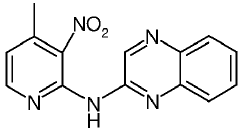 |
| 160 | 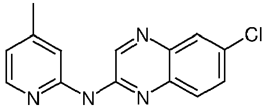 |
| 161 | 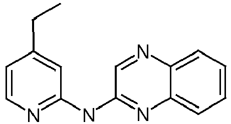 |
| 162 | 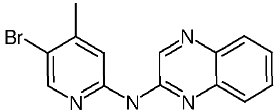 |
| 163 | 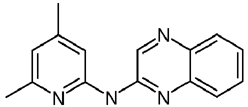 |
| 164 | 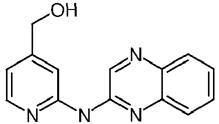 |
| 165 | 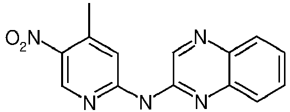 |

| **Formula (Ip) (invention)** | |
|---|---|
| 142 | 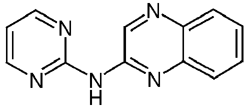 |

| **Formula (Iq) (invention)** | |
|---|---|
| 143 | 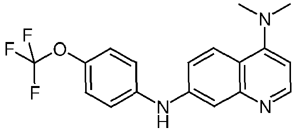 |
| 144 | 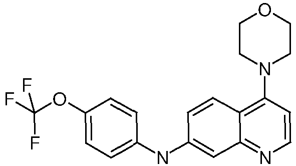 |
| 166 | 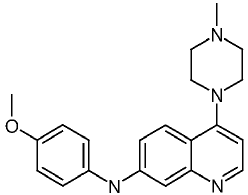 |
| 167 | 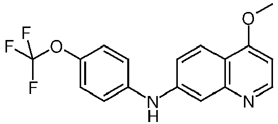 |

| **Formula (Ir) (invention)** | |
|---|---|
| 145 | 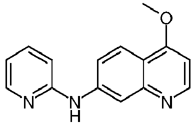 |
| 146 | 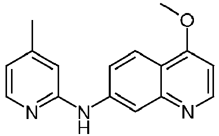 |
| 147 | 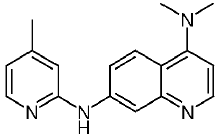 |
| 168 | 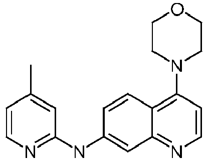 |

| **Formula (lee) (outside the invention)** | |
|---|---|
| 148 | 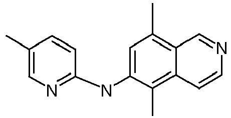 |
| 149 | 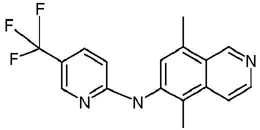 |

**Table II**

| **Ex** | **Characterizations** |
|---|---|
| **1** | MS (ESI) [M+H] ⁺=256 |
| **2** | ¹H NMR (300 MHz, D₂O) δ 8.31 (d, *J* = 5.1, 1H), 8.21 (d, *J* = 9.3, 1H), 7.60 (d, *J* = 7.5, 3H), 7.34 (dd, *J* = 6.2, 15.6, 2H), 7.18 (s, 1H), 6.99 (d, *J* = 9.1, 1H) |
| | MS (ESI) [M+H] ⁺=266 |
| **5** | MS (ESI) [M+H] ⁺=300 |
| **6** | ¹H NMR (300 MHz, DMSO) δ 10.23 (s, 1H), 8.96 (s, 1H), 8.18 (d, *J* = 8.8, 2H), 7.78 (dd, *J* = 7.7, 13.7, 2H), 7.46 (d, *J* = 8.9, 1H), 7.31 (t, *J* = 7.8, 1H), 6.86 (d, *J* = 4.3, 1H), 2.37 (s, 3H). |
| | ¹³C NMR (75 MHz, DMSO) δ 153.63, 153.61, 148.37, 147.32, 142.65, 137.52, 129.68, 129.47, 126.82, 125.06, 123.26, 118.36, 115.10, 113.31, 21.24. |
| | MS (ESI) [M+H] ⁺=270 |
| **7** | ¹H NMR (300 MHz, DMSO) δ 10.71 (s, 1H), 8.71 (d, *J* = 1.4, 1H), 8.62 (d, *J* = 8.9, 1H), 8.24 (d, *J* = 8.9, 1H), 8.17 (dd, *J* = 1.9, 8.9, 1H), 7.89 - 7.74 (m, 2H), 7.66 (dd, *J* = 7.9, 14.2, 2H), 7.42 (t, *J* = 7.3, 1H). ¹³C NMR (75 MHz, DMSO) δ 156.09, 152.40, 152.11, 146.24, 141.07, 137.83, 129.87, 127.67, 126.78, 124.50, 124.21, 118.04, 114.49, 111.67, 100.12. |
| | MS (ESI) [M+H] ⁺=247 |
| **8** | ¹H NMR (300 MHz, CDCl₃) δ 7.92 (d, *J* = 8.9, 1H), 7.79 (d, *J* = 8.4, 1H), 7.65 (t, *J* = 7.7, 3H), 7.59 (dd, *J* = 7.1, 8.3, 1H), 7.31 (t, *J* = 7.0, 1H), 7.20 (d, *J* = 8.5, 2H), 6.88 (d, *J* = 8.9, 1H), 6.80 (s, 1H) |
| | ¹³C NMR (75 MHz, CDCl₃) δ 153.88, 147.62, 144.35, 139.26, 138.11, 130.13, 127.65, 127.12, 124.43, 123.70, 122.20, 120.95, 112.25. |
| | MS (ESI) [M+H] ⁺=305 |
| **10** | ¹H NMR (300 MHz, CDCl₃) δ 9.10 (d, *J* = 2.5, 1H), 8.83 (d, *J* = 2.6, 1H), 8.02 (d, *J* = 7.9, 1H), 7.94 (dd, *J* = 1.3, 5.0, 1H), 7.85 - 7.79 (m, 1H), 7.52 (pd, *J* = 1.5, 6.9, 2H), 7.33 (s, 1H), 7.04 (dd, *J* = 1.2, 7.9, 1H), 6.81 (dd, *J* = 5.1, 7.9, 1H), 3.95 (s, 3H) |
| **11** | MS (ESI) [M+H]+=290 |
| **12** | ¹H NMR (300 MHz, CDCl₃) δ 9.18 (d, *J* = 2.7, 1H), 8.86 (d, *J* = 2.5, 1H), 8.56 (d, *J* = 2.3, 1H), 8.33 (dd, *J* = 2.7, 9.2, 1H), 8.08 (d, *J* = 8.5, 1H), 7.83 (d, *J* = 8.5, 1H), 7.71 - 7.63 (m, 2H), 7.57 (t, *J* = 7.4, 2H), 6.82 (d, *J* = 9.1, 1H) |
| **13** | ¹H NMR (300 MHz, CDCl₃) δ 8.83 (d, *J* = 2.6, 1H), 8.37 (d, *J* = 2.3, 1H), 8.00 (d, *J* = 8.2, 1H), 7.71 (d, *J* = 7.7, 1H), 7.59 - 7.51 (m, 1H), 7.46 (dd, *J* = 7.3, 15.1, 2H), 6.71 (d, *J* = 8.3, 1H), 6.67 (d, *J* = 7.4, 1H), 2.49 (s, 3H) |
| | ¹³C NMR (75 MHz, CDCl₃) δ 157.13, 154.59, 145.81, 144.43, 138.78, 134.54, 129.22, 128.86, 127.41, 127.27, 121.48, 115.41, 106.50, 24.18. |
| | MS (ESI) [M+H] ⁺=236 |
| **14** | MS (ESI) [M+H]+= 266 |
| **15** | MS (ESI) [M+H]+= 290 |
| **16** | ¹H NMR (300 MHz, CDCl₃) δ 8.77 (dd, *J* = 1.5, 4.2, 1H), 8.04 (dd, *J* = 4.7, 8.7, 2H), 7.92 (d, *J* = 2.4, 1H), 7.59 (dd, *J* = 2.5, 9.1, 1H), 7.47 (t, *J* = 7.8, 1H), 7.35 (dd, *J* = 4.2, 8.3, 1H), 6.87 (s, 1H), 6.81 (d, *J* = 8.2, 1H), 6.70 (d, *J* = 7.4, 1H), 2.50 (s, 3H) |
| | MS (ESI) [M+H] ⁺=236 |
| **18** | ¹H NMR (300 MHz, CDCl₃) δ 8.53 (d, *J* = 59.9, 2H), 7.76 (d, *J* = 8.6, 1H), 7.58 (t, *J* = 8.3, 2H), 7.42 (d, *J* = 7.8, 1H), 7.09 (t, *J* = 7.7, 1H), 6.95 (d, *J* = 8.7, 1H), 6.71 (d, *J* = 7.3, 1H), 2.38 (s, 3H) |
| **21** | ¹H NMR (300 MHz, CDCl₃) δ 8.78 (s, 1H), 8.13 (d, *J* = 5.1, 1H), 7.89 (d, *J* = 8.3, 1H), 7.79 (s, 1H), 7.63 (d, *J* = 8.0, 1H), 7.56 (d, *J* = 7.3, 1H), 7.38 (s, 1H), 7.33 (t, *J* = 7.5, 1H), 6.79 (d, *J* = 4.9, 1H), 2.44 (s, 6H) |
| **22** | ¹H NMR (300 MHz, CDCl₃) δ 8.95 (d, *J* = 8.4, 1H), 8.28 (d, *J* = 5.7, 1H), 7.87 (d, *J* = 8.3, 1H), 7.78 (s, 1H), 7.76 - 7.70 (m, 1H), 7.62 (d, *J* = 8.0, 1H), 7.60 - 7.52 (m, 1H), 7.42 (s, 1H), 7.32 (t, *J* = 7.4, 1H), 6.95 (dd, *J* = 5.1, 6.5, 1H), 2.45 (s, 3H) |
| **23** | ¹H NMR (300 MHz, CDCl₃) δ 8.64 (d, *J* = 8.4, 1H), 8.55 (d, *J* = 2.1, 1H), 8.03 (s, 1H), 7.90 (d, *J* = 8.5, 4H), 7.66 (t, *J* = 7.6, 1H), 7.44 (t, *J* = 7.6, 1H), 7.06 (s, 1H), 2.67 (s, 4H) |
| **24** | ¹H NMR (300 MHz, CDCl₃) δ 9.09 (d, *J* = 8.9, 1H), 8.53 (d, *J* = 1.7, 1H), 7.94 (dd, *J* = 2.2, 8.9, 1H), 7.92 - 7.84 (m, 2H), 7.67 (d, *J* = 8.6, 2H), 7.65 - 7.58 (m, 1H), 7.40 (t, *J* = 7.4, 1H), 2.49 (s, 3H) |
| **25** | ¹H NMR (300 MHz, CDCl₃) δ 8.16 (d, *J* = 5.2, 1H), 8.10 (s, 1H), 7.90 (d, *J* = 8.8, 1H), 7.79 (d, *J* = 9.0, 1H), 7.66 (d, *J* = 2.2, 1H), 7.55 (dd, *J* = 2.3, 8.9, 1H), 7.39 (d, *J* = 9.0, 1H), 6.79 (d, *J* = 5.2, 1H), 2.42 (s, 3H) |
| | MS (ESI) [M+H] ⁺=270 |
| **26** | ¹H NMR (300 MHz, CDCl₃) δ 8.06 (d, *J* = 8.3, 1H), 7.70 (d, *J* = 9.0, 1H), 7.64 (d, *J* = 8.9, 1H), 7.49 (t, *J* = 7.9, 2H), 7.40 (dd, *J* = 2.3, 8.9, 1H), 7.18 (d, *J* = 8.9, 1H), 6.68 (d, *J* = 7.4, 1H), 2.38 (s, 3H) |
| | MS (ESI) [M+H] ⁺=270 |
| **27** | ¹H NMR (300 MHz, CDCl₃) δ 9.17 (d, *J* = 2.5, 1H), 8.71 (s, 1H), 8.49 (dd, *J* = 2.6, 9.0, 1H), 7.99 (s, 1H), 7.93 (d, *J* = 8.9, 2H), 7.74 - 7.64 (m, 1H), 7.48 (dd, *J* = 4.2, 11.4, 1H), 7.09 (s, 1H), 2.71 (s, 3H) |
| **28** | ¹H NMR (300 MHz, CDCl₃) δ 8.64 - 8.51 (m, 3H), 8.18 (d, *J* = 9.0, 1H), 7.93 (d, *J* = 8.4, 1H), 7.79 (d, *J* = 8.1, 1H), 7.73 - 7.64 (m, 1H), 7.51 - 7.41 (m, 1H), 7.00 (dd, *J* = 4.6, 8.2, 1H), 6.75 (dd, *J* = 4.6, 8.3, 0H) |
| **29** | ¹H NMR (300 MHz, CDCl₃) δ 10.77 (s, 1H), 8.60 (s, 3H), 8.19 (d, *J* = 8.2, 1H), 7.76 (dd, *J* = 6.6, 25.5, 2H), 7.38 (d, *J* = 7.2, 1H), 7.04 (d, *J* = 4.4, 1H) |
| **30** | ¹H NMR (300 MHz, CDCl₃) δ 8.46 (dd, *J* = 1.9, 5.0, 1H), 7.87 (dd, *J* = 2.0, 7.6, 1H), 7.82 (d, *J* = 7.3, 1H), 7.60 (t, *J* = 7.3, 2H), 7.43 - 7.33 (m, 1H), 6.90 (dd, *J* = 5.0, 7.6, 1H), 2.64 (s, 3H) |
| **31** | ¹H NMR (300 MHz, CDCl₃) δ 8.44 (d, *J* = 9.1, 1H), 8.17 (d, *J* = 4.8, 1H), 8.03 (d, *J* = 9.1, 1H), 7.78 (d, *J* = 8.4, 1H), 7.68 (d, *J* = 8.0, 1H), 7.62 - 7.54 (m, 1H), 7.39 (d, *J* = 7.3, 1H), 7.32 (t, *J* = 7.5, 1H), 6.82 (dd, *J* = 5.0, 7.3, 1H), 2.31 (s, 3H) |
| | MS (ESI) [M+H] ⁺=236 |
| **32** | ¹H NMR (300 MHz, CDCl₃) δ 8.23 (d, *J* = 8.5, 1H), 8.10 (s, 1H), 7.91 (d, *J* = 8.9, 1H), 7.82 (d, *J* = 8.4, 1H), 7.62 (d, *J* = 8.3, 1H), 7.56 (d, *J* = 7.3, 1H), 7.50 (dd, *J* = 1.8, 8.5, 1H), 7.37 - 7.24 (m, 2H), 2.26 (s, 3H) |
| | MS (ESI) [M+H] ⁺=236 |
| **33** | ¹H NMR (300 MHz, CDCl₃) δ 8.87 (s, 1H), 8.32 (d, *J* = 5.0, 1H), 7.95 (d, *J* = 8.8, 1H), 7.84 (d, *J* = 8.3, 1H), 7.60 (dd, *J* = 7.4, 14.1, 2H), 7.32 (t, *J* = 7.5, 1H), 7.04 (dd, *J* = 5.0, 9.0, 2H) |
| | MS (ESI) [M+H] ⁺=247 |
| **34** | ¹H NMR (300 MHz, CDCl₃) δ 8.52 (s, 1H), 8.45 (d, *J* = 8.6, 1H), 8.01 (d, *J* = 8.8, 1H), 7.87 (dd, *J* = 2.5, 8.5, 2H), 7.72 - 7.56 (m, 2H), 7.39 (d, *J* = 9.0, 2H) |
| | MS (ESI) [M+H] ⁺=290 |
| **35** | ¹H NMR (300 MHz, CDCl₃) δ 8.32 (d, *J* = 9.1, 1H), 8.07 (d, *J* = 4.8, 1H), 7.93 (d, *J* = 9.1, 1H), 7.59 (t, *J* = 7.9, 1H), 7.52 (d, *J* = 8.0, 1H), 7.36 (d, *J* = 7.2, 1H), 7.14 (t, *J* = 7.8, 1H), 6.77 (dd, *J* = 5.0, 7.3, 1H), 2.29 (s, 3H) |
| | MS (ESI) [M+H] ⁺=270 |
| **36** | ¹H NMR (300 MHz, CDCl₃) δ 8.70 (d, *J* = 7.2, 1H), 8.01 (s, 1H), 7.82 (d, *J* = 8.9, 1H), 7.62 (d, *J* = 7.6, 1H), 7.53 (dd, *J* = 1.8, 8.6, 1H), 7.46 (d, *J* = 7.9, 1H), 7.12 (t, *J* = 7.8, 1H), 7.05 (d, *J* = 8.8, 1H), 2.21 (s, 3H) |
| | MS (ESI) [M+H] ⁺=270 |
| **37** | ¹H NMR (300 MHz, CDCl₃) δ 9.08 (d, *J* = 8.5, 1H), 8.55 (s, 1H), 8.36 (s, 1H), 8.02 (d, *J* = 8.1, 2H), 7.77 (d, *J* = 7.2, 1H), 7.62 (d, *J* = 7.6, 1H), 7.35 - 7.24 (m, 1H), 7.12 (d, *J* = 8.8, 1H) |
| | MS (ESI) [M+H] ⁺=324 |
| **38** | ¹H NMR (300 MHz, CDCl₃) δ 8.69 (d, *J* = 9.1, 1H), 7.97 (d, *J* = 9.1, 1H), 7.80 - 7.74 (m, 1H), 7.70 (d, *J* = 8.4, 1H), 7.59 (d, *J* = 8.0, 1H), 7.54 - 7.45 (m, 1H), 7.22 (t, *J* = 7.5, 1H), 6.87 (d, *J* = 7.9, 1H), 6.68 (dd, *J* = 5.0, 7.9, 1H), 3.73 (s, 3H) |
| | MS (ESI) [M+H] ⁺=252 |
| **39** | ¹H NMR (300 MHz, CDCl₃) δ 8.57 (d, *J* = 29.4, 1H), 7.80 (d, *J* = 8.8, 1H), 7.66 (t, *J* = 6.7, 2H), 7.46 (d, *J* = 7.9, 1H), 7.14 (t, *J* = 7.8, 1H), 7.06 (d, *J* = 8.8, 1H), 6.79 (d, *J* = 7.3, 1H), 2.73 (dd, *J* = 7.6, 15.2, 2H), 1.28 (t, *J* = 7.7, 3H) |
| **40** | ¹H NMR (300 MHz, DMSO) δ 9.75 (s, 1H), 9.12 (d, *J* = 2.3, 1H), 8.50 (d, *J* = 2.2, 1H), 8.48 (s, 1H), 8.13 (s, 1H), 7.83 (s, 1H), 7.80 (s, 1H), 7.64 (t, *J* = 7.7, 1H), 7.45 (t, *J* = 7.8, 1H) |
| **41** | ¹H NMR (300 MHz, CDCl₃) δ 8.52 (dd, *J* = 2.8, 8.6, 1H), 8.35 (s, 1H), 8.15 (d, *J* = 2.3, 1H), 7.94 (d, *J* = 8.8, 1H), 7.84 (d, *J* = 8.2, 1H), 7.65 (d, *J* = 7.8, 1H), 7.59 (d, *J* = 7.2, 1H), 7.50 - 7.40 (m, 1H), 7.33 (t, *J* = 7.4, 1H), 7.11 (d, *J* = 8.9, 1H) |
| | MS (ESI) [M+H] ⁺=240 |
| **42** | ¹H NMR (300 MHz, CDCl₃) δ 8.55 (d, *J* = 6.8, 1H), 8.01 (d, *J* = 8.9, 2H), 7.82 (dd, *J* = 9.1, 17.3, 2H), 7.69 (d, *J* = 8.0, 1H), 7.63 (t, *J* = 7.6, 1H), 7.37 (t, *J* = 7.5, 1H), 7.32 - 7.18 (m, 2H) |
| | MS (ESI) [M+H] ⁺=290 |
| **43** | ¹H NMR (300 MHz, DMSO) δ 10.41 (s, 1H), 9.08 (dd, *J* = 4.1, 9.3, 1H), 8.31 (d, *J* = 2.9, 1H), 8.20 (d, *J* = 8.9, 1H), 7.88 - 7.70 (m, 3H), 7.44 (d, *J* = 8.9, 1H), 7.32 (t, *J* = 7.8, 1H) |
| | ¹³C NMR (75 MHz, DMSO) δ 156.30, 153.32, 153.04, 150.17, 142.55, 137.73, 135.06, 134.74, 129.58, 129.49, 126.86, 125.29, 125.14, 125.04, 123.36, 114.91, 113.36. |
| | MS (ESI) [M+H] ⁺=274 |
| **44** | ¹H NMR (300 MHz, CDCl₃) δ 11.09 (s, 1H), 8.78 (d, *J* = 9.0, 1H), 8.42 (dd, *J* = 1.9, 4.7, 1H), 8.28 (dd, *J* = 1.9, 7.8, 1H), 8.11 (d, *J* = 9.1, 1H), 7.73 (d, *J* = 7.5, 1H), 7.65 (d, *J* = 8.1, 1H), 7.27 (dd, *J* = 6.4, 9.2, 1H), 6.88 (dd, *J* = 4.8, 7.8, 1H) |
| | MS (ESI) [M+H] ⁺=300 |
| **46** | ¹H NMR (300 MHz, CDCl₃) δ 8.59 (d, *J* = 8.3, 1H), 7.73 (d, *J* = 8.3, 1H), 7.57 (s, 1H), 7.51 (t, *J* = 7.9, 1H), 7.43 (t, *J* = 9.2, 2H), 7.17 (t, *J* = 7.4, 1H), 6.67 (d, *J* = 7.4, 1H), 2.36 (s, 3H), 2.28 (s, 3H) |
| **47** | ¹H NMR (300 MHz, MeOD) δ 8.99 (s, 1H), 8.76 (d, *J* = 9.2, 1H), 8.32 (d, *J* = 8.7, 1H), 8.22 (d, *J* = 8.6, 1H), 8.11 (d, *J* = 7.8, 1H), 8.01 (t, *J* = 7.1, 1H), 7.76 (t, *J* = 7.4, 1H), 7.55 - 7.43 (m, 2H) |
| | MS (ESI) [M+H] ⁺=247 |
| **48** | ¹H NMR (300 MHz, MeOD) δ 8.48 (d, *J* = 9.1, 1H), 8.40 (d, *J* = 6.7, 1H), 7.94 (d, *J* = 8.4, 1H), 7.90 (d, *J* = 7.8, 1H), 7.54 (t, *J* = 8.0, 1H), 7.38 (d, *J* = 8.6, 1H), 7.30 (s, 2H), 2.58 (s, 3H) |
| | MS (ESI) [M+H] ⁺=270 |
| **49** | ¹H NMR (300 MHz, CDCl₃) δ 9.34 (s, 1H), 8.95 (s, 1H), 8.21 (d, *J* = 5.1, 1H), 7.87 (d, *J* = 8.9, 1H), 7.71 (d, *J* = 7.5, 1H), 7.52 (d, *J* = 7.9, 1H), 7.19 (t, *J* = 7.8, 1H), 7.05 (d, *J* = 8.9, 1H), 6.84 (d, *J* = 5.1, 1H), 2.76 (q, *J* = 7.6, 2H), 1.37 (t, *J* = 7.6, 3H) |
| **50** | ¹H NMR (300 MHz, CDCl₃) δ 8.57 (d, *J* = 29.4, 1H), 7.80 (d, *J* = 8.8, 1H), 7.66 (t, *J* = 6.7, 2H), 7.46 (d, *J* = 7.9, 1H), 7.14 (t, *J* = 7.8, 1H), 7.06 (d, *J* = 8.8, 1H), 6.79 (d, *J* = 7.3, 1H), 2.73 (dd, *J* = 7.6, 15.2, 2H), 1.28 (t, *J* = 7.7, 3H) |
| **51** | ¹H NMR (300 MHz, CDCl₃) δ 8.64 (s, 1H), 8.06 (s, 1H), 7.89 (d, *J* = 8.7, 1H), 7.71 (d, *J* = 7.4, 1H), 7.54 (d, *J* = 7.8, 1H), 7.20 (t, *J* = 7.7, 1H), 7.02 (d, *J* = 8.8, 1H), 6.67 (s, 1H), 2.43 (s, 3H), 2.39 (s, 3H) |
| | ¹³C NMR (75 MHz, CDCl₃) δ 156.15, 153.17, 152.82, 150.16, 143.70, 137.92, 131.34, 129.89, 126.49, 125.47, 123.43, 118.62, 114.47, 111.02, 24.13, 21.70. |
| | MS (ESI) [M+H] ⁺=284 |
| **52** | ¹H NMR (300 MHz, CDCl₃) δ 8.89 (d, *J* = 8.8, 1H), 8.05 (d, *J* = 8.8, 1H), 8.01 (s, 1H), 7.93 (d, *J* = 8.8, 1H), 7.79 (d, *J* = 7.5, 1H), 7.64 (d, *J* = 8.0, 1H), 7.32 (t, *J* = 7.8, 1H), 7.13 (d, *J* = 8.8, 1H), 2.67 (s, 3H) |
| **53** | ¹H NMR (300 MHz, CDCl₃) δ 9.27 (s, 1H), 8.33 (d, *J* = 5.7, 1H), 8.13 (d, *J* = 5.2, 1H), 8.00 (d, *J* = 8.8, 1H), 7.76 (d, *J* = 7.4, 1H), 7.60 (d, *J* = 8.0, 1H), 7.29 (d, *J* = 7.9, 1H), 7.07 (d, *J* = 8.9, 1H), 6.97 (d, *J* = 4.8, 1H) |
| **54** | MS (ESI) [M+H] ⁺=250 |
| **55** | ¹H NMR (300 MHz, CDCl₃) δ 8.19 (s, 1H), 7.90 (d, *J* = 9.0, 1H), 7.63 (d, *J* = 7.5, 1H), 7.52 (d, *J* = 7.9, 1H), 7.33 (d, *J* = 7.4, 1H), 7.14 (t, *J* = 7.8, 1H), 6.69 (d, *J* = 7.5, 1H), 2.70 (dd, *J* = 7.3, 14.8, 2H), 2.47 (s, 3H), 1.26 (t, *J* = 7.7, 3H) |
| **56** | ¹H NMR (300 MHz, CDCl₃) δ 8.20 (s, 1H), 7.90 (d, *J* = 9.0, 1H), 7.63 (d, *J* = 7.5, 1H), 7.52 (d, *J* = 7.9, 1H), 7.33 (d, *J* = 7.4, 1H), 7.14 (t, *J* = 7.8, 1H), 6.69 (d, *J* = 7.5, 1H), 2.70 (dd, *J* = 7.3, 14.8, 2H), 2.47 (s, 3H), 1.25 (dd, *J* = 7.5, 15.5, 3H) |
| **57** | MS (ESI) [M+H] ⁺=253 |
| **58** | MS (ESI) [M+H] ⁺=314-316 |
| **59** | ¹H NMR (300 MHz, CDCl₃) δ 8.91 (d, *J* = 1.7, 1H), 8.46 (d, *J* = 8.8, 1H), 8.28 (dd, *J* = 2.0, 8.8, 1H), 8.23 (s, 1H), 8.03 (d, *J* = 8.8, 1H), 7.88 (d, *J* = 8.3, 1H), 7.70 (d, *J* = 8.0, 1H), 7.67 - 7.58 (m, 1H), 7.38 (t, *J* = 7.4, 1H), 7.32 (d, *J* = 8.8, 2H), 3.91 (s, 3H) |
| **60** | ¹H NMR (300 MHz, CDCl₃) δ 8.94 (d, *J* = 8.9, 1H), 8.91 (d, *J* = 1.8, 1H), 8.37 (dd, *J* = 2.2, 8.8, 1H), 8.04 (d, *J* = 8.9, 2H), 7.77 (d, *J* = 7.5, 1H), 7.62 (d, *J* = 7.2, 1H), 7.30 (t, *J* = 7.8, 2H), 7.19 (d, *J* = 8.8, 2H), 3.92 (s, 3H) |
| **61** | ¹H NMR (300 MHz, CDCl₃) δ 8.96 (d, *J* = 8.8, 1H), 8.85 (d, *J* = 1.3, 1H), 8.28 (d, *J* = 9.9, 1H), 7.84 (d, *J* = 8.0, 1H), 7.77 (s, 1H), 7.65 (s, 1H), 7.59 (d, *J* = 8.4, 2H), 7.53 (d, *J* = 8.4, 1H), 7.31 (t, *J* = 7.4, 1H), 3.88 (s, 4H), 2.42 (s, 4H) |
| | MS (ESI) [M+H] ⁺=294 |
| **62** | ¹H NMR (300 MHz, CDCl₃) δ 11.02 (s, 1H), 8.75 (d, *J* = 9.2, 1H), 8.44 (d, *J* = 3.7, 1H), 8.31 (d, *J* = 7.9, 1H), 8.10 (d, *J* = 9.0, 1H), 7.72 (d, *J* = 7.5, 1H), 7.64 (d, *J* = 8.2, 1H), 7.27 (d, *J* = 8.1, 1H), 6.88 (dd, *J* = 4.7, 7.8, 1H), 3.97 (s, 3H) |
| | MS (ESI) [M+H] ⁺=314 |
| **63** | MS (ESI) [M+H] ⁺=266 |
| **64** | ¹H NMR (300 MHz, DMSO) δ 10.38 (s, 1H), 8.56 (s, 1H), 8.28 (d, *J* = 9.1, 1H), 8.20 - 8.03 (m, 3H), 7.50 (d, *J* = 8.7, 1H), 7.45 (d, *J* = 8.0, 1H), 6.88 (d, *J* = 4.4, 1H), 2.37 (s, 3H) |
| **65** | MS (ESI) [M+H] ⁺=314-316 |
| **66** | MS (ESI) [M+H] ⁺=250 |
| **67** | ¹H NMR (300 MHz, DMSO) δ 10.51 (s, 1H), 8.83 (d, *J* = 2.3, 1H), 8.62 (d, *J* = 9.3, 1H), 8.24 (dd, *J* = 2.7, 9.1, 1H), 7.96 (d, *J* = 8.9, 1H), 7.81 (d, *J* = 7.8, 1H), 7.67 (t, *J* = 7.6, 1H), 7.45 (d, *J* = 11.2, 2H), 3.86 (s, 3H), 2.62 (s, 3H) |
| | MS (ESI) [M+H] ⁺=294 |
| **68** | ¹H NMR (300 MHz, CDCl₃) δ 9.57 (s, 1H), 8.44 (d, *J* = 4.8, 1H), 8.05 (d, *J* = 8.8, 1H), 7.86 (s, 1H), 7.80 (d, *J* = 7.5, 1H), 7.64 (d, *J* = 8.0, 1H), 7.31 (t, *J* = 7.8, 1H), 7.19 (d, *J* = 4.3, 1H), 7.04 (d, *J* = 8.8, 1H) |
| **69** | ¹H NMR (300 MHz, CDCl₃) δ 9.12 (s, 1H), 7.94 (d, *J* = 8.6, 1H), 7.71 (d, *J* = 7.5, 1H), 7.57 (d, *J* = 7.8, 1H), 7.40 (s, 1H), 7.25 (d, *J* = 10.2, 2H), 7.17 (s, 1H), 7.05 (s, 1H) |
| **70** | ¹H NMR (300 MHz, CDCl₃) δ 9.07 (d, *J* = 8.5, 1H), 7.97 (d, *J* = 8.8, 1H), 7.90 (t, *J* = 8.0, 1H), 7.84 (s, 1H), 7.75 (dd, *J* = 1.1, 7.5, 1H), 7.62 - 7.55 (m, 1H), 7.31 (d, *J* = 7.6, 1H), 7.27 (t, *J* = 7.8, 1H), 7.08 (d, *J* = 8.8, 1H) |
| | MS (ESI) [M+H] ⁺=274 |
| **71** | MS (ESI) [M+H] ⁺=274 |
| **72** | ¹H NMR (300 MHz, CDCl₃) δ 8.67 (d, *J* = 7.9, 1H), 7.83 (d, *J* = 8.3, 1H), 7.71 (s, 1H), 7.69 - 7.61 (m, 1H), 7.57 (d, *J* = 7.9, 2H), 7.52 (d, *J* = 7.1, 1H), 7.28 (t, *J* = 7.4, 1H), 2.74 (q, *J* = 7.6, 2H), 2.42 (s, 3H), 1.31 (t, *J* = 7.6, 3H) |
| | MS (ESI) [M+H] ⁺=264 |
| **73** | ¹H NMR (300 MHz, CDCl₃) δ 8.91 (dd, *J* = 3.8, 9.0, 1H), 8.11 (d, *J* = 2.9, 1H), 7.81 (d, *J* = 8.3, 1H), 7.71 (s, 1H), 7.56 (dd, *J* = 7.4, 14.1, 2H), 7.51 - 7.42 (m, 1H), 7.29 (d, *J* = 7.2, 1H), 2.38 (s, 3H) |
| | MS (ESI) [M+H] ⁺=254 |
| **74** | ¹H NMR (300 MHz, CDCl₃) δ 8.96 (d, *J* = 8.3, 1H), 8.49 (s, 1H), 7.89 (dd, *J* = 1.9, 9.0, 1H), 7.82 (d, *J* = 8.2, 1H), 7.72 (s, 1H), 7.57 (t, *J* = 8.7, 3H), 7.33 (t, *J* = 7.4, 1H), 2.37 (s, 3H) |
| | MS (ESI) [M+H] ⁺=304 |
| **75** | ¹H NMR (300 MHz, CDCl₃) δ 7.83 (d, *J* = 9.0, 1H), 7.69 (dd, *J* = 1.3, 7.6, 1H), 7.53 (dd, *J* = 1.2, 8.0, 1H), 7.42 (d, *J* = 8.9, 2H), 7.15 (t, *J* = 7.8, 1H), 6.89 (d, *J* = 8.9, 2H), 6.79 (d, *J* = 8.9, 2H), 2.97 (s, 6H) |
| **77** | ¹H NMR (300 MHz, CDCl₃) δ 7.83 (d, *J* = 8.8, 1H), 7.70 (d, *J* = 7.6, 1H), 7.59 (d, *J* = 8.6, 2H), 7.52 (d, *J* = 7.3, 1H), 7.16 (t, *J* = 7.7, 1H), 6.94 (d, *J* = 8.4, 3H), 6.86 (d, *J* = 8.8, 1H), 3.82 (s, 3H) |
| | ¹³C NMR (75 MHz, CDCl₃) δ 156.40, 155.54, 144.29, 138.09, 132.96, 130.44, 129.99, 126.61, 125.22, 123.29, 122.66, 114.73, 112.16, 55.74. |
| | MS (ESI) [M+H] ⁺=285 |
| **78** | ¹H NMR (300 MHz, CDCl₃) δ 7.80 (t, *J* = 7.6, 2H), 7.64 (d, *J* = 8.9, 2H), 7.61 - 7.55 (m, 1H), 7.33 (t, *J* = 7.6, 1H), 7.19 (d, *J* = 8.7, 2H), 2.59 (s, 3H) |
| **79** | ¹H NMR (300 MHz, CDCl₃) δ 7.78 (d, *J* = 8.4, 1H), 7.76 - 7.71 (m, 2H), 7.69 (s, 1H), 7.57 (dd, *J* = 1.1, 8.0, 1H), 7.51 (ddd, *J* = 1.5, 7.0, 8.4, 1H), 7.29 - 7.21 (m, 1H), 6.96 - 6.90 (m, 2H), 3.82 (s, 3H), 2.35 (s, 3H) |
| **80** | ¹H NMR (300 MHz, CDCl₃) δ 7.92 (d, *J* = 8.9 Hz, 2H), 7.84 (d, *J* = 8.3 Hz, 1H), 7.78 (s, 1H), 7.62 (d, *J* = 8.0 Hz, 1H), 7.57 (t, *J* = 7.7 Hz, 1H), 7.32 (t, *J* = 7.4 Hz, 1H), 7.24 (d, *J* = 8.7 Hz, 2H), 6.53 (s, 1H), 2.42 (s, 3H) |
| | ¹³C NMR (75 MHz, CDCl₃) δ 152.46, 146.25, 143.86, 139.33, 136.83, 128.93, 126.96, 126.71, 124.75, 123.56, 121.88, 120.44, 119.95, 17.77. |
| | MS (ESI) [M+H] ⁺=319 |
| **81** | ¹H NMR (300 MHz, CDCl₃) δ 7.75 (d, *J* = 8.3, 1H), 7.66 (d, *J* = 8.5, 3H), 7.55 (d, *J* = 7.8, 1H), 7.48 (t, *J* = 7.6, 1H), 7.20 (d, *J* = 7.2, 1H), 6.80 (d, *J* = 8.8, 2H), 6.32 (s, 1H), 2.93 (s, 7H), 2.35 (s, 3H) |
| **82** | ¹H NMR (300 MHz, CDCl₃) δ 7.92 (d, *J* = 8.9, 1H), 7.82 - 7.70 (m, 2H), 7.66 (d, *J* = 7.8, 1H), 7.59 (t, *J* = 7.6, 1H), 7.30 (dd, *J* = 6.0, 13.5, 1H), 7.14 (s, 1H), 7.11 (s, 1H), 6.84 (d, *J* = 8.9, 1H), 2.32 (s, 3H) |
| | MS (ESI) [M+H] ⁺=319 |
| **83** | ¹H NMR (300 MHz, CDCl₃) δ 7.93 - 7.86 (m, 1H), 7.85 (s, 1H), 7.82 (d, *J* = 8.4, 1H), 7.59 (dd, *J* = 8.2, 15.5, 2H), 7.44 - 7.38 (m, 1H), 7.29 (dd, *J* = 8.3, 16.8, 2H), 6.91 (d, *J* = 9.0, 1H), 6.87 (d, *J* = 8.3, 1H) |
| | MS (ESI) [M+H] ⁺=305 |
| **84** | ¹H NMR (300 MHz, CDCl₃) δ 8.67 (d, *J* = 8.1, 1H), 7.92 (d, *J* = 8.9, 1H), 7.85 (d, *J* = 8.4, 1H), 7.63 (d, *J* = 7.6, 1H), 7.58 (d, *J* = 7.3, 1H), 7.30 (dd, *J* = 6.8, 14.8, 3H), 7.02 (t, *J* = 7.8, 1H), 6.89 (d, *J* = 8.9, 1H) |
| | MS (ESI) [M+H] ⁺=305 |
| **86** | ¹H NMR (300 MHz, CDCl₃) δ 7.93 (d, *J* = 8.9, 1H), 7.83 (d, *J* = 8.3, 1H), 7.70 (d, *J* = 12.0, 1H), 7.61 (dd, *J* = 7.9, 18.1, 2H), 7.32 (d, *J* = 7.9, 1H), 7.31 - 7.25 (m, 1H), 7.21 (t, *J* = 6.5, 1H), 6.92 (d, *J* = 8.9, 1H), 6.79 - 6.68 (m, 1H) |
| | MS (ESI) [M+H] ⁺=239 |
| **87** | ¹H NMR (300 MHz, CDCl₃) δ 8.27 (s, 1H), 7.76 (d, *J* = 8.9, 1H), 7.67 (d, *J* = 7.5, 1H), 7.51 (d, *J* = 8.2, 1H), 7.45 (d, *J* = 7.9, 1H), 7.28 (d, *J* = 8.2, 1H), 7.14 (t, *J* = 7.8, 1H), 6.86 (d, *J* = 10.1, 1H), 6.76 (d, *J* = 8.9, 1H) |
| | MS (ESI) [M+H] ⁺=339 |
| **88** | ¹H NMR (300 MHz, CDCl₃) δ 8.11 (dt, *J* = 2.1, 12.1, 1H), 7.76 (d, *J* = 8.9, 1H), 7.66 (dd, *J* = 1.2, 7.6, 1H), 7.45 (dd, *J* = 1.1, 8.0, 1H), 7.22 (dd, *J* = 1.4, 7.2, 2H), 7.18 (d, *J* = 7.6, 1H), 7.12 (d, *J* = 7.8, 1H), 6.75 (d, *J* = 8.9, 1H), 6.69 (d, *J* = 7.9, 1H) |
| | MS (ESI) [M+H] ⁺=273 |
| **89** | ¹H NMR (300 MHz, DMSO) δ 11.38 (s, 1H), 8.41 (d, *J* = 9.1, 1H), 7.93 (d, *J* = 7.8, 1H), 7.80 (dt, *J* = 8.1, 20.9, 4H), 7.50 (d, *J* = 7.8, 3H), 7.36 (d, *J* = 9.3, 1H) |
| **90** | ¹H NMR (300 MHz, CDCl₃) δ 7.84 (d, *J* = 9.1, 2H), 7.79 (d, *J* = 8.9, 1H), 7.67 (dd, *J* = 1.2, 7.6, 1H), 7.48 (dd, *J* = 1.1, 8.0, 1H), 7.18 (s, 3H), 6.89 (s, 1H), 6.75 (d, *J* = 8.9, 1H) |
| | ¹³C NMR (75 MHz, CDCl₃) δ 153.88, 144.30, 143.91, 139.00, 138.25, 131.13, 130.13, 126.55, 125.42, 123.45, 122.50, 122.17, 120.49, 119.10, 113.24. |
| | MS (ESI) [M+H] ⁺=339 |
| **91** | ¹H NMR (300 MHz, CDCl₃) δ 8.74 (s, 1H), 8.54 (s, 1H), 8.46 (d, *J* = 8.8, 1H), 7.91 (dd, *J* = 5.5, 14.5, 2H), 7.79 (d, *J* = 8.9, 1H), 7.67 (d, *J* = 2.1, 1H), 7.56 (dd, *J* = 2.3, 8.9, 1H), 7.35 (d, *J* = 8.9, 1H) |
| **92** | ¹H NMR (300 MHz, CDCl₃) δ 8.67 (d, *J* = 7.9, 1H), 7.83 (d, *J* = 8.3, 1H), 7.71 (s, 1H), 7.69 - 7.61 (m, 1H), 7.55 (dd, *J* = 7.5, 14.4, 2H), 7.29 (d, *J* = 7.8, 1H), 6.80 (d, *J* = 7.4, 1H) |
| **93** | ¹H NMR (300 MHz, CDCl₃) δ 9.21 (dd, *J* = 1.5, 8.4, 1H), 7.85 (d, *J* = 8.4, 1H), 7.73 (s, 1H), 7.58 (d, *J* = 7.8, 1H), 7.53 (dd, *J* = 1.3, 8.3, 1H), 7.40 - 7.35 (m, 1H), 7.32 (dd, *J* = 1.1, 4.6, 1H), 7.31 - 7.24 (m, 2H), 7.04 (s, 1H), 7.02 - 6.94 (m, 1H), 2.38 (s, 3H) |
| **94** | ¹H NMR (300 MHz, CDCl₃) δ 8.16 (d, *J* = 8.7, 1H), 7.83 (d, *J* = 8.9, 1H), 7.63 (d, *J* = 7.6, 1H), 7.48 (d, *J* = 8.0, 1H), 7.13 (t, *J* = 7.8, 1H), 7.08 (s, 1H), 7.04 (s, 2H), 6.81 (d, *J* = 8.9, 2H), 2.27 (s, 3H) |
| | MS (ESI) [M+H] ⁺=353 |
| **95** | ¹H NMR (300 MHz, MeOD) δ 8.42 (s, 1H), 7.94 (d, *J* = 7.9, 1H), 7.83 (d, *J* = 8.1, 1H), 7.78 (d, *J* = 7.1, 1H), 7.72 (d, *J* = 8.7, 2H), 7.58 (d, *J* = 8.2, 3H), 2.60 (s, 3H) |
| | MS (ESI) [M+H] ⁺=319 |
| **96** | ¹H NMR (300 MHz, CDCl₃) δ 7.79 (d, *J* = 8.9, 1H), 7.70 (d, *J* = 8.9, 1H), 7.64 (d, *J* = 8.9, 2H), 7.59 (d, *J* = 2.1, 1H), 7.50 (dd, *J* = 2.3, 8.9, 1H), 7.19 (d, *J* = 8.6, 2H), 6.85 (d, *J* = 8.9, 1H) |
| | MS (ESI) [M+H] ⁺=281 |
| **97** | ¹H NMR (300 MHz, MeOD) δ 8.11 (d, *J* = 8.4, 1H), 7.81 (s, 2H), 7.62 (d, *J* = 8.7, 3H), 7.51 (d, *J* = 8.3, 2H), 7.12 (s, 1H), 2.77 (s, 3H) |
| | MS (ESI) [M+H] ⁺=319 |
| **98** | MS (ESI) [M+H] ⁺=383-385 |
| **99** | MS (ESI) [M+H] ⁺=320 |
| **100** | MS (ESI) [M+H] ⁺=316 |
| **101** | ¹H NMR (300 MHz, CDCl₃) δ 7.82 (d, *J* = 8.9, 1H), 7.70 - 7.63 (m, 1H), 7.51 (dd, *J* = 5.3, 7.6, 3H), 7.14 (t, *J* = 7.8, 1H), 6.91 (d, *J* = 8.8, 3H), 6.85 (d, *J* = 9.0, 2H), 3.96 (t, *J* = 6.5, 2H), 1.84 - 1.68 (m, 3H), 1.49 (dd, *J* = 7.4, 15.0, 3H), 0.97 (t, *J* = 7.4, 3H) |
| | MS (ESI) [M+H] ⁺=327 |
| **102** | ¹H NMR (300 MHz, CDCl₃) δ 7.89 (d, *J* = 8.9, 1H), 7.76 (d, *J* = 8.5, 1H), 7.63 (d, *J* = 8.1, 1H), 7.59 (s, 1H), 7.54 (d, *J* = 8.8, 2H), 7.38 - 7.24 (m, 3H), 7.09 (d, *J* = 7.4, 1H), 7.02 (dd, *J* = 2.4, 8.8, 4H), 6.90 (d, *J* = 8.9, 1H) |
| | MS (ESI) [M+H] ⁺=313 |
| **103** | MS (ESI) [M+H] ⁺=334 |
| **104** | ¹H NMR (300 MHz, CDCl₃) δ 8.49 (d, *J* = 2.5, 1H), 7.89 (d, *J* = 8.8, 1H), 7.72 (d, *J* = 7.6, 1H), 7.63 (dd, *J* = 2.5, 8.9, 1H), 7.53 (d, *J* = 8.0, 1H), 7.23 (dd, *J* = 6.2, 14.0, 2H), 7.04 (s, 1H), 6.81 (d, *J* = 8.8, 1H) |
| | MS (ESI) [M+H] ⁺=373 |
| **105** | ¹H NMR (300 MHz, CDCl₃) δ 8.85 (d, *J* = 2.6, 1H), 8.45 (d, *J* = 2.3, 1H), 8.01 (d, *J* = 8.1, 1H), 7.71 (d, *J* = 7.8, 1H), 7.58 (s, 1H), 7.53 (d, *J* = 7.6, 1H), 7.51 - 7.45 (m, 2H), 7.45 - 7.36 (m, 1H), 6.72 - 6.62 (m, 2H), 2.48 (s, 3H) 13C NMR (75 MHz, CDCl₃) δ 157.18, 154.80, 145.42, 143.80, 138.17, 135.04, 128.88, 128.76, 127.17, 127.04, 120.69, 115.22, 106.73, 24.38 |
| **106** | ¹H NMR (300 MHz, DMSO) δ 10.24 (s, 1H), 9.06 (d, *J* = 2.3, 1H), 8.65 (d, *J* = 1.8, 1H), 8.60 (d, *J* = 8.3, 1H), 8.56 (d, *J* = 4.5, 1H), 7.97 (dd, *J* = 8.2, 14.4, 2H), 7.69 (t, *J* = 6.9, 1H), 7.59 (t, *J* = 7.4, 1H), 7.08 (dd, *J* = 4.6, 8.3, 1H) |
| | MS (ESI) [M+H] ⁺=267 |
| **107** | NMR (300 MHz, CDCl₃) δ 8.77 (dd, *J* = 1.5, 4.3, 1H), 8.06 (dd, *J* = 10.8, 18.4, 3H), 7.93 (d, *J* = 2.4, 1H), 7.57 (dd, *J* = 2.4, 9.0, 1H), 7.39 (ddd, *J* = 3.1, 8.3, 12.5, 3H), 6.93 (d, *J* = 8.4, 1H), 6.89 (s, 1H), 2.29 (s, 3H) |
| **108** | ¹H NMR (300 MHz, CDCl₃) δ 8.72 (dd, *J* = 1.6, 4.2, 1H), 8.61 (d, *J* = 2.4, 1H), 8.11 (d, *J* = 8.3, 1H), 8.00 (d, *J* = 9.0, 1H), 7.91 (dd, *J* = 1.2, 5.0, 1H), 7.69 (dd, *J* = 2.4,9.1, 1H), 7.35 -7.26 (m, 2H), 7.01 (dd, *J* = 1.2, 7.9, 1H), 6.77 (dd, *J* = 5.1, 7.8, 1H), 3.93 (s, 3H) |
| **109** | ¹H NMR (300 MHz, CDCl₃) δ 9.68 (s, 1H), 8.21 (s, 2H), 7.94 (d, *J* = 8.9, 1H), 7.79 (d, *J* = 9.2, 1H), 7.67 (d, *J* = 2.3, 1H), 7.56 (dd, *J* = 2.3, 8.9, 1H), 7.34 (d, *J* = 8.9, 1H) |
| | MS (ESI) [M+H] ⁺=257 |
| **110** | 1H NMR (300 MHz, CDCl₃) δ 10.32 (s, 1H), 8.33 - 8.21 (m, 2H), 8.05 (d, J = 8.9, 1H), 8.00 (dd, J = 1.2, 7.6, 1H), 7.69 (dd, J = 1.1, 7.8, 1H), 7.61 (s, 1H), 7.30 - 7.22 (m, 3H), 7.16 (d, J = 8.8, 1H). |
| | MS (ESI) [M+H] ⁺=301-303 |
| **111** | ¹H NMR (300 MHz, CDCl₃) δ 7.82 (d, *J* = 8.9, 1H), 7.70 - 7.63 (m, 1H), 7.51 (dd, *J* = 5.3, 7.6, 3H), 7.14 (t, *J* = 7.8, 1H), 6.91 (d, *J* = 8.8, 3H), 6.85 (d, *J* = 9.0, 2H), 3.96 (t, *J* = 6.5, 2H), 1.84 - 1.68 (m, 3H), 1.49 (dd, *J* = 7.4, 15.0, 3H), 0.97 (t, *J* = 7.4, 3H) |
| **112** | ¹H NMR (300 MHz, CDCl₃) δ 7.89 (d, *J* = 8.9, 1H), 7.76 (d, *J* = 8.5, 1H), 7.63 (d, *J* = 8.1, 1H), 7.59 (s, 1H), 7.54 (d, *J* = 8.8, 2H), 7.38 - 7.24 (m, 3H), 7.09 (d, *J* = 7.4, 1H), 7.02 (dd, *J* = 2.4, 8.8, 4H), 6.90 (d, *J* = 8.9, 1H) |
| | ¹³C NMR (75 MHz, DMSO) δ 152.94, 150.19, 142.48, 142.18, 138.20, 137.55, 135.74, 129.71, 126.99, 125.35, 123.84, 114.75. |
| | MS (ESI) [M+H] ⁺=255 |
| **113** | ¹H NMR (300 MHz, CDCl₃) δ 9.74 (s, 1H), 8.20 (s, 2H), 8.03 (d, *J* = 8.6, 1H), 7.87 (d, *J* = 7.6, 1H), 7.80 (s, 1H), 7.70 (d, *J* = 8.0, 1H), 7.63 (t, *J* = 7.7, 1H), 7.37 (t, *J* = 7.4, 1H), 7.30 (d, *J* = 8.7, 1H) |
| **114** | ¹H NMR (300 MHz, CDCl₃) δ 9.67 (s, 1H), 8.34 - 8.12 (m, 2H), 7.84 (d, *J* = 8.0, 2H), 7.70 - 7.54 (m, 1H), 7.38 (t, *J* = 7.6, 1H), 7.17 (s, 1H), 2.61 (s, 3H) |
| | MS (ESI) [M+H] ⁺=237 |
| **115** | ¹H NMR (300 MHz, CDCl₃) δ 10.15 (s, 1H), 8.24 - 8.12 (m, 2H), 7.79 (s, 1H), 7.71 (s, 1H), 7.55 (t, *J* = 8.3, 2H), 7.30 (t, *J* = 7.9, 1H), 2.38 (s, 3H) |
| | MS (ESI) [M+H] ⁺=237 |
| **116** | MS (ESI) [M+H] ⁺=240 |
| **117** | MS (ESI) [M+H] ⁺=253 |
| **118** | MS (ESI) [M+H] ⁺=222 |
| **119** | MS (ESI) [M+H] ⁺=256 |
| **121** | MS (ESI) [M+H] ⁺=222 |
| **124** | ¹H NMR (300 MHz, CDCl₃) δ 8.42 (s, 1H), 7.95 (dd, *J* = 1.3, 8.2, 1H), 7.87 - 7.78 (m, 3H), 7.70 - 7.61 (m, 1H), 7.55 - 7.47 (m, 1H), 7.26 (dd, *J* = 2.4, 6.5, 3H), 6.90 (s, 1H) |
| | MS (ESI) [M+H] ⁺=306 |
| **125** | ¹H NMR (300 MHz, CDCl₃) δ 8.42 (s, 1H), 8.03 (d, *J* = 9.5, 1H), 7.92 (d, *J* = 8.2, 1H), 7.73 (d, *J* = 8.2, 1H), 7.61 (t, *J* = 7.3, 1H), 7.46 (t, *J* = 7.2, 1H), 7.13 (s, 2H), 6.84 (s, 1H), 2.35 (s, 3H) |
| **126** | ¹H NMR (300 MHz, CDCl₃) δ 8.40 (s, 1H), 8.03 (s, 1H), 7.94 (d, *J* = 8.2, 1H), 7.84 (d, *J* = 8.2, 1H), 7.65 (t, *J* = 7.4, 1H), 7.53 (d, *J* = 7.1, 1H), 7.48 (d, *J* = 7.2, 1H), 7.35 (t, *J* = 8.2, 1H), 7.22 (s, 1H), 6.94 (d, *J* = 8.1, 1H) |
| **127** | ¹H NMR (300 MHz, CDCl₃) δ 8.85 (dd, *J* = 1.0, 8.3, 1H), 8.47 (s, 1H), 7.96 (d, *J* = 8.2, 1H), 7.85 (d, *J* = 8.3, 1H), 7.72 - 7.61 (m, 1H), 7.57 - 7.47 (m, 1H), 7.42 - 7.36 (m, 1H), 7.33 (d, *J* = 10.0, 1H),7.14(s, 1H), 7.13 - 7.04 (m, 1H) |
| **128** | ¹H NMR (300 MHz, CDCl₃) δ 9.17 (s, 1H), 8.68 (d, *J* = 9.1, 1H), 8.64 (d, *J* = 4.8, 2H), 8.15 (d, *J* = 9.1, 1H), 7.87 (d, *J* = 8.4, 1H), 7.76 (d, *J* = 8.1, 1H), 7.64 (t, *J* = 7.7, 1H), 7.39 (t, *J* = 7.5, 1H), 6.87 (t, *J* = 4.8, 1H) |
| | ¹³C NMR (75 MHz, CDCl3) δ 158.34, 138.07, 129.85, 127.63, 127.31, 124.34, 114.20, 113.90. |
| **129** | ¹H NMR (300 MHz, CDCl₃) δ 9.14 (s, 1H), 8.73 (d, *J* = 21.2, 3H), 8.17 (s, 1H), 7.73 (d, *J* = 20.3, 2H), 7.28 (d, *J* = 9.6, 2H), 6.91 (s, 1H) |
| **130** | ¹H NMR (300 MHz, CDCl₃) δ 9.05 (s, 1H), 8.64 (d, *J* = 4.8, 2H), 8.52 (s, 1H), 7.89 (dd, *J* = 8.5, 14.6, 2H), 7.63 (t, *J* = 7.5, 1H), 7.41 (t, *J* = 7.4, 1H), 6.86 (t, *J* = 4.8, 1H), 2.74 (s, 3H) |
| | MS (ESI) [M+H] ⁺=237 |
| **132** | ¹H NMR (300 MHz, CDCl₃) δ 8.86 (d, *J* = 2.6, 1H), 8.70 (d, *J* = 2.5, 1H), 8.32 (d, *J* = 1.1, 1H), 8.25 - 8.21 (m, 1H), 8.10 (d, *J* = 2.7, 1H), 8.06 (d, *J* = 8.3, 1H), 7.82 (dd, *J* = 1.2, 7.9, 1H), 7.66 - 7.51 (m, 3H), 6.89 (s, 1H) |
| **135** | ¹H NMR (300 MHz, CDCl₃) δ 9.09 (s, 1H), 8.71 (s, 1H), 8.54 (d, *J* = 8.4, 1H), 8.37 (dd, *J* = 1.0, 4.9, 1H), 7.96 (d, *J* = 8.2, 1H), 7.85 (d, *J* = 8.3, 1H), 7.82 - 7.74 (m, 1H), 7.66 (t, *J* = 7.6, 1H), 7.52 (dd, *J* = 7.0, 8.1, 1H), 7.02 (dd, *J* = 5.0, 7.2, 1H) |
| | MS (ESI) [M+H] ⁺=223 |
| **136** | ¹H NMR (300 MHz, CDCl₃) δ 9.02 (s, 1H), 8.70 (s, 1H), 8.30 (s, 1H), 8.20 (d, *J* = 5.1, 1H), 7.94 (d, *J* = 8.1, 1H), 7.84 (d, *J* = 8.2, 1H), 7.64 (t, *J* = 7.6, 1H), 7.49 (t, *J* = 8.1, 1H), 6.83 (d, *J* = 5.0, 1H), 2.43 (s, 3H) |
| | ¹³C NMR (75 MHz, CDCl₃) δ 153.28, 150.20, 148.55, 147.40, 140.93, 139.83, 138.35, 130.44, 129.16, 127.18, 126.28, 119.70, 113.75, 21.87. |
| | MS (ESI) [M+H] ⁺=237 |
| **137** | ¹H NMR (300 MHz, DMSO) δ 11.10 (s, 1H), 9.03 (s, 1H), 8.82 - 8.75 (m, 1H), 8.56 (d, *J* = 8.9, 1H), 8.24 (dd, *J* = 2.3, 8.9, 1H), 7.96 (dd, *J* = 1.2, 8.2, 1H), 7.87 (dd, *J* = 1.0, 8.3, 1H), 7.79 - 7.71 (m, 1H), 7.61 (ddd, *J* = 1.4, 7.0, 8.3, 1H) |
| | MS (ESI) [M+H] ⁺=248 |
| **138** | ¹H NMR (300 MHz, CDCl₃) δ 8.72 (s, 1H), 8.53 (s, 1H), 8.20 (d, *J* = 8.3, 1H), 7.93 (d, *J* = 8.2, 1H), 7.81 (d, *J* = 8.3, 1H), 7.62 (td, *J* = 3.4, 8.1, 2H), 7.53 - 7.43 (m, 1H), 6.83 (d, *J* = 7.4, 1H), 2.48 (s, 3H) |
| | ¹³C NMR (75 MHz, CDCl₃) δ 156.86, 152.27, 148.40, 140.92, 139.70, 139.00, 138.35, 130.42, 129.13, 127.14, 126.27, 117.76, 110.01, 24.15. |
| | MS (ESI) [M+H] ⁺=237 |
| **139** | ¹H NMR (300 MHz, CDCl₃) δ 8.53 (s, 1H), 8.20 (d, *J* = 4.8, 1H), 8.04 (d, *J* = 8.3, 1H), 7.92 (d, *J* = 8.4, 1H), 7.87 (s, 1H), 7.79 (t, *J* = 7.6, 1H), 7.60 (t, *J* = 7.6, 1H), 6.88 (d, *J* = 4.7, 1H), 2.46 (s, 3H) |
| **140** | ¹H NMR (300 MHz, CDCl₃) δ 9.93 (s, 1H), 8.19 (s, 1H), 8.05 (d, *J* = 8.1, 1H), 7.99 (s, 1H), 7.82 (d, *J* = 8.2, 1H), 7.69 (t, *J* = 7.6, 1H), 7.59 (t, *J* = 8.2, 1H), 2.53 (s, 4H) |
| **141** | ¹H NMR (300 MHz, CDCl₃) δ 9.72 (s, 1H), 9.35 (s, 1H), 8.30 (d, *J* = 5.0, 1H), 8.05 (d, *J* = 7.7, 1H), 7.87 (d, *J* = 7.0, 1H), 7.66 (dd, *J* = 7.4, 16.9, 3H), 6.92 (d, *J* = 4.9, 1H), 2.58 (s, 3H) |
| **143** | ¹H NMR (300 MHz, DMSO) δ 8.85 (s, 1H), 8.42 (d, *J* = 5.3, 1H), 7.96 (d, *J* = 9.1, 1H), 7.44 (s, 1H), 7.30 (s, 4H), 7.28 - 7.21 (m, 2H), 6.66 (d, *J* = 5.3, 1H), 2.99 (s, 6H) |
| | ¹³C NMR (75 MHz, DMSO) δ 156.82, 150.25, 149.69, 143.79, 141.71, 125.95, 122.33, 118.88, 117.37, 115.95, 109.39, 104.92, 43.57 |
| | MS (ESI) [M+H]+ = 348 |
| **144** | MS (ESI) [M+H] ⁺=390 |
| **145** | MS (ESI) [M+H] ⁺=252 |
| **146** | ¹H NMR (300 MHz, DMSO) δ 9.34 (s, 1H), 8.59 (d, *J* = 5.2, 1H), 8.53 (s, 1H), 8.13 (d, *J* = 5.1, 1H), 7.98 (d, *J* = 9.0, 1H), 7.66 (d, *J* = 9.1, 1H), 6.80 (d, *J* = 5.2, 1H), 6.76 (s, 1H), 6.69 (d, *J* = 4.9, 1H), 4.00 (s, 3H), 2.26 (s, 3H) |
| | ¹³C NMR (75 MHz, DMSO) δ 161.31, 155.67, 151.63, 150.25, 147.77, 147.01, 142.97, 121.56, 119.16, 116.61, 114.75, 112.60, 111.41, 98.91, 55.78, 20.66. |
| | MS (ESI) [M+H]+= 266 |
| **147** | MS (ESI) [M+H] ⁺=279 |
| **149** | MS (ESI) [M+H] ⁺=318 outside the invention |
| **150** | MS (ESI) [M+H] ⁺=280 |
| **151** | ¹H NMR (300 MHz, CDCl₃) δ 8.35 (s, 1H), 8.04 (d, *J* = 8.3, 1H), 7.82 (d, *J* = 8.9, 1H), 7.74 (d, *J* = 8.9, 1H), 7.60 (t, *J* = 7.8, 2H), 7.50 (dd, *J* = 2.3, 8.9, 1H), 7.36 (d, *J* = 8.9, 1H), 6.79 (d, *J* = 7.4, 1H), 2.75 (q, *J* = 7.6, 2H), 1.30 (t, *J* = 7.6, 3H). |
| | MS (ESI) [M+H]⁺= 284 |
| **152** | ¹H NMR (300 MHz, CDCl₃) δ 8.30 (d, *J* = 8.5, 1H), 8.08 (s, 1H), 7.90 (d, *J* = 9.0, 1H), 7.77 (d, *J* = 8.9, 1H), 7.65 (d, *J* = 2.2, 1H), 7.55 (td, *J* = 2.0, 8.8, 2H), 7.39 (d, *J* = 9.0, 1H), 2.31 (s, 3H). |
| | MS (ESI) [M+H]+= 270 |
| **153** | ¹H NMR (300 MHz, CDCl₃) δ 8.75 (s, 1H), 8.54 (s, 1H), 8.46 (d, *J* = 8.8, 1H), 7.91 (dd, *J* = 5.5, 14.5, 2H), 7.79 (d, *J* = 8.9, 1H), 7.67 (d, *J* = 2.1, 1H), 7.56 (dd, *J* = 2.3, 8.9, 1H), 7.35 (d, *J* = 8.9, 1H). |
| | MS (ESI) [M+H]+= 324 |
| **154** | ¹H NMR (300 MHz, DMSO) δ 9.08 (s, 1H), 8.12 (d, *J* = 8.4, 1H), 7.73 (d, *J* = 8.2, 2H), 7.66 (d, *J* = 10.0, 1H), 7.53 (s, 1H), 7.25 (s, 1H), 6.82 (s, 1H), 5.10 (s, 2H), 2.16 (s, 4H). |
| | MS (ESI) [M+H]+= 285 |
| **155** | ¹H NMR (300 MHz, CDCl₃) δ 7.68 (d, *J* = 8.3, 1H), 7.61 (s, 1H), 7.56 (d, *J* = 11.5, 2H), 7.44 (d, *J* = 8.3, 1H), 7.38 (d, *J* = 7.8, 1H), 7.13 (t, *J* = 7.4, 1H), 6.80 (d, *J* = 8.7, 2H), 3.85 (t, *J* = 6.5, 2H), 2.18 (s, 3H), 1.73 - 1.58 (m, 2H), 1.48 - 1.31 (m, 2H), 0.88 (t, *J* = 7.3, 3H) |
| | MS (ESI) [M+H]+= 307 |
| **156** | ¹H NMR (300 MHz, CDCl₃) δ 7.75 (d, *J* = 9.1, 1H), 7.62 (d, *J* = 8.9, 1H), 7.58 (d, *J* = 2.2, 1H), 7.48 (dd, *J* = 2.4, 8.9, 1H), 7.30 (d, *J* = 8.9, 2H), 6.86 (d, *J* = 9.0, 1H), 6.77 (d, *J* = 8.9, 2H), 6.71 (s, 1H), 2.97 (s, 6H) |
| | MS (ESI) [M+H]+= 298 |
| **157** | ¹H NMR (300 MHz, CDCl₃) δ 7.98 (d, *J* = 2.6, 1H), 7.89 (d, *J* = 8.9, 1H), 7.72 (d, *J* = 7.5, 1H), 7.62 (dd, *J* = 2.6, 8.8, 1H), 7.55 (d, *J* = 7.8, 1H), 7.20 (t, *J* = 7.8, 1H), 6.95 (d, *J* = 8.9, 1H), 6.84 (d, *J* = 8.9, 1H), 6.79 (s, 1H), 3.91 (s, 3H) |
| | MS (ESI) [M+H]+= 319 |
| **158** | ¹H NMR (300 MHz, CDCl₃) δ 7.89 (d, *J* = 9.0, 1H), 7.70 (dd, *J* = 1.2, 7.5, 1H), 7.56 (dd, *J* = 1.1, 8.0, 1H), 7.30 (d, *J* = 8.6, 1H), 7.20 (t, *J* = 7.8, 1H), 6.71 (t, *J* = 5.9, 2H), 6.64 (d, *J* = 9.5, 1H). |
| | MS (ESI) [M+H]+= 354 |
| **159** | ¹H NMR (300 MHz, CDCl₃) δ 8.80 (d, *J* = 2.6, 1H), 8.37 (d, *J* = 2.6, 1H), 8.01 (d, *J* = 8.1, 1H), 7.91 (dd, *J* = 1.6, 4.9, 1H), 7.78 - 7.70 (m, 1H), 7.58 - 7.43 (m, 2H), 7.09 (dd, *J* = 1.6, 7.6, 1H), 6.84 (dd, *J* = 4.9, 7.6, 1H), 6.69 (s, 1H), 3.82 - 3.07 (m, 2H). |
| **160** | ¹H NMR (300 MHz, CDCl₃) δ 9.68 - 8.90 (m, 1H), 8.77 (s, 1H), 8.35 (s, 1H), 8.14 (d, *J* = 5.0, 1H), 7.96 (s, 1H), 7.79 (d, *J* = 8.8, 1H), 7.61 (d, *J* = 8.5, 1H), 6.88 (d, *J* = 4.8, 1H), 2.46 (s, 3H) |
| **161** | ¹H NMR (300 MHz, CDCl₃) δ 9.98 (s, 1H), 8.70 (s, 1H), 8.45 (s, 1H), 8.27 (d, *J* = 5.2, 1H), 7.94 (d, *J* = 8.1, 1H), 7.84 (d, *J* = 8.2, 1H), 7.63 (t, *J* = 7.5, 1H), 7.48 (t, *J* = 7.5, 1H), 6.87 (d, *J* = 5.0, 1H), 2.74 (q, *J* = 7.6, 2H), 1.34 (t, *J* = 7.6, 3H). |
| | MS (ESI) [M+H]+= 251 |
| **162** | ¹H NMR (300 MHz, CDCl₃) δ 8.73 (s, 1H), 8.70 - 8.60 (m, 1H), 8.48 (s, 1H), 8.31 (s, 1H), 7.98 (d, *J* = 8.1, 1H), 7.86 (d, *J* = 7.9, 1H), 7.68 (t, *J* = 8.2, 1H), 7.54 (t, *J* = 8.1, 1H), 2.49 (s, 3H) |
| | MS (ESI) [M+H]+= 315 |
| **163** | ¹H NMR (300 MHz, CDCl₃) δ 8.75 (s, 1H), 8.68 (s, 1H), 8.01 (s, 1H), 7.95 (d, *J* = 8.2, 1H), 7.84 (d, *J* = 8.3, 1H), 7.64 (t, *J* = 8.2, 1H), 7.49 (t, *J* = 7.0, 1H), 6.69 (s, 1H), 2.45 (s, 3H), 2.38 (s, 3H) |
| | MS (ESI) [M+H]+= 251 |
| **164** | ¹H NMR (300 MHz, DMSO) δ 10.46 (s, 1H), 9.00 (s, 1H), 8.41 (s, 1H), 8.24 (d, *J* = 3.0, 1H), 7.90 (d, *J* = 8.2, 1H), 7.79 (d, *J* = 8.3, 1H), 7.69 (t, *J* = 7.0, 1H), 7.52 (t, *J* = 7.4, 1H), 6.98 (d, *J* = 4.8, 1H), 5.45 (q, *J* = 5.6, 1H), 4.58 (d, *J* = 5.7, 2H). |
| | MS (ESI) [M+H]+= 253 |
| **165** | ¹H NMR (300 MHz, CDCl₃) δ 9.07 (s, 1H), 8.79 (s, 1H), 8.51 (s, 1H), 8.18 (s, 1H), 8.09 - 8.01 (m, 1H), 7.94 (d, *J* = 8.4, 1H), 7.81 - 7.71 (m, 1H), 7.69 - 7.59 (m, 1H), 2.80 (s, 3H) |
| | MS (ESI) [M+H]⁺= 282 |
| **166** | ¹H NMR (300 MHz, CDCl₃) δ 8.49 (d, *J* = 5.0, 1H), 7.77 (d, *J* = 9.0, 1H), 7.32 (d, *J* = 2.0, 1H), 7.12 (d, *J* = 9.0, 2H), 6.99 (dd, *J* = 2.0, *J* = 9.0, 1H), 6.82 (d, *J* = 9.0, 2H), 6.57 (d, *J* = 5.0, 1H), 5.78 (s, 1H), 3.74 (s, 3H), 3.17 (s, 4H), 2.62 (s, 4H), 2.34 (s, 3H) |
| **167** | MS (ESI) [M+H] ⁺=335 |
| **168** | MS (ESI) [M+H] ⁺=321 |

The following examples illustrate in detail the preparation of compounds (2), (3), (4), (7), (8), (26), (31), (82), (105), (113), (128), (135), (136), (137), (138), (142), (146), (13), (108), (16), (123), and (38) according to the invention. The structures of the products obtained have been confirmed by NMR spectra.

### EXAMPLES

### Typical procedure for Pd-catalysed aminations

To a solution of 2-chloro quinoline (82mg, 0.5mmol, 1 equiv) in *tert*-butanol (2mL) were added the amino pyridine derivative / aniline (0.55mmol, 1.1 equiv), Cs₂CO₃ (456mg, 1.4mmol, 2.8 equiv), Xantphos (5.8mg, 0.01mmol, 2mol%), Pd(OAc)2 (2.2mg, 0.01mmol, 2mol%). The reaction mixture was heated at 90°C and stirred for 20 hours under argon. The reaction mixture was concentrated under reduced pressure. The residue was purified by column chromatography on silica gel to yield compounds (2), (3), (4) and (8).

### Example 1:

### 2-(Quinolin-2-ylamino)-isonicotinic acid - (2) of table I

¹H NMR (300 MHz, DMSO) δ 13.16 (s, 1H), 8.72 (d, *J* = 5.2, 1H), 8.63 (d, *J* = 9.0, 1H), 8.28 - 8.13 (m, 2H), 8.05 (d, *J* = 8.0, 1H), 7.90 (t, *J* = 7.5, 1H), 7.74 - 7.67 (m, 2H), 7.67 - 7.59 (m, 2H).

MS (electrospray) m/z (%) 266.1 (100) [M+H]⁺.

### Example 2:

### (4-Methyl-pyridin-2-yl)-quinolin-2-yl-amine - (3) of table I

¹H NMR (300 MHz, CDCl₃) δ 8.92 (s, 1H), 8.21 (d, *J* = 5.3, 2H), 7.95 (d, *J* = 8.9, 1H), 7.89 (d, *J* = 8.4, 1H), 7.67 (d, *J* = 8.0, 1H), 7.62 (t, *J* = 7.7, 1H), 7.40 - 7.28 (m, 2H), 6.78 (d, *J* = 5.1, 1H), 2.41 (s, 3H).

¹³C NMR (75 MHz, CDCl₃) δ 154.3, 153.3, 149.5, 147.3, 137.7, 129.8, 127.6, 127.1, 124.6, 123.7, 118.7, 114.1, 113.4, 21.7.

MS (electrospray) m/z (%) 236.2 (100) [M+H]+.

### Example 3:

### Pyridin-2-yl-quinolin-2-yl-amine - (4) of table I

¹H NMR (300 MHz, CDCl₃) δ 8.38 (d, *J* = 8.4, 1H), 8.31 (dd, *J* = 1.0, 4.9, 1H), 8.01 (d, *J* = 8.9, 1H), 7.87 (d, *J* = 8.4, 1H), 7.77 - 7.68 (m, 3H), 7.64 (t, *J* = 7.7, 1H), 7.36 (t, *J* = 7.5, 1H), 7.31 (d, *J* = 8.9, 1H), 6.94 (dd, *J* = 5.0, 7.2, 1H).

¹³C NMR (75 MHz, CDCl₃) δ 154.1, 153.1, 147.8, 147.3, 138.3, 137.8, 129.9, 127.6, 127.2, 124.6, 123.8, 117.4, 114.0, 113.0.

MS (electrospray) m/z (%) 222.2 (100) [M+H]+.

### Example 4:

### Quinolin-2-yl-(4-trifluoromethoxy-phenyl)-amine - (8) of table I

¹H NMR (300 MHz, CDCl₃) δ 7.97 (d, *J* = 8.8, 1H), 7.82 (d, *J* = 8.4, 1H), 7.69 (t, *J* = 9.4, 3H), 7.62 (t, *J* = 7.7, 1H), 7.34 (t, *J* = 7.5, 1H), 7.23 (d, *J* = 8.7, 2H), 6.92 (d, *J* = 8.9, 1H), 6.74 (s, 1H).

¹³C NMR (75 MHz, CDCl₃) δ 153.9, 147.6, 144.4, 139.3, 138.1, 130.1, 127.7, 127.1, 124.4, 123.7, 122.5, 122.2, 121.0, 119.1, 112.2.

MS (electrospray) m/z (%) 305.0 (100) [M+H]+.

According to route (A), the compound of formula (III) is placed in a protic solvent such as tert-butanol. The compound of formula (IV) is then added in a 1.1 molar ratio with respect to the compound of formula (III) in presence of an inorganic base, such as Cs₂CO₃ or K2CO3, in a 2.8 molar ratio, in the presence of a diphosphine, such as Xantphos (4,5-Bis(diphenylphosphino)-9,9-dimethylxanthene), or X-Phos 2-Dicyclohexylphosphino-2',4',6'-triisopropylbiphenyl in a 2mol% amount relative to the total amount of compound of formula (III), and in the presence of a catalyst, such as Pd(OAc)2,or Pd2dba3 in a 2mol% amount relative to the total amount of compound of formula (III). The reaction mixture is then heated at 90°C, and stirred during 20 hours, under argon. The reaction mixture is concentrated under reduced pressure and the resulting residue is diluted with ethyl acetate. The organic phase is then washed twice with water, dried on magnesium sulphate, filtered and concentrated under reduced pressure. The residue could then be purified by column chromatography on silica gel to yield pure compounds (7), (26), (31), (8), (82), (105), (113), (128), (135), (136), (137), (138), (142), (146).

According to route (B), the compound of formula (V) is placed in a protic solvent such as tert-butanol. The compound of formula (VI) is then added in a 1.1 molar ratio with respect to the compound of formula (V) in presence of Cs₂CO₃ in a 2.8 molar ratio, in the presence of Xantphos (4,5-Bis(diphenylphosphino)-9,9-dimethylxanthene) in a 2mol% amount relative to the total amount of compound of formula (V), and in the presence of a Pd(OAc)2, in a 2mol% amount relative to the total amount of compound of formula (V). The reaction mixture is then heated at 90°C, and stirred during 20 hours, under argon. The reaction mixture is concentrated under reduced pressure and the resulting residue is diluted with ethyl acetate. The organic phase is then washed twice with water, dried on magnesium sulphate, filtered and concentrated under reduced pressure. The residue could then be purified by column chromatography on silica gel to yield pure compound (13), (108), (16), (123), (38).

### Example 5: compound (7) of table I

According to route (A), a mixture of 2-chloroquinoline (1.3 g ), 2-amino-5-cyanopyridine (1.1 g), Pd(OAc)2 (36.5 mg), XantPhos (94 mg) and Cs₂CO₃ (7.4 g) in 32 mL of t-BuOH gave compound (7) (1.6 g).

### Example 6: compound (26) of table I

According to route (A), a mixture of 2,6-dichloroquinoline (98.5mg), 6-amino-3-picoline (59.4mg), Pd(OAc)2 (2.2mg), XantPhos (5.8mg) and Cs₂CO₃ (456mg) in 2 mL of t-BuOH gave compound (26) (92.3mg).

### Example 7: compound (38) of table I

According to route (B), a mixture of 2-aminoquinoline (79.2mg), 3-bromo-2-methoxypyridine (71.5mg), Pd(OAc)2 (2.2mg), XantPhos (5.8mg) and Cs₂CO₃ (456mg) in 2mL of t-BuOH gave compound (38) (73.5mg).

### Example 8: compound (31) of table I

According to route (A), a mixture of 2-chloroquinoline (81.5mg), 2-amino-3-picoline (55µL), Pd(OAc)₂ (2.2mg), XantPhos (5.8mg) and Cs₂CO₃ (456mg) in 2 mL of t-BuOH gave compound (31) (87.1mg).

### Example 9: compound (8) of table I

According to route (A), a mixture of 2-chloroquinoline (1.1g), 4-(trifluoromethoxy)aniline (1.0mL), Pd(OAc)2 (31.4mg), XantPhos (80.9 mg) and Cs₂CO₃ (6.4g) in 28 mL of t-BuOH gave compound (8) (1.3g).

### Example 10: compound (82) of table I

According to route (A), a mixture of 2-chloroquinoline (81.5mg), 2-methyl-4-(trifluoromethoxy)aniline (81µL), Pd(OAc)₂ (2.2mg), XantPhos (5.8mg) and Cs₂CO₃ (456mg) in 2mL of t-BuOH gave compound (82) (64mg).

### Example 11: compound (13) of table I

According to route (B), a mixture of 3-aminoquinoline (79mg), 2-bromo-5-methylpyridine (101mg), Pd(OAc)2 (2.2mg), XantPhos (5.8mg) and Cs₂CO₃ (456mg) in 2mL of t-BuOH gave compound (13) (84.4mg).

### Example 12: compound (105) of table I

According to route (A), a mixture of 3-bromoquinoline (103.5mg), 6-amino-3-picoline (59.4mg), Pd(OAc)2 (2.2mg), XantPhos (5.8mg) and Cs₂CO₃ (456mg) in 2mL of t-BuOH gave compound (105) (66.5mg).

### Example 13: compound (108) of table I

According to route (B), a mixture of 6-aminoquinoline (79.0mg), 2-bromo-3methoxypyridine (94.0mg), Pd(OAc)2 (2.2mg), XantPhos (5.8mg) and Cs₂CO₃ (456mg) in 2mL of t-BuOH gave compound (108) (75.9mg).

### Example 14: compound (16) of table I

According to route (B), a mixture of 6-aminoquinoline (79.3mg), 2-bromo-6methylpyridine, Pd(OAc)₂ (2.2mg), XantPhos (5.8mg) and Cs₂CO₃ (456mg) in 2mL of t-BuOH gave compound (16) (81.2mg ).

### Example 15: compound (113) of table I

According to route (A), a mixture of 2-chloroquinoline (81.5mg), aminopyrazine (52.3mg), Pd(OAc)2 (2.2mg), XantPhos (5.8mg) and Cs₂CO₃ (456mg) in 2mL of t-BuOH gave compound (113) (60.7mg).

### Example 16: compound (123) of table I

According to route (B), a mixture of 3-bromoquinoline (103.5mg), p-anisidine (67.7mg), Pd(OAc)2 (2.2mg), XantPhos (5.8mg) and Cs₂CO₃ (456mg) in 2mL of t-BuOH gave compound (386) (65mg).

¹H NMR (300 MHz, CDCl₃) δ 8.62 (d, J = 2.8, 1H), 7.98 (d, J = 7.4, 1H), 7.61 - 7.54 (m, 1H), 7.45 (ddd, J = 1.9, 4.9, 7.1, 3H), 7.18 (d, J = 8.9, 2H), 6.94 (d, J = 8.9, 2H), 5.86 (s, 1H), 3.84 (s, 3H).

¹³C NMR (75 MHz, CDCl₃) δ 156.26, 144.27, 143.25, 139.18, 134.42, 129.25, 129.18, 127.24, 126.40, 126.04, 123.03, 115.13, 114.26, 55.79.

### Example 17: compound (128) of table I

According to route (A), a mixture of 2-chloroquinoline (81.5mg), 2-aminopyrimidine (52.3mg), Pd(OAc)2 (2.2mg), XantPhos (5.8mg) and Cs₂CO₃ (456mg) in 2mL of t-BuOH gave compound (128) (53.3mg).

### Example 18: compound (135) of table I

According to route (A), a mixture of 2-chloroquinoxaline (82.0mg), 2-aminopyridine (51.7mg), Pd(OAc)2 (2.2mg), XantPhos (5.8mg) and Cs₂CO₃ (456mg) in 2 mL of t-BuOH gave compound (135) (47.7mg).

### Example 19: compound (136) of table I

According to route (A), a mixture of 2-chloroquinoxaline (82.0mg), 2-amino-3methylpyridine (59.4mg), Pd(OAc)₂ (2.2 mg), XantPhos (5.8mg) and Cs₂CO₃ (456mg) in 2 mL of t-BuOH gave compound (136) (35.4mg).

### Example 20: compound (137) of table I

According to route (A), a mixture of 2-chloroquinoxaline (82.0mg), 2-amino-5-cyanopyridine (65.4mg), Pd(OAc)₂ (2.2mg), XantPhos (5.8mg) and Cs₂CO₃ (456mg) in 2mL of t-BuOH gave compound (137) (79.6mg).

### Example 21: compound (138) of table I

According to route (A), a mixture of 2-chloroquinoxaline (82.0mg), 6-amino-2-picoline (59.4mg), Pd(OAc)2 (2.2mg), XantPhos (5.8mg) and Cs₂CO₃ (456mg) in 2 mL of t-BuOH gave compound (138) (89.4mg).

### Example 22: compound (142) of table I

According to route (A), a mixture of 2-chloroquinoxaline (82.0mg), 2-aminopyrimidine (52.3mg), Pd(OAc)2 (2.2mg), XantPhos (5.8mg) and Cs₂CO₃ (456 mg) in 2mL of t-BuOH gave compound (142) (47.0mg).

¹H NMR (300 MHz, CDC13) δ 10.07 (s, 1H), 8.57 (d, J = 4.7, 2H), 8.04 (d, J = 7.8, 1H), 7.83 (d, J = 7.9, 1H), 7.68 (t, J = 8.6, 1H), 7.58 (t, J = 7.4, 1H), 6.92 (t, J = 4.6, 1H).

MS (ESI) [M+H]+= 224

### Example 23: compound (146) of table I

According to route (A), a mixture of 4-methoxy-7-chloro-quinoline^{∗} (500.0mg), 2-amino-4-methylpyridine (0.310g) XPhos (123.0mg), K₂CO₃ (1.41g) and Pd2dba3 (118.0mg) in 14mL of t-BuOH gave compound 146 (500mg).

^{∗}Preparation of 4-methoxy-7-chloro-quinoline:
Into a 1-Neck round-bottom flask 4,7-dichloro- quinoline, (5.0g, 0.025mol) was dissolved in 1.25 M of hydrogen chloride in methanol (16mL). The mixture was heated to reflux overnight. After 16 hours of heating, the mixture was cooled to room temperature. The mixture was concentrated under reduce pressure. The solid was dissolved in sodium bicarbonate (50mL, 0.6mol) (saturated aqueous solution) and the solution was extracted with Ethyl acetate (100mL, 1mol) (4×25ml). The organic layer was washed with sodium chloride (50mL, 0.8mol) (saturated aqueous solution) (2×25ml) and was dried over Na₂SO₄ and was concentrated under reduce pressure to give 4-methoxy-7-chloro-quinoline (4,5g pale yellow powder).

MS (ESI) [M+H]+= 194

### Example 24:

### Pharmacological data

The compounds of the invention have been the subject of pharmacological tests which have demonstrated their relevance as active substances in therapy and in particular for preventing, inhibiting or treating pathological conditions linked with premature aging.

The following materials and methods have been used.

### MATERIAL AND METHODS

### Minigene constructs reproduce aberrant splicing of LMNA mRNA, leading to HGPS

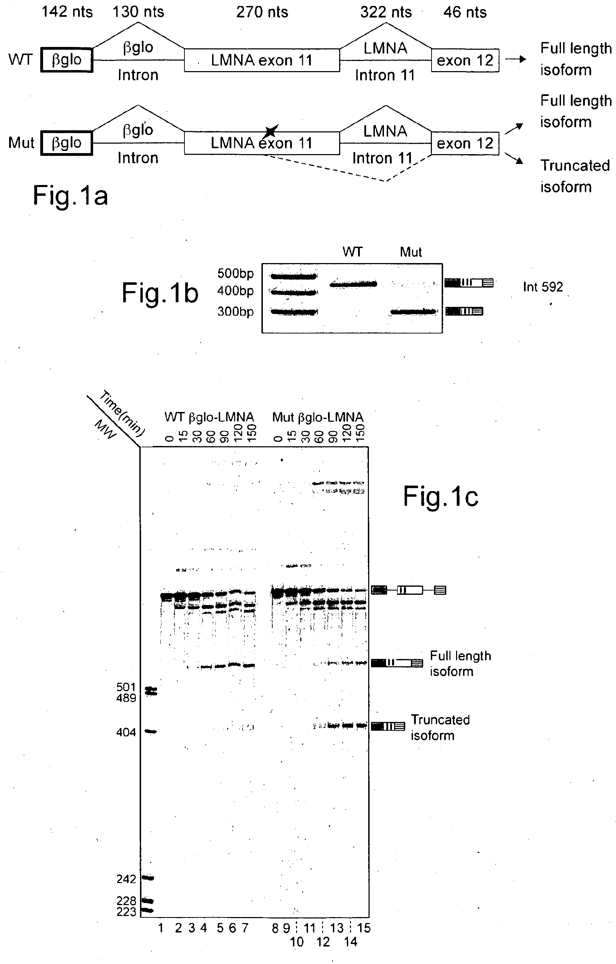

In order to identify and characterize the factor(s) involved in the use of the cryptic 5' splice site in exon 11 of LMNA, an *ex vivo* system has been developed that recapitulates this splicing event. The cloning of mutant and wild type constructs (Fig. 1A, for schematic representation) were carried out using a TOPO-TA cloning vector in which is inserted a minigene containing 142 nts of β-Globin fist exon, 130 nts β-Globin first intron, 270 nts LMNA exon 11 either wild type or mutant, 322 nts intron 11 and 46 nts exon 12. Using this system the splicing event activated by the GGC>GGT mutation in exon 11 of the LMNA gene was confirmed by transfections in cultured HeLa cells (Fig. 1B, lanes WT and Mut) as well as *in vitro* splicing experiments using in vitro synthesized radiolabeled substrate (Panel C). Transfection experiments of minigene constructs containing or not the point mutation demonstrated that like in Progeria patients the mutation leads to a switch from the use of the normal splice site (intron 11 position 1) to the use of the cryptic splice site upstream of the mutation (exon 11 position 1819) (Fig. 1, Panel C, compare lanes WT and Mut). Note that following a kinetics of *in vitro* splicing for 150 minutes, aberrant splicing is observed with the wild type substrate (Fig1C, Lanes 1-7), implying that the mutation is not a perquisite for cryptic splice site usage. The mutation simply enhances the efficacy of selection of this cryptic splice site (Fig1C, Lanes 8-15).

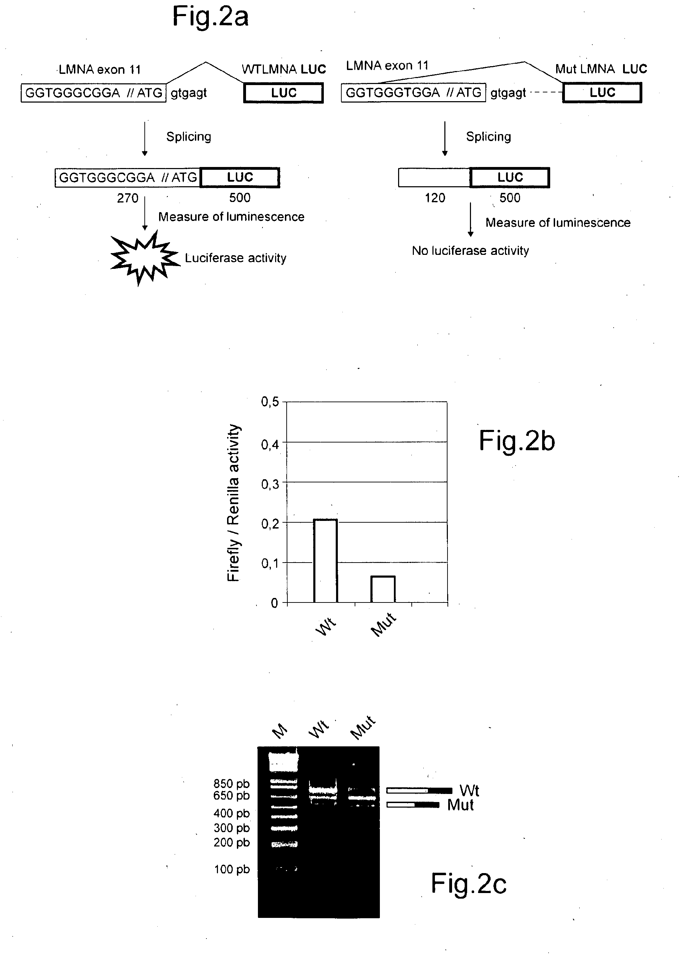

Advantage has been taken of the luciferase system. The luciferase assay is an extremely sensitive and rapid assay. Linear results are seen over at least eight orders of magnitude of enzyme concentration. Moreover, the luciferase assay is well suited for high-throughput applications. To conduct a Mid-throughput screening (MTS) for compounds repressing LMNA aberrant splicing, we have constructed a plasmid in which exon 11, intron 11 and part of exon 12 of LMNA gene were fused with luciferase cDNA (Fig. 2). Both wild type (WtLMNA-luc) and mutant (MutLMNA-luc) substrate harbouring exon 11 mutation have been constructed. In these constructs we have generated a single initiation codon in exon 11 such as correct splicing will lead to luciferase expression, while aberrant splicing will skip the initiation codon and thereby prevent luciferase expression. After transfection in HeLa cells, Luciferase assays (Fig. 2B) and RT-PCR (Fig. 2C) indicate that WtLMNA-luc produces predominantly wild type splicing and large amount of luciferase activity, whereas MutLMNA-luc recapitulate the aberrant splicing profile with reduced luciferase expression (Fig.2 B and C, compare Wt and Mut). In order to use this system in MTS, we have generated a stable 293 cell lines containing a single integrated copy of luciferase reporter containing LMNA mutation (MutLMNA-luc cell line) using the flp system from INVITROGEN. This system allows us to perform a MTS for compounds able to enhance luciferase activity.

***Plasmids constructs.*** LMNA sequences (1278 bp of exon 11, intron 11 and 46 bp of exon 11) were PCR-amplified from either control or patient's cells genomic DNA with specific primer PCR fragments were purified with Concert Rapid PCR purification system (Invitrogen) and subcloned at the BamHI and EcoRI restriction sites of the pSpβm3S1 plasmid containing the βGlobin cassette (Labourier et al., 1999 - Recognition of exonic splicing enhancer sequences by the Drosophila splicing repressor RSF1. Nucleic Acids Res. 27, 2377-2386) to give the βGlo3S1LMNAwt and βGlo3S1LMNAmut constructs. The chimeric PGlo-LMNA sequences were then inserted into the pcDNA3.1D/V5-His-TOPO vector (Invitrogen) to be used in transfection and in vitro splicing experiments. A single initiation codon ATG was kept in exon 11 of LMNA and LMNA sequences described above were fused at their 3' end to Fyrefly luciferase cDNA (LMNAlucWT) in order that removal of intron 11 generates a transcript that encode a fusion protein harbouring luciferase activity, whereas usage of the cryptic splice site of mutated exon 11 (LMNAlucMut) will remove the initiation codon preventing luciferase expression. Both sequences were cloned in pcDNA3 Flp-In vector (Invitrogen).

***Transfection and RT-PCR.*** HeLa cells transfections with splicing reporter constructs were performed with lipofectAMINE 2000 reagent (Invitrogen) according to the manufacturer's instructions. Twenty four hours after transfection, total RNA was purified with RNA-PLUSTM (Quantum Bioprobe). First strand cDNA was synthesized from 2 µg of RNA with the Amersham-Pharmacia First strand cDNA synthesis kit. For PCR analyses, 1/15 of the reaction was amplified with Taq polymerase (Invitrogen). The cycle number was kept to a minimum to maintain linearity. PCR products were separated on a 1.5 % agarose gel containing ethidium bromide and visualized under UV light.

A stable 293 cell line containing a single copy of LMNAlucMut minigene was obtained using the Flp-In system from (Invitrogen) according to manufacture procedure. Several clones were obtained and only one clone was used to screen the whole chemical library (293FLP LMNA LUC cells #8).

***Nuclear extracts preparation, splicing and complementation assays.*** HeLa cells nuclear extracts were prepared according to (Dignam et al., 1983 - Eukaryotic gene transcription with purified components. Methods Enzymol. 101, 582-598). Pre-mRNA were synthesized by in vitro transcription in the presence of 20 units of T7 RNA polymerase, 1µg of the suitable linearized plasmids and 5 µM [α-³²P] UTP (3000 Ci/mmol) in 25µl reactions according to manufacturer conditions. In vitro transcripts were quantified by Cerenkov counting. Splicing reactions were performed under standard conditions as described previously (Tazi et al., 1986 - A protein that specifically recognizes the 3' splice site of mammalian pre-mRNA introns is associated with a small nuclear ribonucleoprotein. Cell 47, 755-766). Splicing products were analyzed by electrophoresis on 7% denaturing polyacrylamide gels and revealed by autoradiography.

### Material

### 293FLP LMNA LUC cells #8

Hygromycin B at 50mg/mL (*invitrogen 10687-010*)
Dulbecco's Modified Eagle Medium (D-MEM) (1X) + GlutaMAX, liquid *(invitrogen 31966-021)*
Dulbecco's Phosphate Buffered Saline (D-PBS) (1X), liquid (*invitrogen 14190-169)*
Trypsin 2.5%
Foetal calf serum (FCS)
Penicillin (P)
Streptomycin (S)
Passive Lysis Buffer (PLB) (5X) *(Promega)*
Bradford Reagent (B6916)
Luciferase assay buffer
96 Well Plate sterile, V-shape *(greiner bio-one 651180)*
96 Well Microplate sterile, flat bottom *(greiner bio-one 655180)*
96 Well Microplate, flat bottom, Chimney Well *(greiner bio-one 655075)*
CellTiter 96^{®} AQueous One Solution *(Promega G3581)*

### Methods

### FIRST DAY

### Plate at 500 µM

In a 96 Well Plate sterile, V-shape one put 0.5µl of drug compounds at 50mM and then add 49,5µl of 10% DMSO.

### Replica plate

One pipets 47µl of drug at 500µM and adds 200µL of DMEM + Hygromycin B. At this stage the concentration of drug compound is 10µM. One shares out 100µl in a 96 Well Microplate sterile, flat bottom (further called luciferase plate) and 50µl in other one (further called toxicity plate).

One washes 293FLP LMNA LUC cells once with D-PBS then adds 1 ml trypsin EDTA. Incubation at 37°C for 2-3 minutes is proceeded. Then one adds 9ml DMEM (with 10% FCS, P/S).

One takes 7µl of cell suspension and adds 14µL blue trypan to count cells. Meanwhile cell suspension is centrifugated at 1200 rpm for 5 minutes at room temperature (RT).

Cell concentration is brought at 105 cells per ml with DMEM + Hygromycin B to have 104 cells per 100µl.

### Luciferase plate

100µl of suspension cells is added (at 104 cells per 100µl) so final concentration of compounds is 5µM.

### Toxicity plate

50µl of suspension cells is added. The final concentration of compounds is 5µM.

### 48 HOURS LATER

### Toxicity plate

20µl of CellTiter 96^{®} AQueous One Solution is added per well. Incubation is proceeded at 37°C for 2 h. Absorbance is red at 490nm.

### Luciferase plate

Medium of the wells is gently removed then washed once by adding slowly 150µl of D-PBS 1X. D-PBS is removed. 40µl of PLB 1X is added and incubated at RT for 30 minutes.

20µl of cell lysate is put in a 96 Well Microplate, flat bottom, Chimney Well. 70 µl of luciferin assay substrate is added. One read luminescence for 1 second.

200µl of Bradford reagent is added on the remaining cell lysate (20µl). Incubation is proceeded at RT for 30 min then one can read absorbance at 595nm. A range has to be made. Usually 5 different concentrations are tested: 0.25, 0.5, 0.75, 1 and 1.25mg/ml.

### RESULTS

The compounds according to the present invention demonstrate an increase of luciferase activity ranging between 3 and 7 fold compared to control untreated MutLMNA-luc cell line.

In particular, the results are as follows for some of the compounds according to the present invention.

| **Compound number (invention)** | **Increase of luciferase activity** |
|---|---|
| 7 | 3.33 |
| 34 | 4.18 |
| 36 | 3.06 |
| 31 | 5.07 |
| 26 | 6.20 |
| 8 | 3.35 |
| 105 | 3.25 |
| 135 | 4.58 |
| 136 | 5.20 |
| 137 | 4.64 |
| 138 | 8.22 |
| 142 | 4.47 |
| 2 | 3.77 |
| 3 | 4.54 |
| 4 | 4.43 |
| 5 | 2.02 |
| 17 | 3.66 |
| 18 | 2.85 |
| 25 | 3.49 |
| 28 | 2.99 |
| 32 | 2.96 |
| 33 | 2.14 |
| 35 | 2.74 |
| 38 | 5.81 |
| 39 | 4.29 |
| 41 | 3.32 |
| 42 | 3.87 |
| 45 | 3.08 |
| 59 | 2.49 |
| 61 | 2.04 |
| 82 | 3.41 |
| 83 | 2.74 |
| 86 | 2.77 |
| 102 | 2.06 |
| 9 | 2.47 |
| 10 | 2.01 |
| 13 | 3.46 |
| 106 | 2.77 |
| 15 | 2.17 |
| 16 | 4.56 |
| 107 | 2.20 |
| 108 | 4.57 |
| 109 | 2.79 |
| 113 | 2.28 |
| 120 | 2.38 |
| 123 | 2.94 |
| 125 | 2.59 |
| 128 | 3.08 |
| 145 | 3.87 |
| 146 | 4.18 |
| 147 | 2.94 |

Therefore, the result of the tests carried out on the compounds disclosed in the present invention show that said compounds may be useful to inhibit, prevent and/or treat diseases with premature aging and that are likely related to an aberrant splicing of the nuclear lamin A gene. Among all, said disease may include Hutchinson Guilford Progeria Syndrome (HGPS), progeria, premature aging associated with HIV infection, muscular dystrophy, Charcot-Marie-Tooth disorder, Werner syndrome, but also atherosclerosis, insulin resistant type II diabetes, cataracts, osteoporosis and aging of the skin such as restrictive dermopathy.

For this purpose an effective amount of a said compound may be administered to a patient suffering from premature aging and in particular from progeria, and from the previous cited diseases.

The present invention is also related to the use of at least a compound chosen among a compound of anyone of formula (I), (Ia), (Ib), (Ic), (Id), (Ie), (If), (Ig), (Ih), (Ii), (Ij), (Ik), (Il), (Im), (Io), (Ip), (Iq), or (Ir) as defined above, and compounds (1) to (168) as defined above, or one of its pharmaceutically acceptable salts according to the present invention for the manufacture of a pharmaceutical composition intended for the treatment of pathological conditions linked with premature aging and in particular progeria.

The present invention also encompasses pharmaceutical compositions comprising at least a compound chosen among new compounds of formula (Ic), (Id), (Ig), (Ii'), (Ij'), (Ij"), (Ij‴), (Ik), (Io), (Ip) and (Ir) as defined above and compounds (10)-(16), (105)-(108), (118), (119), (121), (124)-(130), (132), (135)-(141), (145)-(147), (159)-(165), and (168) , as defined above or any pharmaceutically acceptable salt thereof.

Thus, these pharmaceutical compositions contain an effective amount of said compound, and one or more pharmaceutical excipients.

The aforementioned excipients are selected according to the dosage form and the desired mode of administration.

In this context they can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions, in doses which enable the daily administration of from 0.1 to 1000 mg of active substance.

The present invention is also related to the use of at least a compound chosen among a compound of anyone of formula (I), (Ia), (Ib), (Ic), (Id), (Ie), (If), (Ig), (Ih), (Ii), (Ij), (Ik), (Il), (Im), (Io), (Ip), (Iq), or (Ir) as defined above, and compounds (1) to (147), and (150) to (168) as defined above, or one of its pharmaceutically acceptable salts according to the present invention for the manufacture of a pharmaceutical composition intended for inhibiting, preventing and/or treating pathological conditions linked with premature aging and in particular progeria but also all the previous listed diseases.

## Claims

1. A compound of formula (I): 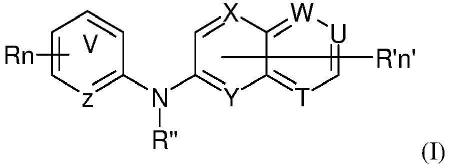 wherein: 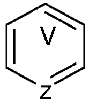
means an aromatic ring wherein V is C or N and when V is N, V is in ortho, meta or para of Z, i.e. forms respectively a pyridazine, a pyrimidine or a pyrazine group,
R independently represent a hydrogen atom, a halogen atom or a group chosen among a -CN group, a hydroxyl group, a -COOR₁ group, a (C₁-C₃)fluoroalkyl group, a (C₁-C₃)fluoroalkoxy group, a -NO₂ group, a -NR₁R₂ group, a (C₁-C₄)alkoxy group, a phenoxy group and a (C₁-C₃)alkyl group, said alkyl being optionally mono-substituted by a hydroxyl group,
R₁ and R₂ are independently a hydrogen atom or a (C₁-C₃)alkyl group,
n is 1, 2 or 3,
n' is 1 or 2,
R' is a hydrogen atom, a halogen atom or a group chosen among a (C₁-C₃)alkyl group, , a hydroxyl group, a -COOR₁ group, a -NO₂ group, a -NR₁R₂ group, a morpholinyl or a morpholino group, a N-methylpiperazinyl group, a (C₁-C₃)fluoroalkyl group, a (C₁-C₄)alkoxy group and a -CN group,
R" is a hydrogen atom or a (C₁-C₄)alkyl group,
- Z is N, V is C, Y is N, X is C, T is C, U is C and W is C,
- Z is C, V is C, Y is N, X is C, T is C, U is C and W is C,
- Z is N, V is C, Y is C, X is N, T is C, U is C and W is C,
- Z is N, V is C, Y is C, X is C, T is C, U is C and W is N,
- Z is N, V is N and is in para of Z, Y is N, X is C, T is C, U is C and W is C
- Z is C, V is N and is in para of Z, Y is C, X is N, T is C, U is C and W is C,
- Z is C, V is N and is in meta of Z and is in para of the bond linked to NR", Y is N, X is C, T is C, U is C and W is C,
- Z is C, V is N and is in meta of Z and is in para of the bond linked to NR", Y is C, X is N, T is C, U is C and W is C,
- Z is C, V is C, Y is C, X is N, T is C, U is C and W is C,
- Z is C, V is C, Y is N, X is N, T is C, U is C and W is C,
- Z is N, V is N and is in meta of Z and in ortho of the bond linked to NR", Y is N, X is C, T is C, U is C and W is C,
- Z is N, V is N and is in para of Z, Y is C, X is C, T is C, U is C and W is N,
- Z is N, V is N and is in para of Z, Y is C, X is N, T is C, U is C and W is C,
- Z is N, V is C, Y is N, X is N, T is C, U is C and W is C,
- Z is N, V is N and is in meta of Z and is in ortho of the bond linked to NR", Y is N, X is N, T is C, U is C and W is C,
- Z is C, V is C, Y is C, X is C, T is N, U is C and W is C, or
- Z is N, V is C, Y is C, X is C, T is N, U is C and W is C,
or anyone of its pharmaceutically acceptable salt,
for use as an agent for preventing, inhibiting or treating pathological conditions linked with premature aging.

2. A compound of formula (I) according to claim 1, wherein alternatively:
- Z is N, V is C, Y is N, X is C, T is C, U is C and W is C,
- Z is C, V is C, Y is N, X is C, T is C, U is C and W is C,
- Z is N, V is C, Y is C, X is N, T is C, U is C and W is C,
- Z is N, V is C, Y is C, X is C, T is C, U is C and W is N,
- Z is N, V is N and is in para of Z, Y is N, X is C, T is C, U is C and W is C,
- Z is C, V is C, Y is C, X is N, T is C, U is C and W is C,
- Z is C, V is C, Y is N, X is N, T is C, U is C and W is C,
- Z is N, V is N and is in meta of Z and is in ortho of the bond linked to NR", Y is N, X is C, T is C, U is C and W is C,
- Z is N, V is C, Y is N, X is N, T is C, U is C and W is C,
- Z is N, V is N and is in meta of Z and is in ortho of the bond linked to NR", Y is N, X is N, T is C, U is C and W is C, or
- Z is N, V is C, Y is C, X is C, T is N, U is C and W is C,
for use as an agent for preventing, inhibiting or treating pathological conditions linked with premature aging.

3. A compound of formula (I) according to anyone of the preceding claims, for use as an agent for preventing, inhibiting or treating pathological conditions linked with premature aging, which is alternatively chosen among:(1) 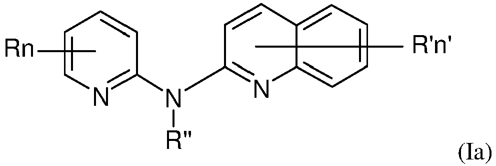 wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a -NO₂ group, a -NR₁R₂ group and a (C₁-C₃)alkoxy group,
R" is as defined in claim 1 and is advantageously a hydrogen atom,
n is as defined in claim 1 and is advantageously 1,
n' is as defined in claim 1 and is advantageously 1,
R' is a hydrogen atom, a halogen atom or a group chosen among a (C₁-C₃)alkyl group, a -NO₂ group, a (C₁-C₃)alkoxy group and a -NR₁R₂ group,
R₁ and R₂ are a hydrogen atom or a (C₁-C₃)alkyl group, 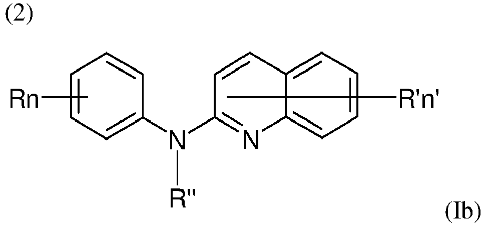
wherein :
R independently represent a hydrogen atom, a halogen atom or a group chosen among a (C₁-C₃)alkyl group, a -NR₁R₂ group, a (C₁-C₃)fluoroalkoxy group, a -NO₂ group, a phenoxy group and a (C₁-C₄)alkoxy group,
R₁ and R₂ are independently a hydrogen atom or a (C₁-C₃)alkyl group,
R" is as defined in claim 1 and is advantageously a hydrogen atom,
n is as defined in claim 1 and is preferably 1 or 2,
n' is as defined in claim 1 and is preferably 1,
R' is a hydrogen atom, a halogen atom or a group chosen among a (C₁-C₃)alkyl group and a (C₁-C₄)alkoxy group, 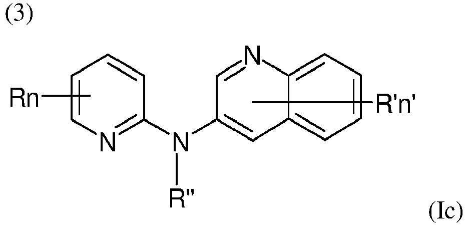
wherein:
R independently represent a hydrogen atom or a group chosen among a (C₁-C₃)alkyl group, a (C₁-C₃)fluoroalkyl group, a -NR₁R₂ group, a -COOR₁ group, a -NO₂ group and a (C₁-C₃)alkoxy group,
R" is as defined in claim 1 and is advantageously a hydrogen atom,
n is as defined in claim 1 and is advantageously 1,
n' is as defined in claim 1 and is advantageously 1,
R' is a hydrogen atom,
R₁ and R₂ are independently a hydrogen atom or a (C₁-C₃)alkyl group, 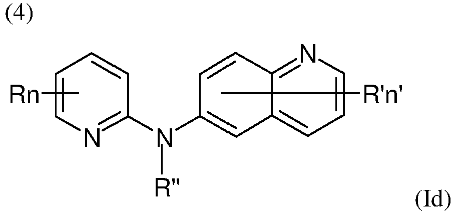
wherein:
R independently represent a hydrogen atom or a group chosen among a (C₁-C₃)alkyl group, a (C₁-C₃)fluoroalkyl group and a (C₁-C₃)alkoxy group,
R" is as defined in claim 1 and is advantageously a hydrogen atom,
n is as defined in claim 1 and is advantageously 1,
n' is as defined in claim 1 and is advantageously 1,
R' is a hydrogen atom, 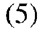 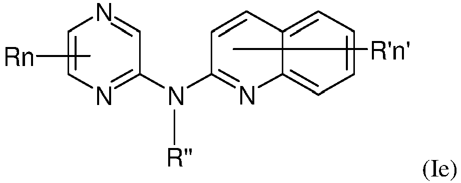
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom, a halogen atom or a group chosen among a (C₁-C₃)alkyl group and a (C₁-C₃)alkoxy group, 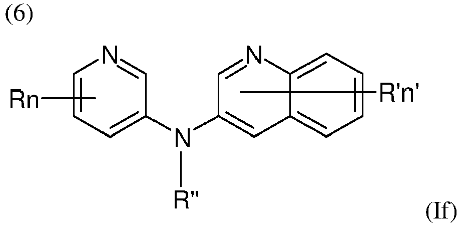
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom, 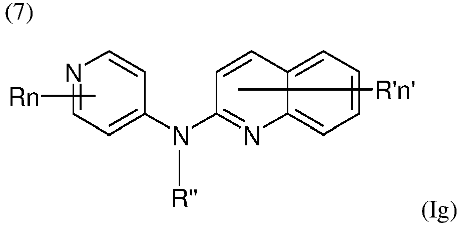
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom or a halogen atom, 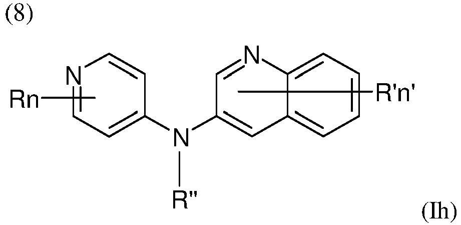
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom, 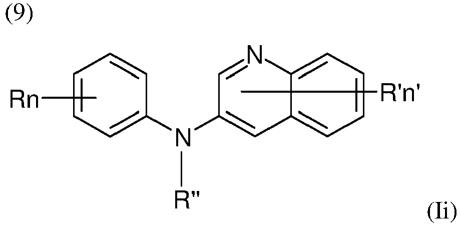
wherein:
R independently represent a hydrogen atom or a group chosen among a (C₁-C₃)fluoroalkoxy group and a (C₁-C₃)alkoxy group,
R" is as defined in claim 1 and is advantageously a hydrogen atom,
n is as defined in claim 1 and is advantageously 1,
n' is as defined in claim 1 and is advantageously 1,
R' is a hydrogen atom, 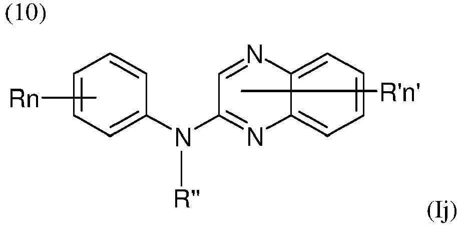
wherein:
R independently represent a hydrogen atom or a group chosen among a (C₁-C₃)fluoroalkoxy group and a (C₁-C₃)alkyl group,
R" is as defined in claim 1 and is advantageously a hydrogen atom,
n is as defined in claim 1 and is advantageously 1,
n' is as defined in claim 1 and is advantageously 1,
R' is a hydrogen atom, 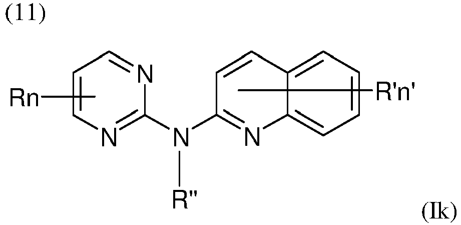
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom, a halogen atom or a (C₁-C₃)alkyl group, 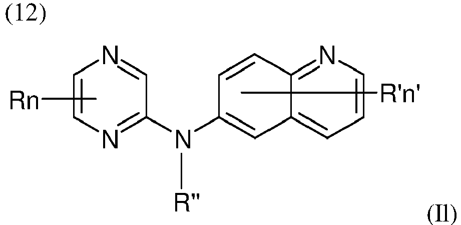
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom, 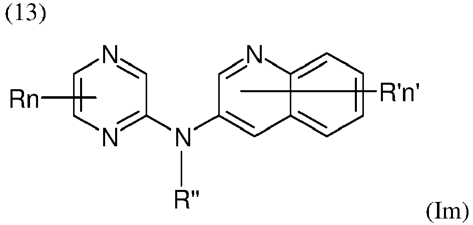
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom, 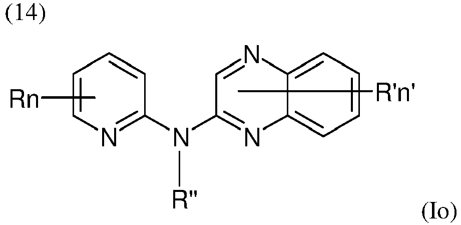
wherein:
R independently represent a hydrogen atom, a halogen atom or a group chosen among, a -NO₂ group, a -CN group and a (C₁-C₃)alkyl group, said alkyl being optionally mono-substituted by a hydroxyl group,
R" is as defined in claim 1 and is advantageously a hydrogen atom,
n is as defined in claim 1 and is advantageously 1,
n' is as defined in claim 1 and is advantageously 1,
R' is a hydrogen atom, a halogen atom or a (C₁-C₃)fluoroalkyl group, 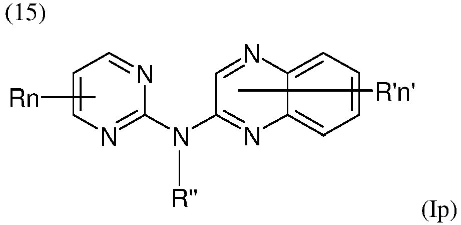
wherein:
R represents a hydrogen atom,
R" is as defined in claim 1 and is advantageously a hydrogen atom,
n is as defined in claim 1 and is advantageously 1,
n' is as defined in claim 1 and is advantageously 1,
R' is a hydrogen atom, 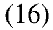 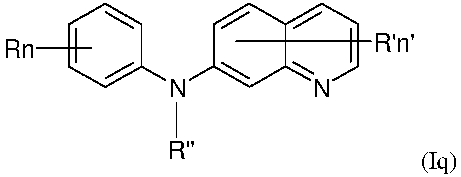
wherein:
R independently represent a hydrogen atom, a (C₁-C₃)alkoxy group or a (C₁-C₃)fluoroalkoxy group,
R" is as defined in claim 1 and is advantageously a hydrogen atom,
n is as defined in claim 1 and is advantageously 1,
n' is as defined in claim 1 and is advantageously 1,
R' is a hydrogen atom or a group chosen among a -NR₁R₂ group, a N-methylpiperazinyl group, a (C₁-C₃)alkoxy group and a morpholino group,
R₁ and R₂ are independently a hydrogen atom or a (C₁-C₃)alkyl group, 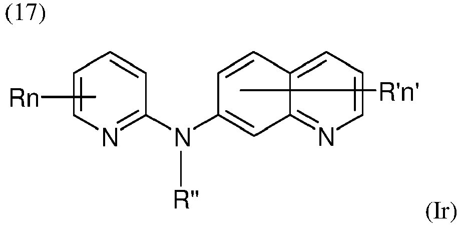
wherein:
R independently represent a hydrogen atom or a (C₁-C₃)alkyl group,
R" is as defined in claim 1 and is advantageously a hydrogen atom,
n is as defined in claim 1 and is advantageously 1,
n' is as defined in claim 1 and is advantageously 1,
R' is a hydrogen atom or a group chosen among a -NR₁R₂ group, a morpholino group and a (C₁-C₃)alkoxy group,
R₁ and R₂ are independently a hydrogen atom or a (C₁-C₃)alkyl group, (18)
or one of its pharmaceutically acceptable salt.

4. A compound of formula (I) according to anyone of the preceding claims, for use as an agent for preventing, inhibiting or treating pathological conditions linked with premature aging, which is alternatively chosen among: 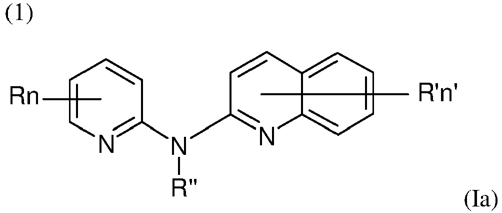 wherein:
R independently represent a hydrogen atom, a halogen atom or a group chosen among a (C₁-C₃)alkyl group, a -CN group, a -COOR₁ group, a (C₁-C₃)fluoroalkyl group, a - NO2 group and a (C₁-C₃)alkoxy group,
R₁ is a hydrogen atom or a (C₁-C₃)alkyl group,
R" is as defined in claim 1 and more preferably is a hydrogen atom,
n is as defined in claim 1 and more preferably is 1,
n' is as defined in claim 1,
R' is a hydrogen atom, a halogen atom or a (C₁-C₃)alkyl group, 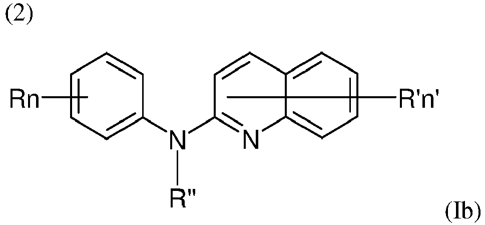
wherein:
R independently represent a hydrogen atom, a halogen atom or a group chosen among a (C₁-C₃)alkyl group, a (C₁-C₃)fluoroalkoxy group and a phenoxy group,
R₁ is a hydrogen atom or a (C₁-C₃)alkyl group,
R" is as defined in claim 1 and more preferably is a hydrogen atom,
n is as defined in claim 1 and more preferably is 1,
n' is as defined in claim 1,
R' is a hydrogen atom, 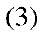 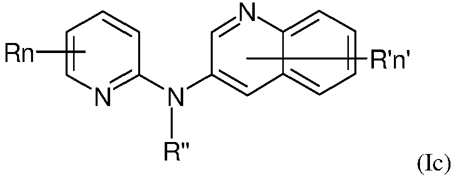
wherein:
R independently represent a hydrogen atom or a group chosen among a (C₁-C₃)alkyl group, a -NO₂ group and a (C₁-C₃)alkoxy group,
R" is as defined in claim 1 and more preferably is a hydrogen atom,
n is as defined in claim 1 and more preferably is 1,
n' is as defined in claim 1,
R' is a hydrogen atom, 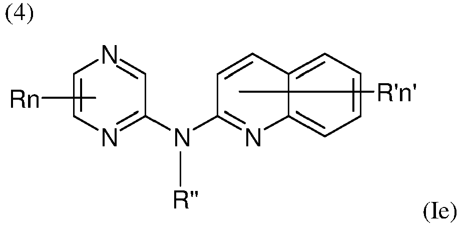
wherein:
R represents a hydrogen atom,
R" is as defined in claim 1 and is advantageously a hydrogen atom,
n is as defined in claim 1 and is advantageously 1,
n' is as defined in claim 1,
R' is a hydrogen atom or a halogen atom, 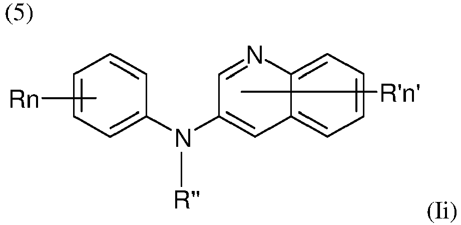
wherein:
R independently represent a hydrogen atom or a (C₁-C₃)alkoxy group,
R" is as defined above and is advantageously a hydrogen atom,
n is as defined above and is advantageously 1,
n' is as defined above and is advantageously 1,
R' is a hydrogen atom, 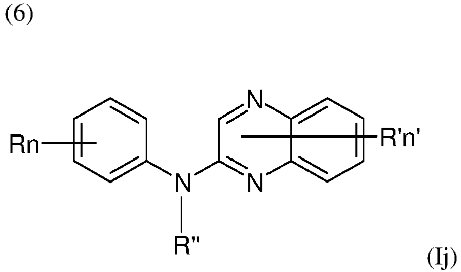
wherein:
R independently represent a hydrogen atom or a group chosen among a (C₁-C₃)fluoroalkoxy group and a (C₁-C₃)alkyl group,
R" is as defined in claim 1 and more preferably is a hydrogen atom,
n is as defined in claim 1 and more preferably is 2,
n' is as defined in claim 1,
R' is a hydrogen atom, 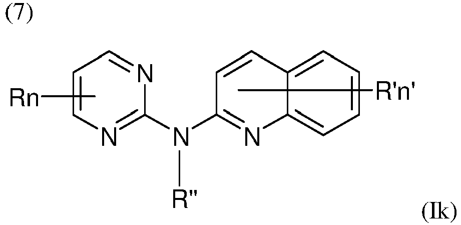
wherein:
R represents a hydrogen atom,
R" is as defined in claim 1 and more preferably is a hydrogen atom,
n is as defined in claim 1 and more preferably is 1,
n' is as defined in claim 1,
R' is a hydrogen atom, 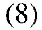 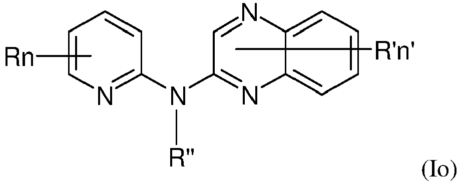
wherein:
R independently represent a hydrogen atom or a group chosen among a (C₁-C₃)alkyl group and a -CN group,
R" is as defined in claim 1 and more preferably is a hydrogen atom,
n is as defined in claim 1 and more preferably is 1,
n' is as defined in claim 1 and more preferably is 1,
R' is a hydrogen atom, (9)
or one of its pharmaceutically acceptable salt.

5. A compound of formula (I) according to anyone of the claims 1 to 3, which is alternatively chosen among,
(1) a compound of formula (Ig) as defined in claim 3
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group, and a (C₁-C₃)alkoxy group,
n is 1 or 2,
n' is 1 or 2,
R' is a hydrogen atom or a group chosen among a (C₁-C₃)alkyl group, a halogen atom, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
R₁ and R₂ are independently a hydrogen atom or a (C₁-C₃)alkyl group,
with the proviso that R and R' are not simultaneously a hydrogen atom,
and when n and n' are 1 and R is a hydrogen atom then R' is not a -COOH group,
or anyone of its pharmaceutically acceptable salt,
(2) A compound of formula (Ic) as defined in claim 3
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
n' is 1 or 2,
R" is as defined in formula (Ic) in claim 3,
R' is a hydrogen atom or a group chosen among a (C₁-C₃)alkyl group, a -NO₂ group, a -NR₁R₂ group and a (C₁-C₃)alkoxy group,
with the proviso that
R and R' are not simultaneously a hydrogen atom,
R is not a bromine atom when R' is a hydrogen atom,
or one of its pharmaceutically acceptable salt,
(3) A compound of formula (Id) as defined in claim 3
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group and a -NR₁R₂ group,
R₁ and R₂ are independently a hydrogen atom or a (C₁-C₃)alkyl group,
n is 1 or 2, and advantageously 1,
n' is 1 or 2,
R" is as defined in formula (I) in claim 1 and is advantageously a hydrogen atom,
R' is a hydrogen atom or a group chosen among a (C₁-C₃)alkyl group, a -NO₂ group, a -NR₁R₂ group and a (C₁-C₃)alkoxy group,
with the proviso that when R' is a hydrogen atom, R is different from a -NO₂ group, a -NH2 group or a -COOH group,
or one of its pharmaceutically acceptable salt,
(4) A compound of formula (Ii') 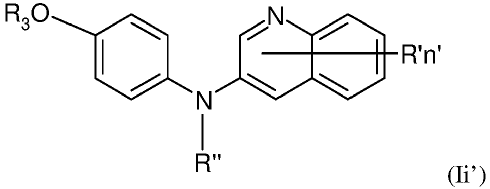 wherein:
R3 is a (C₁-C₃)fluoroalkyl group or a (C₁-C₃)alkyl group,
R' is a hydrogen atom, a halogen atom or a group chosen among a (C₁-C₃)alkyl group, a hydroxyl group, a -COOR₁ group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined in claim 1 and is advantageously a hydrogen atom,
n' is as defined in claim 1 and is advantageously 1,
with the proviso that
when R' is a hydrogen atom, R3 is not a methyl group or a trifluoromethyl group
or one of its pharmaceutically acceptable salt.

6. A compound of formula (I) according to anyone of the claims 1 to 3, which is alternatively chosen among,
(1) a compound of formula (Ik) as defined in claim 3
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy,
R₁ and R₂ are independently a hydrogen atom or a (C₁-C₃)alkyl group,
n is 1 or 2, and is advantageously 1,
n' is 1 or 2,
R" is as defined in formula (Ik) in claim 3,
R' is a hydrogen atom, a halogen atom or a group chosen among a (C₁-C₃)alkyl group, a hydroxyl group, a -COOR₁ group, a -NO₂ group, a -NR₁R₂ group, a (C₁-C₃)alkoxy group and a -CN group,
or one of its pharmaceutically acceptable salt,
(2) A compound of formula (Io) as defined in claim 3
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1, 2 or 3,
n' is 1 or 2,
R' is a hydrogen atom, a halogen atom or a group chosen among a (C₁-C₃)alkyl group, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
with the proviso that
when R is a hydrogen atom and n' is 1, R' is not a hydroxyl group,
or one of its pharmaceutically acceptable salt,
(3) A compound of formula (Ij') 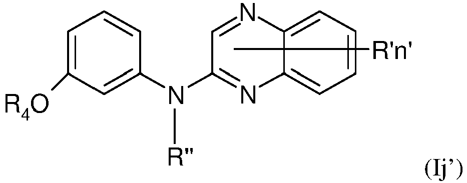 wherein:
R4 is a (C₁-C₃)fluoroalkyl group or a (C₁-C₃)alkyl group,
R' is a hydrogen atom, a halogen atom or a group chosen among a (C₁-C₃)alkyl group, a hydroxyl group, a -COOR₁ group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined in claim 1 and is advantageously a hydrogen atom,
n' is as defined in claim 1 and is advantageously 1,
with the proviso that
when R' is a hydrogen atom, R4 is not a methyl group
or one of its pharmaceutically acceptable salt,
(4) A compound of formula (Ij") 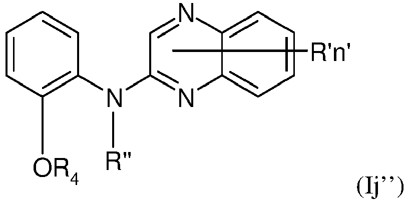 wherein:
R4 is a (C₁-C₃)fluoroalkyl group or a (C₁-C₃)alkyl group,
R' is a hydrogen atom, a halogen atom or a group chosen among a (C₁-C₃)alkyl group, a hydroxyl group, a -COOR₁ group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined in claim 1 and is advantageously a hydrogen atom,
n' is as defined in claim 1 and is advantageously 1,
or one of its pharmaceutically acceptable salt,
(5) A compound of formula (Ij‴) 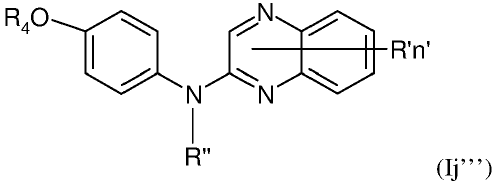 wherein:
R4 is a (C₁-C₃)fluoroalkyl group or a (C₁-C₃)alkyl group,
R' is a hydrogen atom, a halogen atom or a group chosen among a (C₁-C₃)alkyl group, a hydroxyl group, a -NO₂ group, a -NR₁R₂ group, a (C₁-C₃)alkoxy group and a -CN group,
R₁ and R₂ are independently a hydrogen atom or a (C₁-C₃)alkyl group,
R" is as defined in claim 1 and is advantageously a hydrogen atom,
n' is as defined in claim 1 and is advantageously 1,
with the proviso that
when R' is a chlorine atom or a hydrogen atom, R₄ is not an ethyl group or a methyl group,
when R' is a methyl group or a tertio-butyl group, R4 is not a methyl group,
or one of its pharmaceutically acceptable salt.

7. A compound of formula (I) according to anyone of the claims 1 to 3, which is alternatively chosen among,
(1) a compound of formula (Ip) as defined in claim 3
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
n' is 1 or 2,
R' is a hydrogen atom, a halogen atom or a group chosen among a (C₁-C₃)alkyl group, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
with the proviso that
wherein R and R' are not simultaneously a hydrogen atom,
when n and n' are 2 then R and R' are not simultaneously a methyl group.
or one of its pharmaceutically acceptable salt,
(2) A compound of formula (Ir) as defined in claim 3
wherein:
R independently represent a hydrogen atom, a halogen atom or a group chosen among a (C₁-C₃)alkyl group, a -CN group, a hydroxyl group, a -COOR₁ group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
n' is 1 or 2,
R' is a hydrogen atom, a halogen atom or a group chosen among a (C₁-C₃)alkyl group, a hydroxyl group, a -COOR₁ group, a -NO₂ group, a -NR₁R₂ group, a (C₁-C₃)alkoxy group and a -CN group,
R" is a hydrogen atom or a (C₁-C₄)alkyl group,
or one of its pharmaceutically acceptable salt.

8. A compound for use according to any one of claims 1 to 4, said compound being chosen among:
- (1) (8-Chloro-quinolin-2-yl)-pyridin-2-yl-amine
- (2) 2-(Quinolin-2-ylamino)-isonicotinic acid
- (3) (4-Methyl-pyridin-2-yl)-quinolin-2-yl-amine
- (4) Pyridin-2-yl-quinolin-2-yl-amine
- (5) 2-(8-Chloro-quinolin-2-ylamino)-isonicotinic acid
- (6) (8-Chloro-quinolin-2-yl)-(4-methyl-pyridin-2-yl)-amine
- (7) 6-(Quinolin-2-ylamino)-nicotinonitrile
- (8) Quinolin-2-yl-(4-trifluoromethoxy-phenyl)-amine
- (9) Pyridin-2-yl-quinolin-3-yl-amine
- (10) (3-Methoxy-pyridin-2-yl)-quinolin-3-yl-amine
- (11) Quinolin-3-yl-(5-trifluoromethyl-pyridin-2-yl)-amine
- (12) (5-Nitro-pyridin-2-yl)-quinolin-3-yl-amine
- (13) (5-Methyl-pyridin-2-yl)-quinolin-3-yl-amine
- (14) 2-(Quinolin-3-ylamino)-isonicotinic acid
- (15) Quinolin-6-yl-(5-trifluoromethyl-pyridin-2-yl)-amine
- (16) (6-Methyl-pyridin-2-yl)-quinolin-6-yl-amine
- (17) N-(6-methylpyridin-2-yl)quinolin-2-amine
- (18) 8-chloro-N-(6-methylpyridin-2-yl)quinolin-2-amine
- (19) 4-methyl-N-(pyridin-2-yl)quinolin-2-amine
- (20) 4-methyl-N-(4-methylpyridin-2-yl)quinolin-2-amine
- (21) 3-methyl-N-(4-methylpyridin-2-yl)quinolin-2-amine
- (22) 3-methyl-N-(pyridin-2-yl)quinolin-2-amine
- (23) 6-((4-methylquinolin-2-yl)amino)nicotinonitrile
- (24) 6-((3-methylquinolin-2-yl)amino)nicotinonitrile
- (25) 6-chloro-N-(4-methylpyridin-2-yl)quinolin-2-amine
- (26) 6-chloro-N-(6-methylpyridin-2-yl)quinolin-2-amine
- (27) 4-methyl-N-(5-nitropyridin-2-yl)quinolin-2-amine
- (28) N-(3-nitropyridin-2-yl)quinolin-2-amine
- (29) 8-chloro-N-(3-nitropyridin-2-yl)quinolin-2-amine
- (30) 2-((4-methylquinolin-2-yl)amino)nicotinonitrile
- (31) N-(3-methylpyridin-2-yl)quinolin-2-amine
- (32) N-(5-methylpyridin-2-yl)quinolin-2-amine
- (33) 2-(quinolin-2-ylamino)isonicotinonitrile
- (34) N-(5-(trifluoromethyl)pyridin-2-yl)quinolin-2-amine
- (35) 8-chloro-N-(3-methylpyridin-2-yl)quinolin-2-amine
- (36) 8-chloro-N-(5-methylpyridin-2-yl)quinolin-2-amine
- (37) 8-chloro-N-(5-(trifluoromethyl)pyridin-2-yl)quinolin-2-amine
- (38) N-(3-methoxypyridin-2-yl)quinolin-2-amine
- (39) N-(5-nitropyridin-2-yl)quinolin-2-amine
- (40) 6-((8-chloroquinolin-2-yl)amino)nicotinonitrile
- (41) N-(5-fluoropyridin-2-yl)quinolin-2-amine
- (42) N-(6-(trifluoromethyl)pyridin-2-yl)quinolin-2-amine
- (43) 8-chloro-N-(5-fluoropyridin-2-yl)quinolin-2-amine
- (44) 2-((8-chloroquinolin-2-yl)amino)nicotinic acid
- (45) 4-methyl-N-(6-methylpyridin-2-yl)quinolin-2-amine
- (46) 3-methyl-N-(6-methylpyridin-2-yl)quinolin-2-amine
- (47) 5-cyano-2-(quinolin-2-ylamino)pyridin-1-ium chloride
- (48) 2-((8-chloroquinolin-2-yl)amino)-4-methylpyridin-1-ium chloride
- (49) 8-chloro-N-(4-ethylpyridin-2-yl)quinolin-2-amine
- (50) 8-chloro-N-(6-ethylpyridin-2-yl)quinolin-2-amine
- (51) 8-chloro-N-(4,6-dimethylpyridin-2-yl)quinolin-2-amine
- (52) 6-((8-chloroquinolin-2-yl)amino)-2-methylnicotinonitrile
- (53) 8-chloro-N-(4-chloropyridin-2-yl)quinolin-2-amine
- (54) 8-methyl-N-(4-methylpyridin-2-yl)quinolin-2-amine
- (55) N-(5-bromo-4-methylpyridin-2-yl)-8-chloroquinolin-2-amine
- (56) 8-chloro-N-(3-ethyl-6-methylpyridin-2-yl)quinolin-2-amine
- (57) 8-fluoro-N-(4-methylpyridin-2-yl)quinolin-2-amine
- (58) 8-bromo-N-(4-methylpyridin-2-yl)quinolin-2-amine
- (59) methyl 6-(quinolin-2-ylamino)nicotinate
- (60) methyl 6-[(8-chloroquinolin-2-yl)amino]pyridine-3-carboxylate
- (61) methyl 6-[(3-methylquinolin-2-yl)amino]pyridine-3-carboxylate
- (62) methyl 2-[(8-chloroquinolin-2-yl)amino]pyridine-3-carboxylate
- (63) 8-methoxy-N-(4-methylpyridin-2-yl)quinolin-2-amine
- (64) N-(4-methylpyridin-2-yl)-5-nitroquinolin-2-amine
- (65) 2-N-(4-methylpyridin-2-yl)quinoline-2,8-diamine
- (66) N-(4-methylpyridin-2-yl)-5-aminoquinolin-2-amine
- (67) methyl 6-[(4-methylquinolin-2-yl)amino]pyridine-3-carboxylate
- (68) 8-chloro-N-[4-(trifluoromethyl)pyridin-2-yl]quinolin-2-amine
- (69) 2-[(8-chloroquinolin-2-yl)amino]pyridin-3-ol
- (70) 8-chloro-N-[6-(trifluoromethyl)pyridin-2-yl]quinolin-2-amine
- (71) 6-chloro-N-(5-fluoropyridin-2-yl)quinolin-2-amine
- (72) N-(6-ethylpyridin-2-yl)-3-methylquinolin-2-amine
- (73) N-(5-fluoropyridin-2-yl)-3-methylquinolin-2-amine
- (74) 3-methyl-N-[5-(trifluoromethyl)pyridin-2-yl]quinolin-2-amine
- (75) 4-N-(8-chloroquinolin-2-yl)-1-N,1-N-dimethylbenzene-1,4-diamine
- (76) N-(4-methoxyphenyl)quinolin-2-amine
- (77) 8-chloro-N-(4-methoxyphenyl)quinolin-2-amine
- (78) 4-methyl-N-[4-(trifluoromethoxy)phenyl]quinolin-2-amine
- (79) N-(4-methoxyphenyl)-3-methylquinolin-2-amine
- (80) 3-methyl-N-[4-(trifluoromethoxy)phenyl]quinolin-2-amine
- (81) 1-N,1-N-dimethyl-4-N-(3 -methylquinolin-2-yl)benzene-1,4-diamine
- (82) N-[2-methyl-4-(trifluoromethoxy)phenyl]quinolin-2-amine
- (83) N-[3-(trifluoromethoxy)phenyl]quinolin-2-amine
- (84) N-[2-(trifluoromethoxy)phenyl]quinolin-2-amine
- (85) N-(4-nitrophenyl)quinolin-2-amine
- (86) N-(3-fluorophenyl)quinolin-2-amine
- (87) 8-chloro-N-[3-(trifluoromethoxy)phenyl]quinolin-2-amine
- (88) 8-chloro-N-(3-fluorophenyl)quinolin-2-amine
- (89) 2-{[4-(trifluoromethoxy)phenyl]amino}quinolin-1-ium chloride
- (90) 8-chloro-N-[4-(trifluoromethoxy)phenyl]quinolin-2-amine
- (91) 3-methyl-N-[2-methyl-4-(trifluoromethoxy)phenyl]quinolin-2-amine
- (92) 3-methyl-N-[3-(trifluoromethoxy)phenyl]quinolin-2-amine
- (93) 3-methyl-N-[2-(trifluoromethoxy)phenyl]quinolin-2-amine
- (94) 8-chloro-N-[2-methyl-4-(trifluoromethoxy)phenyl]quinolin-2-amine
- (95) 3-methyl-2-{[4-(trifluoromethoxy)phenyl]amino}quinolin-1-ium chloride
- (96) 6-chloro-N-(4-(trifluoromethoxy)phenyl)quinolin-2-amine
- (97) 4-methyl-2-{[4-(trifluoromethoxy)phenyl]amino}quinolin-1-ium chloride
- (98) 8-bromo-N-[4-(trifluoromethoxy)phenyl]quinolin-2-amine
- (99) 8-fluoro-N-[4-(trifluoromethoxy)phenyl]quinolin-2-amine
- (100) 8-methyl-N-[4-(trifluoromethoxy)phenyl]quinolin-2-amine
- (101) N-(4-butoxyphenyl)-8-chloroquinolin-2-amine
- (102) N-(4-phenoxyphenyl)quinolin-2-amine
- (103) 8-methoxy-N-[4-(trifluoromethoxy)phenyl]quinolin-2-amine
- (104) 8-chloro-N-[3-chloro-4-(trifluoromethoxy)phenyl]quinolin-2-amine
- (105) N-(6-methylpyridin-2-yl)quinolin-3-amine
- (106) N-(3-nitropyridin-2-yl)quinolin-3-amine
- (107) N-(5-methylpyridin-2-yl)quinolin-6-amine
- (108) N-(3-methoxypyridin-2-yl)quinolin-6-amine
- (109) 6-chloro-N-(pyrazin-2-yl)quinolin-2-amine
- (110) 8-bromo-N-(pyrazin-2-yl)quinolin-2-amine
- (111) 8-methyl-N-(pyrazin-2-yl)quinolin-2-amine
- (112) 8-chloro-N-(pyrazin-2-yl)quinolin-2-amine
- (113) N-(pyrazin-2-yl)quinolin-2-amine
- (114) 4-methyl-N-(pyrazin-2-yl)quinolin-2-amine
- (115) 3-methyl-N-(pyrazin-2-yl)quinolin-2-amine
- (116) 8-fluoro-N-(pyrazin-2-yl)quinolin-2-amine
- (117) 8-methoxy-N-(pyrazin-2-yl)quinolin-2-amine
- (118) N-(pyridin-3-yl)quinolin-3-amine
- (119) 8-chloro-N-(pyridin-4-yl)quinolin-2-amine
- (120) N-(pyridin-4-yl)quinolin-2-amine
- (121) N-(pyridin-4-yl)quinolin-3-amine
- (122) N-[4-(trifluoromethoxy)phenyl]quinolin-3-amine
- (123) N-(4-methoxyphenyl)quinolin-3-amine
- (124) N-[4-(trifluoromethoxy)phenyl]quinoxalin-2-amine
- (125) N-[2-methyl-4-(trifluoromethoxy)phenyl]quinoxalin-2-amine
- (126) N-[3-(trifluoromethoxy)phenyl]quinoxalin-2-amine
- (127) N-[2-(trifluoromethoxy)phenyl]quinoxalin-2-amine
- (128) N-(pyrimidin-2-yl)quinolin-2-amine
- (129) 8-chloro-N-(pyrimidin-2-yl)quinolin-2-amine
- (130) 4-methyl-N-(pyrimidin-2-yl)quinolin-2-amine
- (131) N-(pyrazin-2-yl)quinolin-6-amine
- (132) N-(pyrazin-2-yl)quinolin-3-amine
- (133) 6-methyl-N-(naphthalen-2-yl)pyridin-2-amine
- (134) N-(naphthalen-2-yl)pyridin-2-amine
- (135) N-(pyridin-2-yl)quinoxalin-2-amine
- (136) N-(4-methylpyridin-2-yl)quinoxalin-2-amine
- (137) 6-(quinoxalin-2-ylamino)pyridine-3-carbonitrile
- (138) N-(6-methylpyridin-2-yl)quinoxalin-2-amine
- (139) N-(4-methylpyridin-2-yl)-3-(trifluoromethyl)quinoxalin-2-amine
- (140) N-(3,5-dichloro-4-methylpyridin-2-yl)quinoxalin-2-amine
- (141) N-(4-methyl-3-nitropyridin-2-yl)quinoxalin-2-amine
- (142) N-(pyrimidin-2-yl)quinoxalin-2-amine
- (143) 4-N,4-N-dimethyl-7-N-[4-(trifluoromethoxy)phenyl]quinoline-4,7-diamine
- (144) 4-(morpholin-4-yl)-N-[4-(trifluoromethoxy)phenyl]quinolin-7-amine
- (145) 4-methoxy-N-(pyridin-2-yl)quinolin-7-amine
- (146) 4-methoxy-N-(4-methylpyridin-2-yl)quinolin-7-amine
- (147) 4-N,4-N-dimethyl-7-N-(4-methylpyridin-2-yl)quinoline-4,7-diamine
- (150) N-(4-methylpyridin-2-yl)-8-nitroquinolin-2-amine
- (151) 6-chloro-N-(6-ethylpyridin-2-yl)quinolin-2-amine
- (152) 6-chloro-N-(5-methylpyridin-2-yl)quinolin-2-amine
- (153) 6-chloro-N-[5-(trifluoromethyl)pyridin-2-yl]quinolin-2-amine
- (154) N2-(8-chloroquinolin-2-yl)-4-methylpyridine-2,3-diamine
- (155) N-(4-butoxyphenyl)-3-methylquinolin-2-amine
- (156) 4-N-(6-chloroquinolin-2-yl)-1-N,1-N-dimethylbenzene-1,4-diamine
- (157) 8-chloro-N-(3-chloro-4-methoxyphenyl)quinolin-2-amine
- (158) N1-(8-chloroquinolin-2-yl)-4-(trifluoromethoxy)benzene-1,2-diamine
- (159) N-(3-aminopyridin-2-yl)quinolin-3-amine
- (160) 6-chloro-N-(4-methylpyridin-2-yl)quinoxalin-2-amine
- (161) N-(4-ethylpyridin-2-yl)quinoxalin-2-amine
- (162) N-(5-bromo-4-methylpyridin-2-yl)quinoxalin-2-amine
- (163) N-(4,6-dimethylpyridin-2-yl)quinoxalin-2-amine
- (164) [2-(quinoxalin-2-ylamino)pyridin-4-yl]methanol
- (165) N-(4-methyl-5-nitropyridin-2-yl)quinoxalin-2-amine
- (166) N-(4-methoxyphenyl)-4-(4-methylpiperazin-1-yl)quinolin-7-amine
- (167) 4-methoxy-N-[4-(trifluoromethoxy)phenyl]quinolin-7-amine
- (168) N-(4-methylpyridin-2-yl)-4-(morpholin-4-yl)quinolin-7-amine
- and their pharmaceutically acceptable salts.

9. A compound according to any one of claims 5, 6, and 7, said compound being chosen among the compounds:
- (10) (3-Methoxy-pyridin-2-yl)-quinolin-3-yl-amine
- (11) Quinolin-3-yl-(5-trifluoromethyl-pyridin-2-yl)-amine
- (12) (5-Nitro-pyridin-2-yl)-quinolin-3-yl-amine
- (13) (5-Methyl-pyridin-2-yl)-quinolin-3-yl-amine
- (14) 2-(Quinolin-3-ylamino)-isonicotinic acid
- (15) Quinolin-6-yl-(5-trifluoromethyl-pyridin-2-yl)-amine
- (16) (6-Methyl-pyridin-2-yl)-quinolin-6-yl-amine
- (105) N-(6-methylpyridin-2-yl)quinolin-3-amine
- (106) N-(3-nitropyridin-2-yl)quinolin-3-amine
- (107) N-(5-methylpyridin-2-yl)quinolin-6-amine
- (108) N-(3-methoxypyridin-2-yl)quinolin-6-amine
- (119) 8-chloro-N-(pyridin-4-yl)quinolin-2-amine
- (124) N-[4-(trifluoromethoxy)phenyl]quinoxalin-2-amine
- (125) N-[2-methyl-4-(trifluoromethoxy)phenyl]quinoxalin-2-amine
- (126) N-[3-(trifluoromethoxy)phenyl]quinoxalin-2-amine
- (127) N-[2-(trifluoromethoxy)phenyl]quinoxalin-2-amine
- (128) N-(pyrimidin-2-yl)quinolin-2-amine
- (129) 8-chloro-N-(pyrimidin-2-yl)quinolin-2-amine
- (130) 4-methyl-N-(pyrimidin-2-yl)quinolin-2-amine
- (135) N-(pyridin-2-yl)quinoxalin-2-amine
- (136) N-(4-methylpyridin-2-yl)quinoxalin-2-amine
- (137) 6-(quinoxalin-2-ylamino)pyridine-3-carbonitrile
- (138) N-(6-methylpyridin-2-yl)quinoxalin-2-amine
- (139) N-(4-methylpyridin-2-yl)-3-(trifluoromethyl)quinoxalin-2-amine
- (140) N-(3,5-dichloro-4-methylpyridin-2-yl)quinoxalin-2-amine
- (141) N-(4-methyl-3-nitropyridin-2-yl)quinoxalin-2-amine
- (145) 4-methoxy-N-(pyridin-2-yl)quinolin-7-amine
- (146) 4-methoxy-N-(4-methylpyridin-2-yl)quinolin-7-amine
- (147) 4-N,4-N-dimethyl-7-N-(4-methylpyridin-2-yl)quinoline-4,7-diamine
- (159) N-(3-aminopyridin-2-yl)quinolin-3-amine
- (160) 6-chloro-N-(4-methylpyridin-2-yl)quinoxalin-2-amine
- (161) N-(4-ethylpyridin-2-yl)quinoxalin-2-amine
- (162) N-(5-bromo-4-methylpyridin-2-yl)quinoxalin-2-amine
- (163) N-(4,6-dimethylpyridin-2-yl)quinoxalin-2-amine
- (164) [2-(quinoxalin-2-ylamino)pyridin-4-yl]methanol
- (165) N-(4-methyl-5-nitropyridin-2-yl)quinoxalin-2-amine, and
- (168) N-(4-methylpyridin-2-yl)-4-(morpholin-4-yl)quinolin-7-amine
as well as their pharmaceutically acceptable salts, such as hydrochloride, hydrobromide, tartrate, fumarate, citrate, trifluoroacetate, ascorbate, triflate, mesylate, tosylate, formate, acetate and malate.

10. A pharmaceutical composition comprising at least one compound as defined in anyone of claims 5, 6, 7 and 9.

## Patentansprüche

1. Verbindung der Formel (I): 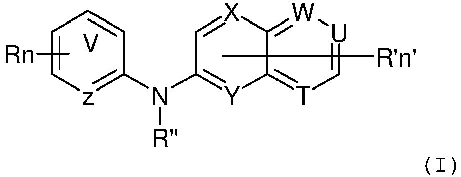 wobei: 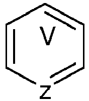
für einen aromatischen Ring steht, wobei V für C oder N steht und dann, wenn V für N steht, V in ortho-, meta- oder para-Position zu Z steht, d. h. eine Pyridazin-, eine Pyrimidin- bzw. eine Pyrazingruppe bildet,
R unabhängig für ein Wasserstoffatom, ein Halogenatom oder eine Gruppe, die aus einer -CN-Gruppe, einer Hydroxylgruppe, einer -COOR₁-Gruppe, einer (C₁-C₃)Fluoralkylgruppe, einer (C₁-C₃)Fluoralkoxygruppe, einer -NO₂-Gruppe, einer -NR₁R₂-Gruppe, einer (Ci-C₄)Alkoxygruppe, einer Phenoxygruppe und einer (Ci-C₃)Alkylgruppe, wobei das Alkyl gegebenenfalls einfach durch eine Hydroxylgruppe substituiert sein kann, ausgewählt ist, steht,
R₁ und R₂ unabhängig für ein Wasserstoffatom oder eine (C₁-C₃)Alkylgruppe stehen,
n für 1, 2 oder 3 steht,
n' für 1 oder 2 steht,
R' für ein Wasserstoffatom, ein Halogenatom oder eine Gruppe, die aus einer (C₁-C₃)Alkyl-Gruppe, einer Hydroxylgruppe, einer -COOR₁-Gruppe, einer -NO₂-Gruppe, einer -NR₁R₂-Gruppe, einer Morpholinyl- oder einer Morpholinogruppe, einer N-Methylpiperazinylgruppe, einer (C₁-C₃)Fluoralkylgruppe, einer (C₁-C₄)Alkoxygruppe und einer -CN-Gruppe ausgewählt ist, steht,
R" für ein Wasserstoffatom oder eine (C₁-C₄)Alkylgruppe steht,
- Z für N steht, V für C steht, Y für N steht, X für C steht, T für C steht, U für C steht und W für C steht,
- Z für C steht, V für C steht, Y für C steht, X für C steht, T für C steht, U für C steht und W für C steht,
- Z für N steht, V für C steht, Y für C steht, X für N steht, T für C steht, U für C steht und W für C steht,
- Z für N steht, V für C steht, Y für C steht, X für C steht, T für C steht, U für C steht und W für N steht,
- Z für N steht, V für N steht und in para-Position zu Z steht, Y für N steht, X für C steht, T für C steht, U für C steht und W für C steht,
- Z für C steht, V für N steht und in para-Position zu Z steht, Y für C steht, X für N steht, T für C steht, U für C steht und W für C steht,
- Z für C steht, V für N steht und in meta-Position zu Z steht und in para-Position zu der an NR" geknüpften Bindung steht, Y für N steht, X für C steht, T für C steht, U für C steht und W für C steht,
- Z für C steht, V für N steht und in meta-Position zu Z steht und in para-Position zu der an NR" geknüpften Bindung steht, Y für C steht, X für N steht, T für C steht, U für C steht und W für C steht,
- Z für C steht, V für C steht, Y für C steht, X für N steht, T für C steht, U für C steht und W für C steht,
- Z für C steht, V für C steht, Y für N steht, X für N steht, T für C steht, U für C steht und W für C steht,
- Z für N steht, V für N steht und in meta-Position zu Z steht und in ortho-Position zu der an NR" geknüpften Bindung steht, Y für N steht, X für C steht, T für C steht, U für C steht und W für C steht,
- Z für N steht, V für N steht und in para-Position zu Z steht, Y für C steht, X für C steht, T für C steht, U für C steht und W für N steht,
- Z für N steht, V für N steht und in para-Position zu Z steht, Y für C steht, X für N steht, T für C steht, U für C steht und W für C steht,
- Z für N steht, V für C steht, Y für N steht, X für N steht, T für C steht, U für C steht und W für C steht,
- Z für N steht, V für N steht und in meta-Position zu Z steht und in ortho-Position zu der an NR" geknüpften Bindung steht, Y für N steht, X für N steht, T für C steht, U für C steht und W für C steht,
- Z für C steht, V für C steht, Y für C steht, X für C steht, T für N steht, U für C steht und W für C steht oder
- Z für N steht, V für C steht, Y für C steht, X für C steht, T für N steht, U für C steht und W für C steht,
oder ein pharmazeutisch unbedenkliches Salz davon
zur Verwendung als Mittel zur Prävention, Inhibierung oder Behandlung von Leiden, die mit vorzeitiger Alterung verbunden sind.

2. Verbindung der Formel (I) nach Anspruch 1, wobei alternativ:
- Z für N steht, V für C steht, Y für N steht, X für C steht, T für C steht, U für C steht und W für C steht,
- Z für C steht, V für C steht, Y für N steht, X für C steht, T für C steht, U für C steht und W für C steht,
- Z für N steht, V für C steht, Y für C steht, X für N steht, T für C steht, U für C steht und W für C steht,
- Z für N steht, V für C steht, Y für C steht, X für C steht, T für C steht, U für C steht und W für N steht,
- Z für N steht, V für N steht und in para-Position zu Z steht, Y für N steht, X für C steht, T für C steht, U für C steht und W für C steht,
- Z für C steht, V für C steht, Y für C steht, X für N steht, T für C steht, U für C steht und W für C steht,
- Z für C steht, V für C steht, Y für N steht, X für N steht, T für C steht, U für C steht und W für C steht,
- Z für N steht, V für N steht und in meta-Position zu Z steht und in ortho-Position zu der an NR" geknüpften Bindung steht, Y für N steht, X für C steht, T für C steht, U für C steht und W für C steht,
- Z für N steht, V für C steht, Y für N steht, X für N steht, T für C steht, U für C steht und W für C steht,
- Z für N steht, V für N steht und in meta-Position zu Z steht und in ortho-Position zu der an NR" geknüpften Bindung steht Y für N steht, X für N steht, T für C steht, U für C steht und W für C steht oder
- Z für N steht, V für C steht, Y für C steht, X für C steht, T für N steht, U für C steht und W für C steht,
zur Verwendung als Mittel zur Prävention, Inhibierung oder Behandlung von Leiden, die mit vorzeitiger Alterung verbunden sind.

3. Verbindung der Formel (I) nach einem der vorhergehenden Ansprüche zur Verwendung als Mittel zur Prävention, Inhibierung oder Behandlung von Leiden, die mit vorzeitiger Alterung verbunden sind, die alternativ ausgewählt ist aus:
(1) 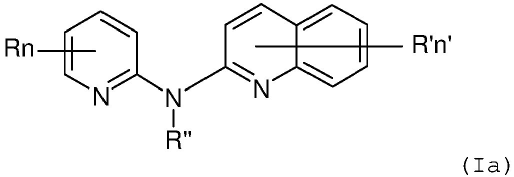 wobei:
R unabhängig für ein Wasserstoffatom, ein Halogenatom oder eine Gruppe, die aus einer (C₁-C₃) Alkylgruppe, einer -CN-Gruppe, einer Hydroxylgruppe, einer -COOR₁-Gruppe, einer (C₁-C₃) Fluoralkylgruppe, einer -NO₂-Gruppe, einer -NR₁R₂-Gruppe und einer (C₁-C₃)Alkoxygruppe ausgewählt ist, steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom, ein Halogenatom oder eine Gruppe, die aus einer (C₁-C₃)Alkylgruppe, einer - NO₂-Gruppe, einer (C₁-C₃) Alkoxygruppe und einer -NR₁R₂-Gruppe ausgewählt ist, steht,
R₁ und R₂ für ein Wasserstoffatom oder eine (C₁-C₃) Alkylgruppe stehen,
(2) 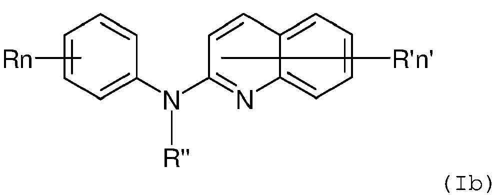 wobei:
R unabhängig für ein Wasserstoffatom, ein Halogenatom oder eine Gruppe, die aus einer (C₁-C₃) Alkylgruppe, einer NR₁R₂-Gruppe, einer (C₁-C₃) Fluoralkoxygruppe, einer -NO₂-Gruppe, einer Phenoxygruppe und einer (C₁-C₄)Alkoxygruppe ausgewählt ist, steht,
R₁ und R₂ unabhängig für ein Wasserstoffatom oder eine (C₁-C₃)Alkylgruppe stehen,
R" wie in Anspruch 1 definiert ist und vorteilhafterweise für ein Wasserstoffatom steht,
n wie in Anspruch 1 definiert ist und vorteilhafterweise für 1 oder 2 steht,
n' wie in Anspruch 1 definiert ist und vorteilhafterweise für 1 steht,
R' für ein Wasserstoffatom, ein Halogenatom oder eine Gruppe, die aus einer (C₁-C₃)Alkylgruppe und einer (C₁-C₄)Alkoxygruppe ausgewählt ist, steht,
(3) 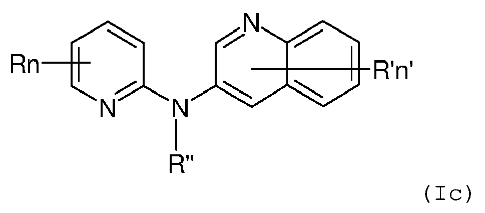 wobei:
R unabhängig für ein Wasserstoffatom oder eine Gruppe, die aus einer (C₁-C₃) Alkylgruppe, einer (C₁-C₃) Fluoralkylgruppe, einer NR₁R₂-Gruppe, einer -COOR₁-Gruppe, einer -NO₂-Gruppe und einer (C₁-C₃)Alkoxygruppe ausgewählt ist, steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
R₁ und R₂ für ein Wasserstoffatom oder eine (C₁-C₃) Alkylgruppe stehen,
(4) 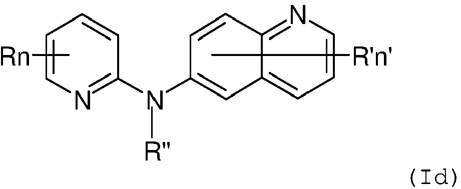 wobei:
R unabhängig für ein Wasserstoffatom oder eine Gruppe, die aus einer (C₁-C₃) Alkylgruppe, einer (C₁-C₃) Fluoralkylgruppe und einer (C₁-C₃) Alkoxygruppe ausgewählt ist, steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(5) 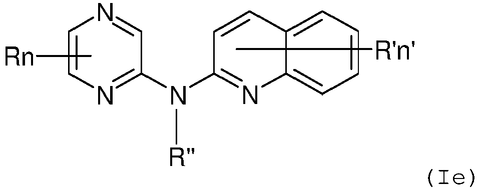 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom, ein Halogenatom oder eine Gruppe, die aus einer (C₁-C₃)Alkylgruppe und einer (C₁-C₃)Alkoxygruppe ausgewählt ist, steht,
(6) 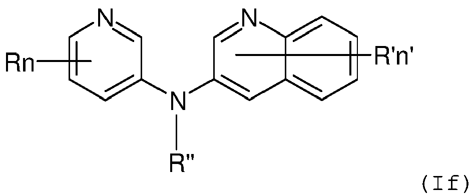 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(7) 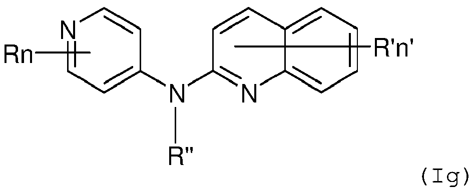 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom oder ein Halogenatom steht,
(8) 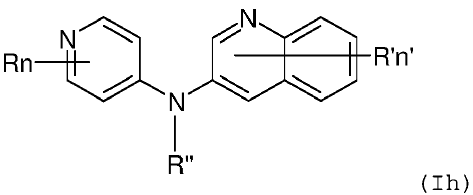 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(9) 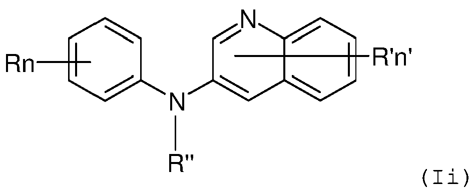 wobei:
R unabhängig für ein Wasserstoffatom oder eine Gruppe, die aus einer (C₁-C₃)Fluoralkoxygruppe und einer (C₁-C₃)Alkoxygruppe ausgewählt ist, steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(10) 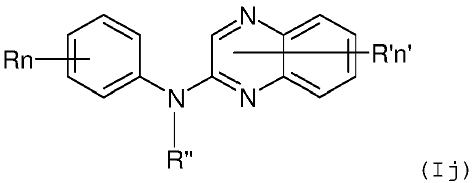 wobei:
R unabhängig für ein Wasserstoffatom oder eine Gruppe, die aus einer (C₁-C₃)Fluoralkoxygruppe und einer (C₁-C₃)Alkylgruppe ausgewählt ist, steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(11) 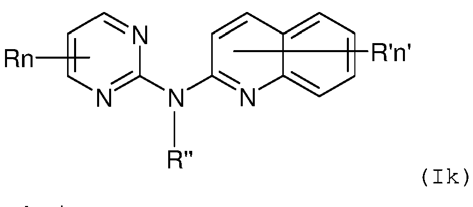 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom, ein Halogenatom oder eine (C₁-C₃)Alkylgruppe steht,
(12) 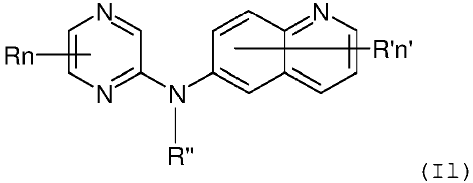 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(13) 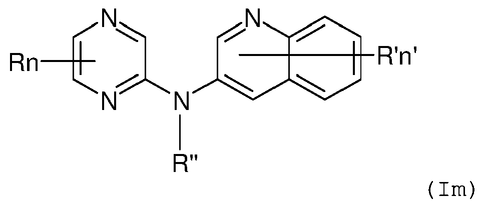 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(14) 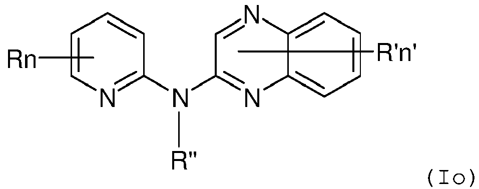 wobei:
R unabhängig für ein Wasserstoffatom, ein Halogenatom oder eine Gruppe, die aus einer -NO₂-Gruppe, einer -CN-Gruppe und einer (C₁-C₃)Alkylgruppe, wobei das Alkyl gegebenenfalls einfach durch eine Hydroxylgruppe substituiert sein kann, ausgewählt ist, steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom, ein Halogenatom oder eine (C₁-C₃)Fluoralkylgruppe steht,
(15) 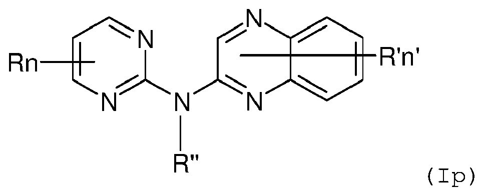 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom steht,
(16) 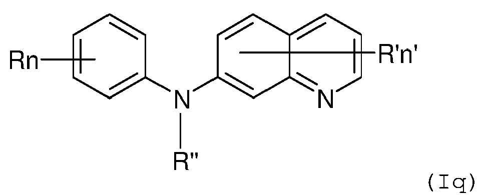 wobei:
R unabhängig für ein Wasserstoffatom, eine (C₁-C₃)Alkoxygruppe oder eine (C₁-C₃)Fluoralkylgruppe steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom oder eine Gruppe, die aus einer -NR₁R₂-Gruppe, einer N-Methylpiperazinylgruppe, einer (C₁-C₃)Alkoxygruppe und einer Morpholinogruppe ausgewählt ist, steht,
R₁ und R₂ für ein Wasserstoffatom oder eine (C₁-C₃) Alkylgruppe stehen,
(17) 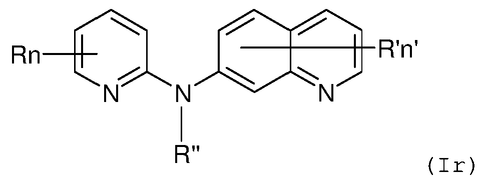 wobei:
R unabhängig für ein Wasserstoffatom oder eine (C₁-C₃)Alkylgruppe steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist und vorteilhafterweise für 1 steht,
R' für ein Wasserstoffatom oder eine Gruppe, die aus einer -NR₁R₂-Gruppe, einer Morpholinogruppe und einer (C₁-C₃)Alkoxygruppe ausgewählt ist, steht,
R₁ und R₂ unabhängig für ein Wasserstoffatom oder eine (C₁-C₃)Alkylgruppe stehen,
(18)
oder einem pharmazeutisch unbedenklichen Salz davon.

4. Verbindung der Formel (I) nach einem der vorhergehenden Ansprüche zur Verwendung als Mittel zur Prävention, Inhibierung oder Behandlung von Leiden, die mit vorzeitiger Alterung verbunden sind, die alternativ ausgewählt ist aus:
(1) 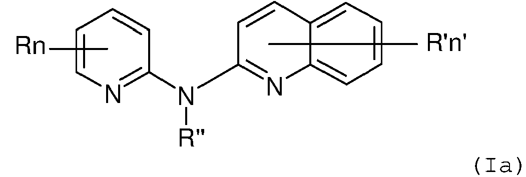 wobei:
R unabhängig für ein Wasserstoffatom, ein Halogenatom oder eine Gruppe, die aus einer (C₁-C₃) Alkylgruppe, einer -CN-Gruppe, einer -COOR₁-Gruppe, einer (C₁-C₃)Fluoralkylgruppe, einer -NO₂-Gruppe und einer (C₁-C₃)Alkoxygruppe ausgewählt ist, steht,
R₁ für ein Wasserstoffatom oder eine (C₁-C₃) Alkylgruppe steht,
R" wie in Anspruch 1 definiert ist und weiter bevorzugt für ein Wasserstoffatom steht,
n wie in Anspruch 1 definiert ist und weiter bevorzugt für 1 steht,
n' wie in Anspruch 1 definiert ist,
R' für ein Wasserstoffatom, ein Halogenatom oder eine (C₁-C₃)Alkylgruppe steht,
(2) 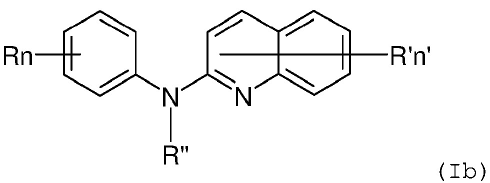 wobei:
R unabhängig für ein Wasserstoffatom, ein Halogenatom oder eine Gruppe, die aus einer (C₁-C₃) Alkylgruppe, einer (C₁-C₃) Fluoralkoxygruppe und einer Phenoxygruppe ausgewählt ist, steht,
R₁ für ein Wasserstoffatom oder eine (C₁-C₃) Alkylgruppe steht,
R" wie in Anspruch 1 definiert ist und weiter bevorzugt für ein Wasserstoffatom steht,
n wie in Anspruch 1 definiert ist und weiter bevorzugt für 1 steht,
n' wie in Anspruch 1 definiert ist,
R' für ein Wasserstoffatom steht,
(3) 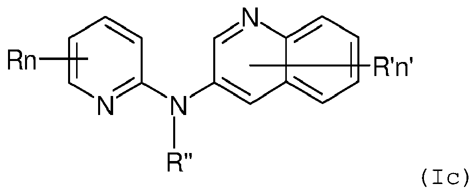 wobei:
R unabhängig für ein Wasserstoffatom oder eine Gruppe, die aus einer (C₁-C₃) Alkylgruppe, einer -NO₂-Gruppe und einer (C₁-C₃)Alkoxygruppe ausgewählt ist, steht,
R" wie in Anspruch 1 definiert ist und weiter bevorzugt für ein Wasserstoffatom steht,
n wie in Anspruch 1 definiert ist und weiter bevorzugt für 1 steht,
n' wie in Anspruch 1 definiert ist,
R' für ein Wasserstoffatom steht,
(4) 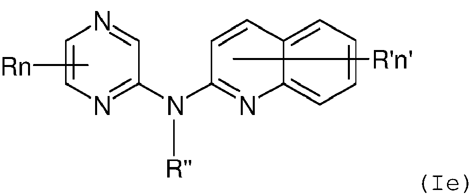 wobei:
R für ein Wasserstoffatom steht,
R" wie in Anspruch 1 definiert ist und vorteilhafterweise für ein Wasserstoffatom steht,
n wie in Anspruch 1 definiert ist und vorteilhafterweise für 1 steht,
n' wie in Anspruch 1 definiert ist,
R' für ein Wasserstoffatom oder ein Halogenatom steht,
(5) 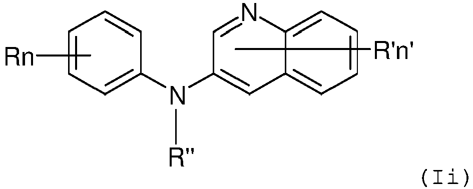 wobei:
R unabhängig für ein Wasserstoffatom oder eine (C₁-C₃)Alkoxygruppe steht,
R" wie oben definiert ist und vorteilhafterweise für ein Wasserstoffatom steht,
n wie oben definiert ist und vorteilhafterweise für 1 steht,
n' wie oben definiert ist und vorteilhafterweise für 1 steht,
R' für ein Wasserstoffatom steht,
(6) 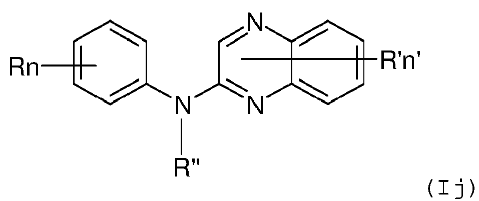 wobei:
R unabhängig für ein Wasserstoffatom oder eine Gruppe, die aus einer (C₁-C₃)Fluoralkoxygruppe und einer (C₁-C₃)Alkylgruppe ausgewählt ist, steht,
R" wie in Anspruch 1 definiert ist und weiter bevorzugt für ein Wasserstoffatom steht,
n wie in Anspruch 1 definiert ist und weiter bevorzugt für 2 steht,
n' wie in Anspruch 1 definiert ist,
R' für ein Wasserstoffatom steht,
(7) 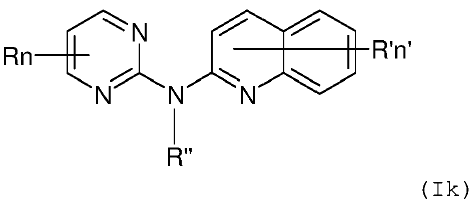 wobei:
R für ein Wasserstoffatom steht,
R" wie in Anspruch 1 definiert ist und weiter bevorzugt für ein Wasserstoffatom steht,
n wie in Anspruch 1 definiert ist und weiter bevorzugt für 1 steht,
n' wie in Anspruch 1 definiert ist,
R' für ein Wasserstoffatom steht,
(8) 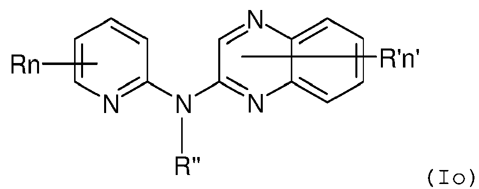 wobei:
R unabhängig für ein Wasserstoffatom oder eine Gruppe, die aus einer (C₁-C₃)Alkylgruppe und einer -CN-Gruppe ausgewählt ist, steht,
R" wie in Anspruch 1 definiert ist und weiter bevorzugt für ein Wasserstoffatom steht,
n wie in Anspruch 1 definiert ist und weiter bevorzugt für 1 steht,
n' wie in Anspruch 1 definiert ist und weiter bevorzugt für 1 steht,
R' für ein Wasserstoffatom steht,
(9)
oder einem pharmazeutisch unbedenklichen Salz davon.

5. Verbindung der Formel (I) nach einem der Ansprüche 1 bis 3, die alternativ ausgewählt ist aus
(1) einer Verbindung der Formel (Ig) gemäß Anspruch 3,
wobei:
R unabhängig für ein Wasserstoffatom, ein Halogenatom oder eine Gruppe, die aus einer (Ci-C₃)Alkylgruppe, einer -CN-Gruppe, einer Hydroxylgruppe, einer -COOR₁-Gruppe, einer (C₁-C₃)Fluoralkylgruppe, einer (C₁-C₃) Fluoralkoxygruppe, einer -NO₂-Gruppe, einer -NR₁R₂-Gruppe und einer (C₁-C₃)Alkoxygruppe ausgewählt ist, steht,
n für 1 oder 2 steht,
n' für 1 oder 2 steht,
R' für ein Wasserstoffatom oder eine Gruppe, die aus einer (C₁-C₃) Alkylgruppe, einem Halogenatom, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
R" für ein Wasserstoffatom oder eine (C₁-C₄)Alkylgruppe steht,
R₁ und R₂ unabhängig für ein Wasserstoffatom oder eine (C₁-C₃)Alkylgruppe stehen,
mit der Maßgabe, dass R und R' nicht gleichzeitig für ein Wasserstoffatom stehen,
und dann, wenn n und n' für 1 stehen und R für ein Wasserstoffatom steht, R' nicht für eine -COOH-Gruppe steht,
oder einem pharmazeutisch unbedenklichen Salz davon,
(2) einer Verbindung der Formel (Ic) gemäß Anspruch 3,
wobei:
R unabhängig für ein Wasserstoffatom, ein Halogenatom oder eine Gruppe, die aus einer (Ci-C₃)Alkylgruppe, einer -CN-Gruppe, einer Hydroxylgruppe, einer -COOR₁-Gruppe, einer (C₁-C₃)Fluoralkylgruppe, einer -NO₂-Gruppe, einer -NR₁R₂-Gruppe und einer (Ci-C₃)Alkoxygruppe ausgewählt ist, steht,
R₁ und R₂ unabhängig für ein Wasserstoffatom oder eine (C₁-C₃)Alkylgruppe stehen,
n für 1 oder 2 und vorteilhafterweise 1 steht,
n' für 1 oder 2 steht,
R" wie in Formel (Ic) in Anspruch 3 definiert ist,
R' für ein Wasserstoffatom oder eine Gruppe, die aus einer (C₁-C₃) Alkylgruppe, einer -NO₂-Gruppe, einer -NR₁R₂-Gruppe und einer (C₁-C₃)Alkoxygruppe ausgewählt ist, steht,
mit der Maßgabe, dass
R und R' nicht gleichzeitig für ein Wasserstoffatom stehen,
R nicht für ein Bromatom steht, wenn R' für ein Wasserstoffatom steht,
oder einem pharmazeutisch unbedenklichen Salz davon,
(3) einer Verbindung der Formel (Id) gemäß Anspruch 3,
wobei:
R unabhängig für ein Wasserstoffatom, ein Halogenatom oder eine Gruppe, die aus einer (Ci-C₃)Alkylgruppe, einer -CN-Gruppe, einer Hydroxylgruppe, einer -COOR₁-Gruppe, einer (C₁-C₃)Fluoralkylgruppe, einer (C₁-C₃)Fluoralkoxygruppe, einer -NO₂-Gruppe und einer - NR₁R₂-Gruppe ausgewählt ist, steht,
R₁ und R₂ unabhängig für ein Wasserstoffatom oder eine (C₁-C₃)Alkylgruppe stehen,
n für 1 oder 2 und vorteilhafterweise 1 steht,
n' für 1 oder 2 steht,
R" wie in Formel (I) in Anspruch 1 definiert ist und vorteilhafterweise für ein Wasserstoffatom steht,
R' für ein Wasserstoffatom oder eine Gruppe, die aus einer (C₁-C₃) Alkylgruppe, einer -NO₂-Gruppe, einer -NR₁R₂-Gruppe und einer (C₁-C₃)Alkoxygruppe ausgewählt ist, steht,
mit der Maßgabe, dass dann, wenn R' für ein Wasserstoffatom steht, R von einer -NO₂-Gruppe, einer - NH₂-Gruppe oder einer -COOH-Gruppe verschieden ist,
oder einem pharmazeutisch unbedenklichen Salz davon,
(4) einer Verbindung der Formel (Ii') 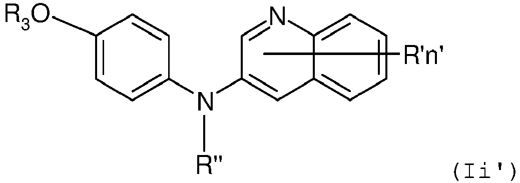 wobei:
R₃ für eine (C₁-C₃) Fluoralkylgruppe oder eine (C₁-C₃)Alkylgruppe steht,
R' für ein Wasserstoffatom, ein Halogenatom oder eine Gruppe, die aus einer (C₁-C₃)Alkylgruppe, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
R₁ und R₂ unabhängig für ein Wasserstoffatom oder eine (C₁-C₃)Alkylgruppe stehen,
R" wie in Anspruch 1 definiert ist und vorteilhafterweise für ein Wasserstoffatom steht,
n' wie in Anspruch 1 definiert ist und vorteilhafterweise für 1 steht,
mit der Maßgabe, dass
dann, wenn R' für ein Wasserstoffatom steht, R₃ nicht für eine Methylgruppe oder eine Trifluormethylgruppe steht,
oder einem pharmazeutisch unbedenklichen Salz davon.

6. Verbindung der Formel (I) nach einem der Ansprüche 1 bis 3, die alternativ ausgewählt ist aus
(1) einer Verbindung der Formel (Ik) gemäß Anspruch 3,
wobei:
R unabhängig für ein Wasserstoffatom, ein Halogenatom oder eine Gruppe, die aus einer (Ci-C₃)Alkylgruppe, einer -CN-Gruppe, einer Hydroxylgruppe, einer -COOR₁-Gruppe, einer (C₁-C₃)Fluoralkylgruppe, einer (C₁-C₃) Fluoralkoxygruppe, einer -NO₂-Gruppe, einer -NR₁R₂-Gruppe und einer (C₁-C₃)Alkoxygruppe ausgewählt ist, steht,
R₁ und R₂ unabhängig für ein Wasserstoffatom oder eine (C₁-C₃)Alkylgruppe stehen,
n für 1 oder 2 steht und vorteilhafterweise für 1 steht,
n' für 1 oder 2 steht,
R" wie in Formel (Ik) in Anspruch 3 definiert ist,
R' für ein Wasserstoffatom, ein Halogenatom oder eine Gruppe, die aus einer (C₁-C₃)Alkylgruppe, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
oder einem pharmazeutisch unbedenklichen Salz davon,
(2) einer Verbindung der Formel (Io) gemäß Anspruch 3,
wobei:
R unabhängig für ein Wasserstoffatom, ein Halogenatom oder eine Gruppe, die aus einer (Ci-C₃)Alkylgruppe, einer -CN-Gruppe, einer Hydroxylgruppe, einer -COOR₁-Gruppe, einer (C₁-C₃)Fluoralkylgruppe, einer (C₁-C₃) Fluoralkoxygruppe, einer -NO₂-Gruppe, einer -NR₁R₂-Gruppe und einer (C₁-C₃)Alkoxygruppe ausgewählt ist, steht,
R₁ und R₂ unabhängig für ein Wasserstoffatom oder eine (C₁-C₃)Alkylgruppe stehen,
n für 1, 2 oder 3 steht,
n' für 1 oder 2 steht,
R' für ein Wasserstoffatom, ein Halogenatom oder eine Gruppe, die aus einer (C₁-C₃)Alkylgruppe, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
R" für ein Wasserstoffatom oder eine (C₁-C₄)Alkylgruppe steht,
mit der Maßgabe, dass
dann, wenn R für ein Wasserstoffatom steht und n' für 1 steht, R' nicht für eine Hydroxylgruppe steht,
oder einem pharmazeutisch unbedenklichen Salz davon,
(3) einer Verbindung der Formel (Ij') 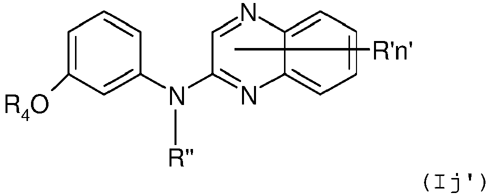 wobei:
R₄ für eine (C₁-C₃) Fluoralkylgruppe oder eine (C₁-C₃)Alkylgruppe steht,
R' für ein Wasserstoffatom, ein Halogenatom oder eine Gruppe, die aus einer (C₁-C₃)Alkylgruppe, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
R₁ und R₂ unabhängig für ein Wasserstoffatom oder eine (C₁-C₃)Alkylgruppe stehen,
R" wie in Anspruch 1 definiert ist und vorteilhafterweise für ein Wasserstoffatom steht,
n' wie in Anspruch 1 definiert ist und vorteilhafterweise für 1 steht,
mit der Maßgabe, dass dann
wenn R' für ein Wasserstoffatom steht, R₄ nicht für eine Methylgruppe steht,
oder einem pharmazeutisch unbedenklichen Salz davon,
(4) einer Verbindung der Formel (Ij") 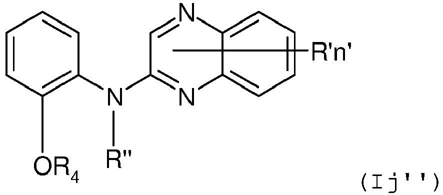 wobei:
R₄ für eine (C₁-C₃) Fluoralkylgruppe oder eine (C₁-C₃)Alkylgruppe steht,
R' für ein Wasserstoffatom, ein Halogenatom oder eine Gruppe, die aus einer (C₁-C₃)Alkylgruppe, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
R₁ und R₂ unabhängig für ein Wasserstoffatom oder eine (C₁-C₃)Alkylgruppe stehen,
R" wie in Anspruch 1 definiert ist und vorteilhafterweise für ein Wasserstoffatom steht, n' wie in Anspruch 1 definiert ist und vorteilhafterweise für 1 steht,
oder einem pharmazeutisch unbedenklichen Salz davon,
(5) einer Verbindung der Formel (Ij‴) 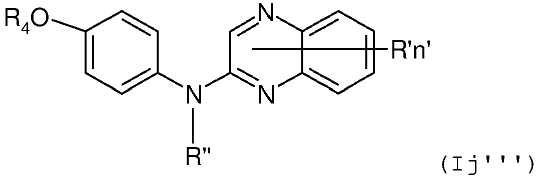 wobei:
R₄ für eine (C₁-C₃) Fluoralkylgruppe oder eine (C₁-C₃)Alkylgruppe steht,
R' für ein Wasserstoffatom, ein Halogenatom oder eine Gruppe, die aus einer (C₁-C₃)Alkylgruppe, einer Hydroxylgruppe, einer -NO₂-Gruppe, einer -NR₁R₂-Gruppe, einer (C₁-C₃)Alkoxygruppe und einer -CN-Gruppe ausgewählt ist, steht,
R₁ und R₂ unabhängig für ein Wasserstoffatom oder eine (C₁-C₃)Alkylgruppe stehen,
R" wie in Anspruch 1 definiert ist und vorteilhafterweise für ein Wasserstoffatom steht,
n' wie in Anspruch 1 definiert ist und vorteilhafterweise für 1 steht,
mit der Maßgabe, dass
dann, wenn R' für ein Chloratom oder ein Wasserstoffatom steht, R₄ nicht für eine Ethylgruppe oder eine Methylgruppe steht,
dann, wenn R' für eine Methylgruppe oder eine tert-Butylgruppe steht, R₄ nicht für eine Methylgruppe steht,
oder einem pharmazeutisch unbedenklichen Salz davon.

7. Verbindung der Formel (I) nach einem der Ansprüche 1 bis 3, die alternativ ausgewählt ist aus
(1) einer Verbindung der Formel (Ip) gemäß Anspruch 3,
wobei:
R unabhängig für ein Wasserstoffatom, ein Halogenatom oder eine Gruppe, die aus einer (Ci-C₃)Alkylgruppe, einer -CN-Gruppe, einer Hydroxylgruppe, einer -COOR₁-Gruppe, einer (C₁-C₃)Fluoralkylgruppe, einer (C₁-C₃) Fluoralkoxygruppe, einer -NO₂-Gruppe, einer -NR₁R₂-Gruppe und einer (C₁-C₃)Alkoxygruppe ausgewählt ist, steht,
R₁ und R₂ unabhängig für ein Wasserstoffatom oder eine (C₁-C₃)Alkylgruppe stehen,
n für 1 oder 2 und vorteilhafterweise 1 steht,
n' für 1 oder 2 steht,
R' für ein Wasserstoffatom, ein Halogenatom oder eine Gruppe, die aus einer (C₁-C₃)Alkylgruppe, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
R" für ein Wasserstoffatom oder eine (C₁-C₄)Alkylgruppe steht,
mit der Maßgabe, dass
wobei R und R' nicht gleichzeitig für ein Wasserstoffatom stehen,
dann, wenn n und n' für 2 stehen, R und R' nicht gleichzeitig für eine Methylgruppe stehen,
oder einem pharmazeutisch unbedenklichen Salz davon,
(2) einer Verbindung der Formel (Ir) gemäß Anspruch 3,
wobei:
R unabhängig für ein Wasserstoffatom, ein Halogenatom oder eine Gruppe, die aus einer (Ci-C₃)Alkylgruppe, einer -CN-Gruppe, einer Hydroxylgruppe, einer -COOR₁-Gruppe, einer (C₁-C₃)Fluoralkylgruppe, einer (C₁-C₃) Fluoralkoxygruppe, einer -NO₂-Gruppe, einer -NR₁R₂-Gruppe und einer (C₁-C₃)Alkoxygruppe ausgewählt ist, steht,
R₁ und R₂ unabhängig für ein Wasserstoffatom oder eine (C₁-C₃)Alkylgruppe stehen,
n für 1 oder 2 und vorteilhafterweise 1 steht,
n' für 1 oder 2 steht,
R' für ein Wasserstoffatom, ein Halogenatom oder eine Gruppe, die aus einer (C₁-C₃)Alkylgruppe, einer Hydroxylgruppe, einer -COOR₁-Gruppe, einer -NO₂-Gruppe, einer -NR₁R₂-Gruppe, einer (C₁-C₃)Alkoxygruppe und einer -CN-Gruppe ausgewählt ist, steht,
R" für ein Wasserstoffatom oder eine (C₁-C₄)Alkylgruppe steht,
oder einem pharmazeutisch unbedenklichen Salz davon.

8. Verbindung zur Verwendung nach einem der Ansprüche 1 bis 4, wobei die Verbindung ausgewählt ist aus:
- (1) (8-Chlorchinolin-2-yl)-pyridin-2-ylamin
- (2) 2-(Chinolin-2-ylamino)isonicotinsäure
- (3) (4-Methylpyridin-2-yl)-chinolin-2-ylamin
- (4) Pyridin-2-ylchinolin-2-ylamin
- (5) 2-(8-Chlorchinolin-2-ylamino)isonicotinsäure
- (6) (8-Chlorchinolin-2-yl)-(4-methylpyridin-2-yl)amin
- (7) 6-(Chinolin-2-ylamino)-nicotinonitril
- (8) Chinolin-2-yl-(4-trifluormethoxyphenyl)amin
- (9) Pyridin-2-ylchinolin-3-ylamin
- (10) (3-Methoxypyridin-2-yl)-chinolin-3-ylamin
- (11) Chinolin-3-yl-(5-trifluormethylpyridin-2-yl)amin
- (12) (5-Nitropyridin-2-yl)-chinolin-3-ylamin
- (13) (5-Methylpyridin-2-yl)-chinolin-3-ylamin
- (14) 2-(Chinolin-3-ylamino)isonicotinsäure
- (15) Chinolin-6-yl-(5-trifluormethylpyridin-2-yl)amin
- (16) (6-Methylpyridin-2-yl)-chinolin-6-ylamin
- (17) N-(6-Methylpyridin-2-yl)chinolin-2-amin
- (18) 8-Chlor-N-(6-methylpyridin-2-yl)chinolin-2-amin
- (19) 4-Methyl-N-(pyridin-2-yl)chinolin-2-amin
- (20) 4-Methyl-N-(4-methylpyridin-2-yl)chinolin-2-amin
- (21) 3-Methyl-N-(4-methylpyridin-2-yl)chinolin-2-amin
- (22) 3-Methyl-N-(pyridin-2-yl)chinolin-2-amin
- (23) 6-((4-Methylchinolin-2-yl)amino)nicotino-nitril
- (24) 6-((3-methylchinolin-2-yl)amino)nicotino-nitril
- (25) 6-Chlor-N-(4-methylpyridin-2-yl)chinolin-2-amin
- (26) 6-Chlor-N-(6-methylpyridin-2-yl)chinolin-2-amin
- (27) 4-Methyl-N-(5-nitropyridin-2-yl)chinolin-2-amin
- (28) N-(3-Nitropyridin-2-yl)chinolin-2-amin
- (29) 8-Chlor-N-(3-nitropyridin-2-yl)chinolin-2-amin
- (30) 2-((4-Methylchinolin-2-yl)amino)nicotino-nitril
- (31) N-(3-Methylpyridin-2-yl)chinolin-2-amin
- (32) N-(5-Methylpyridin-2-yl)chinolin-2-amin
- (33) 2-(Chinolin-2-ylamino)isonicotinonitril
- (34) N-(5-(Trifluormethyl)pyridin-2-yl)chinolin-2-amin
- (35) 8-Chlor-N-(3-methylpyridin-2-yl)chinolin-2-amin
- (36) 8-Chlor-N-(5-methylpyridin-2-yl)chinolin-2-amin
- (37) 8-Chlor-N-(5-(trifluormethyl)pyridin-2-yl)chinolin-2-amin
- (38) N-(3-Methoxypyridin-2-yl)chinolin-2-amin
- (39) N-(5-Nitropyridin-2-yl)chinolin-2-amin
- (40) 6-((8-Chlorchinolin-2-yl)amino)nicotino-nitril
- (41) N-(5-Fluorpyridin-2-yl)chinolin-2-amin
- (42) N-(6-(Trifluormethyl)pyridin-2-yl)chinolin-2-amin
- (43) 8-Chlor-N-(5-fluorpyridin-2-yl)chinolin-2-amin
- (44) 2-((8-Chlorchinolin-2-yl)amino)nicotinsäure
- (45) 4-Methyl-N-(6-methylpyridin-2-yl)chinolin-2-amin
- (46) 3-Methyl-N-(6-methylpyridin-2-yl)chinolin-2-amin
- (47) 5-Cyano-2-(chinolin-2-ylamino)pyridin-1-iumchlorid
- (48) 2-((8-Chlorchinolin-2-yl)amino)-4-methylpyridin-1-iumchlorid
- (49) 8-Chlor-N-(4-ethylpyridin-2-yl)chinolin-2-amin
- (50) 8-Chlor-N-(6-ethylpyridin-2-yl)chinolin-2-amin
- (51) 8-Chlor-N-(4,6-dimethylpyridin-2-yl)chinolin-2-amin
- (52) 6-((8-Chlorchinolin-2-yl)amino)-2-methylnicotinonitril
- (53) 8-Chlor-N-(4-chlorpyridin-2-yl)chinolin-2-amin
- (54) 8-Methyl-N-(4-methylpyridin-2-yl)chinolin-2-amin
- (55) N-(5-Brom-4-methylpyridin-2-yl)-8-chlorchinolin-2-amin
- (56) 8-Chlor-N-(3-ethyl-6-methylpyridin-2-yl)chinolin-2-amin
- (57) 8-Fluor-N-(4-methylpyridin-2-yl)chinolin-2-amin
- (58) 8-Brom-N-(4-methylpyridin-2-yl)chinolin-2-amin
- (59) 6-(Chinolin-2-ylamino)nicotinsäuremethyl-ester
- (60) 6-[(8-Chlorchinolin-2-yl)amino]pyridin-3-carbonsäuremethylester
- (61) 6-[(3-Methylchinolin-2-yl)amino]pyridin-3-carbonsäuremethylester
- (62) 2-[(8-Chlorchinolin-2-yl)amino]pyridin-3-carbonsäuremethylester
- (63) 8-Methoxy-N-(4-methylpyridin-2-yl)chinolin-2-amin
- (64) N-(4-Methylpyridin-2-yl)-5-nitrochinolin-2-amin
- (65) 2-N-(4-Methylpyridin-2-yl)chinolin-2,8-diamin
- (66) N-(4-Methylpyridin-2-yl)-5-aminochinolin-2-amin
- (67) 6-[(4-Methylchinolin-2-yl)amino]pyridin-3-carbonsäuremethylester
- (68) 8-Chlor-N-[4-(trifluormethyl)pyridin-2-yl]chinolin-2-amin
- (69) 2-[(8-Chlorchinolin-2-yl)amino]pyridin-3-ol
- (70) 8-Chlor-N-[6-(trifluormethyl)pyridin-2-yl]chinolin-2-amin
- (71) 6-Chlor-N-(5-fluorpyridin-2-yl)chinolin-2-amin
- (72) N-(6-Ethylpyridin-2-yl)-3-methylchinolin-2-amin
- (73) N-(5-Fluorpyridin-2-yl)-3-methylchinolin-2-amin
- (74) 3-Methyl-N-[5-(trifluormethyl)pyridin-2-yl]chinolin-2-amin
- (75) 4-N-(8-Chlorchinolin-2-yl)-1-N,1-N-dimethylbenzol-1,4-diamin
- (76) N-(4-Methoxyphenyl)chinolin-2-amin
- (77) 8-Chlor-N-(4-methoxyphenyl)chinolin-2-amin
- (78) 4-Methyl-N-[4-(trifluormethoxy)phenyl]-chinolin-2-amin
- (79) N-(4-Methoxyphenyl)-3-methylchinolin-2-amin
- (80) 3-Methyl-N-[4-(trifluormethoxy)phenyl]-chinolin-2-amin
- (81) 1-N,1-N-Dimethyl-4-N-(3-methylchinolin-2-yl)benzol-1,4-diamin
- (82) N-[2-Methyl-4-(trifluormethoxy)phenyl]-chinolin-2-amin
- (83) N-[3-(Trifluormethoxy)phenyl]chinolin-2-amin
- (84) N-[2-(Trifluormethoxy)phenyl]chinolin-2-amin
- (85) N-(4-Nitrophenyl)chinolin-2-amin
- (86) N-(3-Fluorphenyl)chinolin-2-amin
- (87) 8-Chlor-N-[3-(trifluormethoxy)phenyl]-chinolin-2-amin
- (88) 8-Chlor-N-(3-fluorphenyl)chinolin-2-amin
- (89) 2-{[4-(Trifluormethoxy)phenyl]amino}-chinolin-1-iumchlorid
- (90) 8-Chlor-N-[4-(trifluormethoxy)phenyl]-chinolin-2-amin
- (91) 3-Methyl-N-[2-methyl-4-(trifluormethoxy)-phenyl]chinolin-2-amin
- (92) 3-Methyl-N-[3-(trifluormethoxy)phenyl]-chinolin-2-amin
- (93) 3-Methyl-N-[2-(trifluormethoxy)phenyl]-chinolin-2-amin
- (94) 8-Chlor-N-[2-methyl-4-(trifluormethoxy)-phenyl]chinolin-2-amin
- (95) 3-Methyl-2-{[4-(trifluormethoxy)phenyl]-amino}chinolin-1-iumchlorid
- (96) 6-Chlor-N-(4-(trifluormethoxy)phenyl)-chinolin-2-amin
- (97) 4-Methyl-2-{[4-(trifluormethoxy)phenyl]-amino}chinolin-1-iumchlorid
- (98) 8-Brom-N-[4-(trifluormethoxy)phenyl]-chinolin-2-amin
- (99) 8-Fluor-N-[4-(trifluormethoxy)phenyl]-chinolin-2-amin
- (100) 8-Methyl-N-[4-(trifluormethoxy)phenyl]-chinolin-2-amin
- (101) N-(4-Butoxyphenyl)-8-chlorchinolin-2-amin
- (102) N-(4-Phenoxyphenyl)chinolin-2-amin
- (103) 8-Methoxy-N-[4-(trifluormethoxy)phenyl]-chinolin-2-amin
- (104) 8-Chlor-N-[3-chlor-4-(trifluormethoxy)-phenyl]chinolin-2-amin
- (105) N-(6-Methylpyridin-2-yl)chinolin-3-amin
- (106) N-(3-Nitropyridin-2-yl)chinolin-3-amin
- (107) N-(5-Methylpyridin-2-yl)chinolin-6-amin
- (108) N-(3-Methoxypyridin-2-yl)chinolin-6-amin
- (109) 6-Chlor-N-(pyrazin-2-yl)chinolin-2-amin
- (110) 8-Brom-N-(pyrazin-2-yl)chinolin-2-amin
- (111) 8-Methyl-N-(pyrazin-2-yl)chinolin-2-amin
- (112) 8-Chlor-N-(pyrazin-2-yl)chinolin-2-amin
- (113) N-(Pyrazin-2-yl)chinolin-2-amin
- (114) 4-Methyl-N-(pyrazin-2-yl)chinolin-2-amin
- (115) 3-Methyl-N-(pyrazin-2-yl)chinolin-2-amin
- (116) 8-Fluor-N-(pyrazin-2-yl)chinolin-2-amin
- (117) 8-Methoxy-N-(pyrazin-2-yl)chinolin-2-amin
- (118) N-(Pyridin-3-yl)chinolin-3-amin
- (119) 8-Chlor-N-(pyridin-4-yl)chinolin-2-amin
- (120) N-(Pyridin-4-yl)chinolin-2-amin
- (121) N-(Pyridin-4-yl)chinolin-3-amin
- (122) N-[4-(Trifluormethoxy)phenyl]chinolin-3-amin
- (123) N-(4-Methoxyphenyl)chinolin-3-amin
- (124) N-[4-(Trifluormethoxy)phenyl]chinoxalin-2-amin
- (125) N-[2-Methyl-4-(trifluormethoxy)phenyl]-chinoxalin-2-amin
- (126) N-[3-(Trifluormethoxy)phenyl]chinoxalin-2-amin
- (127) N-[2-(Trifluormethoxy)phenyl]chinoxalin-2-amin
- (128) N-(Pyrimidin-2-yl)chinolin-2-amin
- (129) 8-Chlor-N-(pyrimidin-2-yl)chinolin-2-amin
- (130) 4-Methyl-N-(pyrimidin-2-yl)chinolin-2-amin
- (131) N-(Pyrazin-2-yl)chinolin-6-amin
- (132) N-(Pyrazin-2-yl)chinolin-3-amin
- (133) 6-Methyl-N-(naphthalin-2-yl)pyridin-2-amin
- (134) N-(Naphthalin-2-yl)pyridin-2-amin
- (135) N-(Pyridin-2-yl)chinoxalin-2-amin
- (136) N-(4-Methylpyridin-2-yl)chinoxalin-2-amin
- (137) 6-(Chinoxalin-2-ylamino)pyridin-3-carbonitril
- (138) N-(6-Methylpyridin-2-yl)chinoxalin-2-amin
- (139) N-(4-Methylpyridin-2-yl)-3-(trifluormethyl)chinoxalin-2-amin
- (140) N-(3,5-Dichlor-4-methylpyridin-2-yl)chinoxalin-2-amin
- (141) N-(4-Methyl-3-nitropyridin-2-yl)chinoxalin-2-amin
- (142) N-(Pyrimidin-2-yl)chinoxalin-2-amin
- (143) 4-N,4-N-Dimethyl-7-N-[4-(trifluormethoxy)-phenyl]chinolin-4,7-diamin
- (144) 4-(Morpholin-4-yl)-N-[4-(trifluormethoxy)-phenyl]chinolin-7-amin
- (145) 4-Methoxy-N-(pyridin-2-yl)chinolin-7-amin
- (146) 4-Methoxy-N-(4-methylpyridin-2-yl)chinolin-7-amin
- (147) 4-N,4-N-Dimethyl-7-N-(4-methylpyridin-2-yl)chinolin-4,7-diamin
- (150) N-(4-Methylpyridin-2-yl)-8-nitrochinolin-2-amin
- (151) 6-Chlor-N-(6-ethylpyridin-2-yl)chinolin-2-amin
- (152) 6-Chlor-N-(5-methylpyridin-2-yl)chinolin-2-amin
- (153) 6-Chlor-N-[5-(trifluormethyl)pyridin-2-yl]chinolin-2-amin
- (154) N2-(8-Chlorchinolin-2-yl)-4-methylpyridin-2,3-diamin
- (155) N-(4-Butoxyphenyl)-3-methylchinolin-2-amin
- (156) 4-N-(6-Chlorchinolin-2-yl)-1-N,1-N-dimethylbenzol-1,4-diamin
- (157) 8-Chlor-N-(3-chlor-4-methoxyphenyl)-chinolin-2-amin
- (158) N1-(8-Chlorchinolin-2-yl)-4-(trifluormethoxy)benzol-1,2-diamin
- (159) N-(3-Aminopyridin-2-yl)chinolin-3-amin
- (160) 6-Chlor-N-(4-methylpyridin-2-yl)chinoxalin-2-amin
- (161) N-(4-Ethylpyridin-2-yl)chinoxalin-2-amin
- (162) N-(5-Brom-4-methylpyridin-2-yl)chinoxalin-2-amin
- (163) N-(4,6-Dimethylpyridin-2-yl)chinoxalin-2-amin
- (164) [2-(Chinoxalin-2-ylamino)pyridin-4-yl]methanol
- (165) N-(4-Methyl-5-nitropyridin-2-yl)chinoxalin-2-amin
- (166) N-(4-Methoxyphenyl)-4-(4-methylpiperazin-1-yl)chinolin-7-amin
- (167) 4-Methoxy-N-[4-(trifluormethoxy)phenyl]-chinolin-7-amin
- (168) N-(4-Methylpyridin-2-yl)-4-(morpholin-4-yl)chinolin-7-amin
- und pharmazeutisch unbedenklichen Salzen davon.

9. Verbindung nach einem der Ansprüche 5, 6, und 7, wobei die Verbindung aus den folgenden Verbindungen ausgewählt ist:
- (10) (3-Methoxypyridin-2-yl)-chinolin-3-ylamin
- (11) Chinolin-3-yl-(5-trifluormethylpyridin-2-yl)amin
- (12) (5-Nitropyridin-2-yl)-chinolin-3-ylamin
- (13) (5-Methylpyridin-2-yl)-chinolin-3-ylamin
- (14) 2-(Chinolin-3-ylamino)isonicotinsäure
- (15) Chinolin-6-yl-(5-trifluormethylpyridin-2-yl)amin
- (16) (6-Methylpyridin-2-yl)-chinolin-6-ylamin
- (105) N-(6-Methylpyridin-2-yl)chinolin-3-amin
- (106) N-(3-Nitropyridin-2-yl)chinolin-3-amin
- (107) N-(5-Methylpyridin-2-yl)chinolin-6-amin
- (108) N-(3-Methoxypyridin-2-yl)chinolin-6-amin
- (119) 8-Chlor-N-(pyridin-4-yl)chinolin-2-amin
- (124) N-[4-(Trifluormethoxy)phenyl]chinoxalin-2-amin
- (125) N-[2-Methyl-4-(trifluormethoxy)phenyl]-chinoxalin-2-amin
- (126) N-[3-(Trifluormethoxy)phenyl]chinoxalin-2-amin
- (127) N-[2-(Trifluormethoxy)phenyl]chinoxalin-2-amin
- (128) N-(Pyrimidin-2-yl)chinolin-2-amin
- (129) 8-Chlor-N-(pyrimidin-2-yl)chinolin-2-amin
- (130) 4-Methyl-N-(pyrimidin-2-yl)chinolin-2-amin
- (135) N-(Pyridin-2-yl)chinoxalin-2-amin
- (136) N-(4-Methylpyridin-2-yl)chinoxalin-2-amin
- (137) 6-(Chinoxalin-2-ylamino)pyridin-3-carbonitril
- (138) N-(6-Methylpyridin-2-yl)chinoxalin-2-amin
- (139) N-(4-Methylpyridin-2-yl)-3-(trifluormethyl)chinoxalin-2-amin
- (140) N-(3,5-Dichlor-4-methylpyridin-2-yl)chinoxalin-2-amin
- (141) N-(4-Methyl-3-nitropyridin-2-yl)chinoxalin-2-amin
- (145) 4-Methoxy-N-(pyridin-2-yl)chinolin-7-amin
- (146) 4-Methoxy-N-(4-methylpyridin-2-yl)chinolin-7-amin
- (147) 4-N,4-N-Dimethyl-7-N-(4-methylpyridin-2-yl)chinolin-4,7-diamin
- (159) N-(3-Aminopyridin-2-yl)chinolin-3-amin
- (160) 6-Chlor-N-(4-methylpyridin-2-yl)chinoxalin-2-amin
- (161) N-(4-Ethylpyridin-2-yl)chinoxalin-2-amin
- (162) N-(5-Brom-4-methylpyridin-2-yl)chinoxalin-2-amin
- (163) N-(4,6-Dimethylpyridin-2-yl)chinoxalin-2-amin
- (164) [2-(Chinoxalin-2-ylamino)pyridin-4-yl]methanol
- (165) N-(4-Methyl-5-nitropyridin-2-yl)chinoxalin-2-amin und
- (168) N-(4-Methylpyridin-2-yl)-4-(morpholin-4-yl)chinolin-7-amin
sowie pharmazeutisch unbedenklichen Salzen davon, wie Hydrochlorid, Hydrobromid, Tartrat, Fumarat, Citrat, Trifluoracetat, Ascorbat, Triflat, Mesylat, Tosylat, Formiat, Acetat und Malat.

10. Pharmazeutische Zusammensetzung, umfassend mindestens eine Verbindung gemäß einem der Ansprüche 5, 6, 7 und 9.

## Revendications

1. Composé de formule (I) : 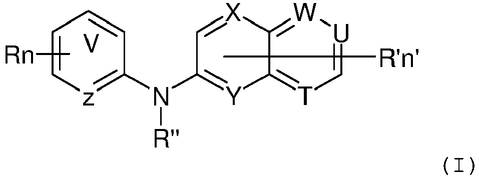 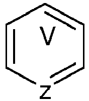
signifiant un cycle aromatique, V étant C ou N et lorsque V est N, V est en ortho, méta ou para de Z, c'est-à-dire formant respectivement un groupe pyridazine, un groupe pyrimidine ou un groupe pyrazine,
R représentant indépendamment un atome d'hydrogène, un atome d'halogène ou un groupe choisi parmi un groupe - CN, un groupe hydroxyle, un groupe -COOR₁, un groupe (Ci-C₃)fluoroalkyle, un groupe (C₁-C₃)fluoroalcoxy, un groupe -NO₂, un groupe -NR₁R₂, un groupe (C₁-C₄) alcoxy, un groupe phénoxy et un groupe (C₁-C₃) alkyle, ledit alkyle étant éventuellement monosubstitué par un groupe hydroxyle,
R₁ et R₂ étant indépendamment un atome d'hydrogène ou un groupe (C₁-C₃) alkyle,
n étant 1, 2 ou 3,
n' étant 1 ou 2,
R' étant un atome d'hydrogène, un atome d'halogène ou un groupe choisi parmi un groupe (C₁-C₃)alkyle, un groupe hydroxyle, un groupe -COOR₁, un groupe -NO₂, un groupe - NR₁R₂, un groupe morpholinyle ou un groupe morpholino, un groupe N-méthylpipérazinyle, un groupe (Ci-C₃)fluoroalkyle, un groupe (C₁-C₄)alcoxy et un groupe - CN,
R" étant un atome d'hydrogène ou un groupe (C₁-C₄) alkyle,
- Z étant N, V étant C, Y étant N, X étant C, T étant C, U étant C et W étant C,
- Z étant C, V étant C, Y étant N, X étant C, T étant C, U étant C et W étant C,
- Z étant N, V étant C, Y étant C, X étant N, T étant C, U étant C et W étant C,
- Z étant N, V étant C, Y étant C, X étant C, T étant C, U étant C et W étant N,
- Z étant N, V étant N et étant en para de Z, Y étant N, X étant C, T étant C, U étant C et W étant C
- Z étant C, V étant N et étant en para de Z, Y étant C, X étant N, T étant C, U étant C et W étant C,
- Z étant C, V étant N et étant en méta de Z et étant en para de la liaison liée à NR", Y étant N, X étant C, T étant C, U étant C et W étant C,
- Z étant C, V étant N et étant en méta de Z et étant en para de la liaison liée à NR", Y étant C, X étant N, T étant C, U étant C et W étant C,
- Z étant C, V étant C, Y étant C, X étant N, T étant C, U étant C et W étant C,
- Z étant C, V étant C, Y étant N, X étant N, T étant C, U étant C et W étant C,
- Z étant N, V étant N et étant en méta de Z et en ortho de la liaison liée à NR", Y étant N, X étant C, T étant C, U étant C et W étant C,
- Z étant N, V étant N et étant en para de Z, Y étant C, X étant C, T étant C, U étant C et W étant N,
- Z étant N, V étant N et étant en para de Z, Y étant C, X étant N, T étant C, U étant C et W étant C,
- Z étant N, V étant C, Y étant N, X étant N, T étant C, U étant C et W étant C,
- Z étant N, V étant N et étant en méta de Z et étant en ortho de la liaison liée à NR", Y étant N, X étant N, T étant C, U étant C et W étant C,
- Z étant C, V étant C, Y étant C, X étant C, T étant N, U étant C et W étant C, ou
- Z étant N, V étant C, Y étant C, X étant C, T étant N, U étant C et W étant C,
ou l'un quelconque de son sel pharmaceutiquement acceptable,
pour une utilisation en tant qu'agent pour la prévention, l'inhibition ou le traitement d'affections pathologiques liées au vieillissement prématuré.

2. Composé de formule (I) selon la revendication 1, en variante :
- Z étant N, V étant C, Y étant N, X étant C, T étant C, U étant C et W étant C,
- Z étant C, V étant C, Y étant N, X étant C, T étant C, U étant C et W étant C,
- Z étant N, V étant C, Y étant C, X étant N, T étant C, U étant C et W étant C,
- Z étant N, V étant C, Y étant C, X étant C, T étant C, U étant C et W étant N,
- Z étant N, V étant N et étant en para de Z, Y étant N, X étant C, T étant C, U étant C et W étant C,
- Z étant C, V étant C, Y étant C, X étant N, T étant C, U étant C et W étant C,
- Z étant C, V étant C, Y étant N, X étant N, T étant C, U étant C et W étant C,
- Z étant N, V étant N et étant en méta de Z et étant en ortho de la liaison liée à NR", Y étant N, X étant C, T étant C, U étant C et W étant C,
- Z étant N, V étant C, Y étant N, X étant N, T étant C, U étant C et W étant C,
- Z étant N, V étant N et étant en méta de Z et étant en ortho de la liaison liée à NR", Y étant N, X étant N, T étant C, U étant C et W étant C, ou
- Z étant N, V étant C, Y étant C, X étant C, T étant N, U étant C et W étant C,
pour une utilisation en tant qu'agent pour la prévention, l'inhibition ou le traitement d'affections pathologiques liées au vieillissement prématuré.

3. Composé de formule (I) selon l'une quelconque des revendications précédentes, pour une utilisation en tant qu'agent pour la prévention, l'inhibition ou le traitement d'affections pathologiques liées au vieillissement prématuré, qui est en variante choisi parmi :(1) 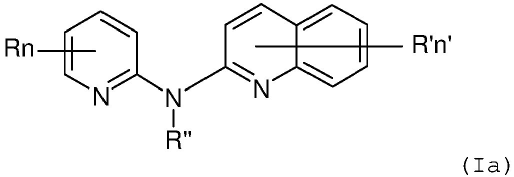
R représentant indépendamment un atome d'hydrogène, un atome d'halogène ou un groupe choisi parmi un groupe (Ci-C₃)alkyle, un groupe -CN, un groupe hydroxyle, un groupe -COOR₁, un groupe (C₁-C₃)fluoroalkyle, un groupe -NO₂, un groupe -NR₁R₂ et un groupe (C₁-C₃)alcoxy,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, un atome d'halogène ou un groupe choisi parmi un groupe (C₁-C₃)alkyle, un groupe - NO₂, un groupe (C₁-C₃)alcoxy et un groupe -NR₁R₂,
R₁ et R₂ étant un atome d'hydrogène ou un groupe (Ci-C₃) alkyle, 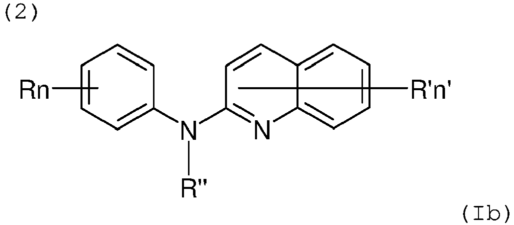
R représentant indépendamment un atome d'hydrogène, un atome d'halogène ou un groupe choisi parmi un groupe (Ci-C₃) alkyle, un groupe -NR₁R₂, un groupe (Ci-C₃)fluoroalcoxy, un groupe -NO₂, un groupe phénoxy et un groupe (C₁-C₄) alcoxy,
R₁ et R₂ étant indépendamment un atome d'hydrogène ou un groupe (C₁-C₃) alkyle,
R" étant tel que défini dans la revendication 1 et étant avantageusement un atome d'hydrogène,
n étant tel que défini dans la revendication 1 et étant préférablement 1 ou 2,
n' étant tel que défini dans la revendication 1 et étant préférablement 1,
R' étant un atome d'hydrogène, un atome d'halogène ou un groupe choisi parmi un groupe (C₁-C₃)alkyle et un groupe (C₁-C₄) alcoxy, 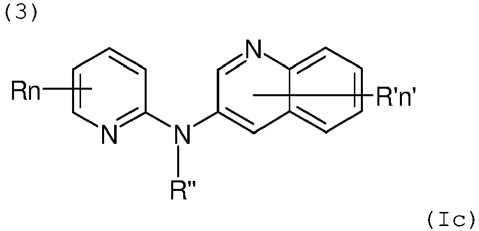
R représentant indépendamment un atome d'hydrogène ou un groupe choisi parmi un groupe (C₁-C₃)alkyle, un groupe (C₁-C₃) fluoroalkyle, un groupe -NR₁R₂, un groupe -COOR₁, un groupe -NO₂ et un groupe (C₁-C₃) alcoxy,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène,
R₁ et R₂ étant indépendamment un atome d'hydrogène ou un groupe (C₁-C₃) alkyle, 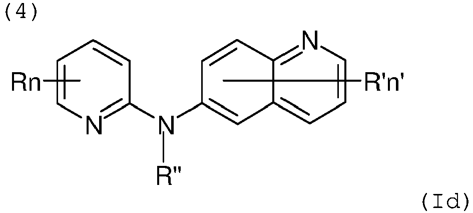
R représentant indépendamment un atome d'hydrogène ou un groupe choisi parmi un groupe (C₁-C₃)alkyle, un groupe (C₁-C₃) fluoroalkyle et un groupe (C₁-C₃) alcoxy,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 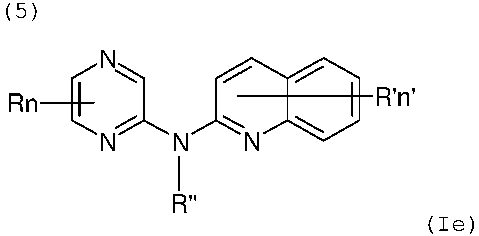
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, un atome d'halogène ou un groupe choisi parmi un groupe (C₁-C₃)alkyle et un groupe (C₁-C₃) alcoxy, 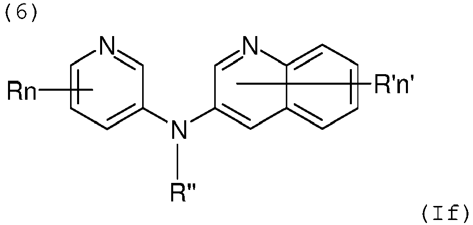
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 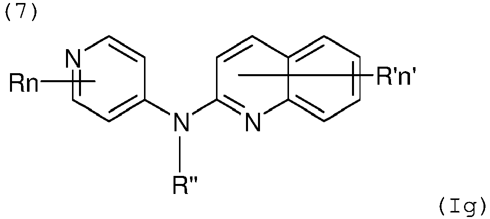
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène ou un atome d'halogène, 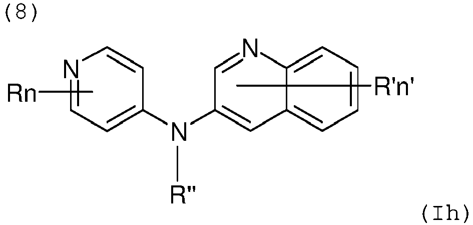
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 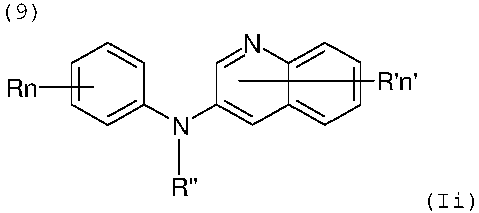
R représentant indépendamment un atome d'hydrogène ou un groupe choisi parmi un groupe (C₁-C₃)fluoroalcoxy et un groupe (C₁-C₃) alcoxy,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 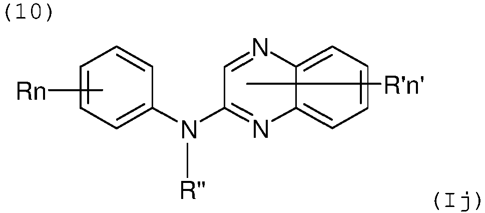
R représentant indépendamment un atome d'hydrogène ou un groupe choisi parmi un groupe (C₁-C₃)fluoroalcoxy et un groupe (C₁-C₃) alkyle,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 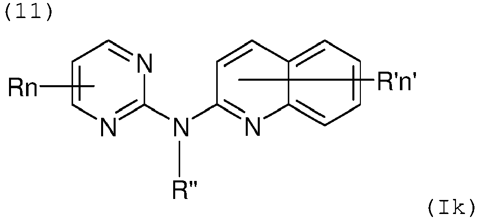
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, un atome d'halogène ou un groupe (C₁-C₃) alkyle, 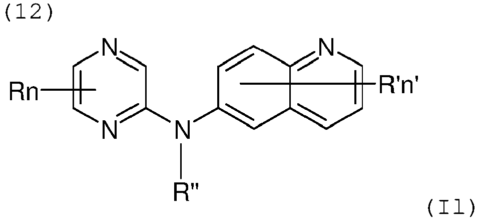
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 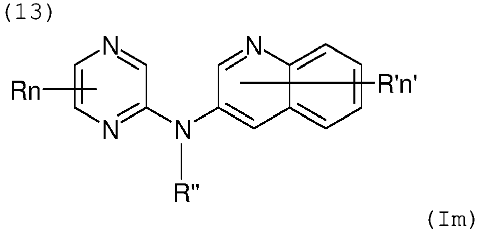
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 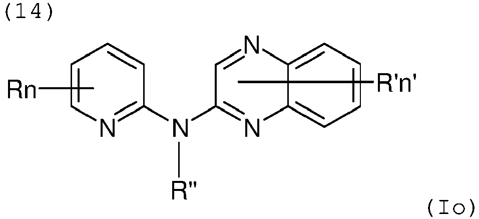
R représentant indépendamment un atome d'hydrogène, un atome d'halogène ou un groupe choisi parmi, un groupe - NO₂, un groupe -CN et un groupe (C₁-C₃) alkyle, ledit alkyle étant éventuellement monosubstitué par un groupe hydroxyle,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, un atome d'halogène ou un groupe (C₁-C₃) fluoroalkyle, 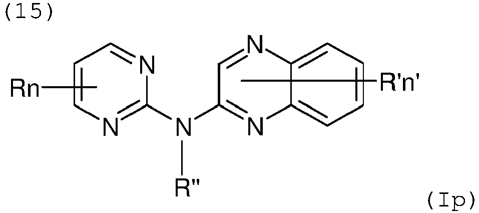
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène, 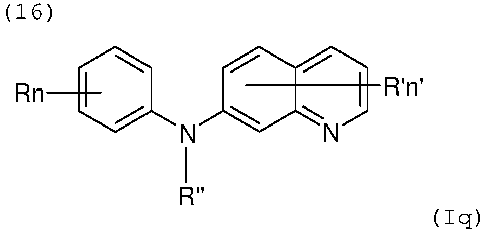
R représentant indépendamment un atome d'hydrogène, un groupe (C₁-C₃)alcoxy ou un groupe (C₁-C₃)fluoroalcoxy,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène ou un groupe choisi parmi un groupe -NR₁R₂, un groupe N-méthylpipérazinyle, un groupe (C₁-C₃)alcoxy et un groupe morpholino,
R₁ et R₂ étant indépendamment un atome d'hydrogène ou un groupe (C₁-C₃) alkyle, 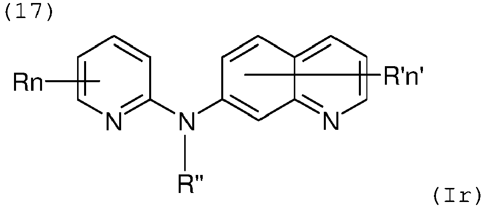
R représentant indépendamment un atome d'hydrogène ou un groupe (C₁-C₃) alkyle,
R" étant tel que défini dans la revendication 1 et étant avantageusement un atome d'hydrogène,
n étant tel que défini dans la revendication 1 et étant avantageusement 1,
n' étant tel que défini dans la revendication 1 et étant avantageusement 1,
R' étant un atome d'hydrogène ou un groupe choisi parmi un groupe -NR₁R₂, un groupe morpholino et un groupe (Ci-C₃) alcoxy,
R₁ et R₂ étant indépendamment un atome d'hydrogène ou un groupe (C₁-C₃) alkyle, (18)
ou l'un de son sel pharmaceutiquement acceptable.

4. Composé de formule (I) selon l'une quelconque des revendications précédentes, pour une utilisation en tant qu'agent pour la prévention, l'inhibition ou le traitement d'affections pathologiques liées au vieillissement prématuré, qui est en variante choisi parmi :
(1) 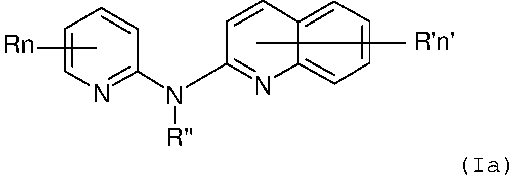
R représentant indépendamment un atome d'hydrogène, un atome d'halogène ou un groupe choisi parmi un groupe (Ci-C₃)alkyle, un groupe -CN, un groupe -COOR₁, un groupe (Ci-C₃)fluoroalkyle, un groupe -NO₂ et un groupe (Ci-C₃) alcoxy,
R₁ étant un atome d'hydrogène ou un groupe (C₁-C₃) alkyle, R" étant tel que défini dans la revendication 1 et plus préférablement étant un atome d'hydrogène,
n étant tel que défini dans la revendication 1 et plus préférablement étant 1,
n' étant tel que défini dans la revendication 1,
R' étant un atome d'hydrogène, un atome d'halogène ou un groupe (C₁-C₃) alkyle,
(2) 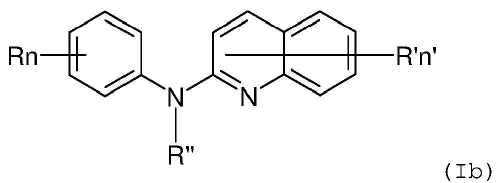
R représentant indépendamment un atome d'hydrogène, un atome d'halogène ou un groupe choisi parmi un groupe (Ci-C₃) alkyle, un groupe (C₁-C₃)fluoroalcoxy et un groupe phénoxy,
R₁ étant un atome d'hydrogène ou un groupe (C₁-C₃) alkyle, R" étant tel que défini dans la revendication 1 et plus préférablement étant un atome d'hydrogène,
n étant tel que défini dans la revendication 1 et plus préférablement étant 1,
n' étant tel que défini dans la revendication 1,
R' étant un atome d'hydrogène,
(3) 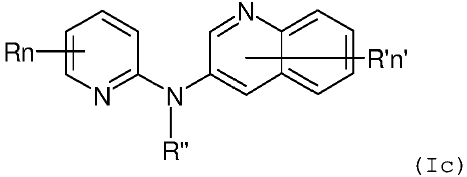
R représentant indépendamment un atome d'hydrogène ou un groupe choisi parmi un groupe (C₁-C₃)alkyle, un groupe - NO₂ et un groupe (C₁-C₃) alcoxy,
R" étant tel que défini dans la revendication 1 et plus préférablement étant un atome d'hydrogène,
n étant tel que défini dans la revendication 1 et plus préférablement étant 1,
n' étant tel que défini dans la revendication 1,
R' étant un atome d'hydrogène,
(4) 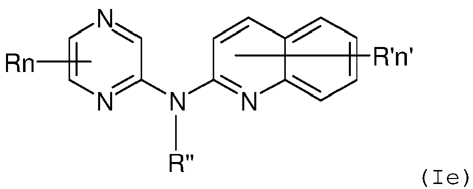
R représentant un atome d'hydrogène,
R" étant tel que défini dans la revendication 1 et étant avantageusement un atome d'hydrogène,
n étant tel que défini dans la revendication 1 et étant avantageusement 1,
n' étant tel que défini dans la revendication 1,
R' étant un atome d'hydrogène ou un atome d'halogène,
(5) 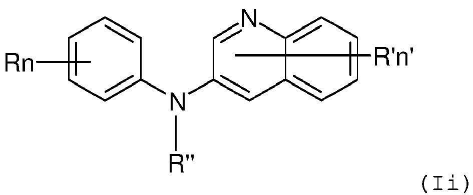
R représentant indépendamment un atome d'hydrogène ou un groupe (C₁-C₃) alcoxy,
R" étant tel que défini ci-dessus et étant avantageusement un atome d'hydrogène,
n étant tel que défini ci-dessus et étant avantageusement 1,
n' étant tel que défini ci-dessus et étant avantageusement 1,
R' étant un atome d'hydrogène,
(6) 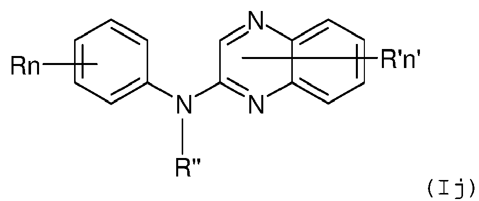
R représentant indépendamment un atome d'hydrogène ou un groupe choisi parmi un groupe (C₁-C₃)fluoroalcoxy et un groupe (C₁-C₃) alkyle,
R" étant tel que défini dans la revendication 1 et plus préférablement étant un atome d'hydrogène,
n étant tel que défini dans la revendication 1 et plus préférablement étant 2,
n' étant tel que défini dans la revendication 1,
R' étant un atome d'hydrogène,
(7) 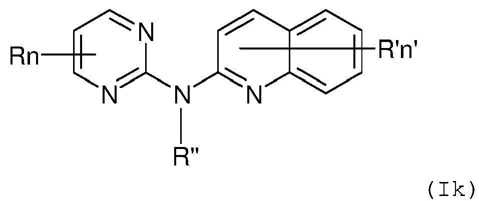
R représentant un atome d'hydrogène,
R" étant tel que défini dans la revendication 1 et plus préférablement étant un atome d'hydrogène,
n étant tel que défini dans la revendication 1 et plus préférablement étant 1,
n' étant tel que défini dans la revendication 1,
R' étant un atome d'hydrogène,
(8) 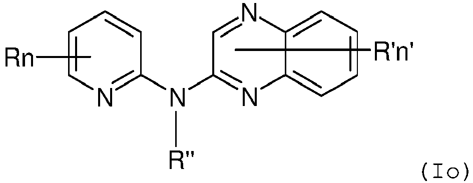
R représentant indépendamment un atome d'hydrogène ou un groupe choisi parmi un groupe (C₁-C₃)alkyle et un groupe -CN,
R" étant tel que défini dans la revendication 1 et plus préférablement étant un atome d'hydrogène,
n étant tel que défini dans la revendication 1 et plus préférablement étant 1,
n' étant tel que défini dans la revendication 1 et plus préférablement étant 1,
R' étant un atome d'hydrogène,
(9)
ou l'un de son sel pharmaceutiquement acceptable.

5. Composé de formule (I) selon l'une quelconque des revendications 1 à 3, qui est en variante choisi parmi,
(1) composé de formule (Ig) tel que défini dans la revendication 3
R représentant indépendamment un atome d'hydrogène, un atome d'halogène ou un groupe choisi parmi un groupe (Ci-C₃)alkyle, un groupe -CN, un groupe hydroxyle, un groupe -COOR₁, un groupe (C₁-C₃)fluoroalkyle, un groupe (Ci-C₃)fluoroalcoxy, un groupe -NO₂, un groupe -NR₁R₂, et un groupe (C₁-C₃) alcoxy,
n étant 1 ou 2,
n' étant 1 ou 2,
R' étant un atome d'hydrogène ou un groupe choisi parmi un groupe (C₁-C₃) alkyle, un atome d'halogène, un groupe hydroxyle, un groupe -COOR₁, un groupe -NO₂, un groupe - NR₁R₂, un groupe (C₁-C₃) alcoxy et un groupe -CN,
R" étant un atome d'hydrogène ou un groupe (C₁-C₄) alkyle, R₁ et R₂ étant indépendamment un atome d'hydrogène ou un groupe (C₁-C₃) alkyle,
à la condition que R et R' ne soient pas simultanément un atome d'hydrogène,
et lorsque n et n' sont 1 et R est un atome d'hydrogène alors R' n'est pas un groupe -COOH,
ou l'un quelconque de son sel pharmaceutiquement acceptable,
(2) composé de formule (Ic) tel que défini dans la revendication 3
R représentant indépendamment un atome d'hydrogène, un atome d'halogène ou un groupe choisi parmi un groupe (Ci-C₃)alkyle, un groupe -CN, un groupe hydroxyle, un groupe -COOR₁, un groupe (C₁-C₃)fluoroalkyle, un groupe -NO₂, un groupe -NR₁R₂ et un groupe (C₁-C₃)alcoxy,
R₁ et R₂ étant indépendamment un atome d'hydrogène ou un groupe (C₁-C₃) alkyle,
n étant 1 ou 2, et avantageusement 1,
n' étant 1 ou 2,
R" étant tel que défini dans la formule (Ic) dans la revendication 3,
R' étant un atome d'hydrogène ou un groupe choisi parmi un groupe (C₁-C₃) alkyle, un groupe -NO₂, un groupe -NR₁R₂ et un groupe (C₁-C₃)alcoxy,
à la condition que
R et R' ne soient pas simultanément un atome d'hydrogène, R ne soit pas un atome de brome lorsque R' est un atome d'hydrogène,
ou l'un de son sel pharmaceutiquement acceptable,
(3) composé de formule (Id) tel que défini dans la revendication 3
R représentant indépendamment un atome d'hydrogène, un atome d'halogène ou un groupe choisi parmi un groupe (Ci-C₃)alkyle, un groupe -CN, un groupe hydroxyle, un groupe -COOR₁, un groupe (C₁-C₃)fluoroalkyle, un groupe (Ci-C₃) fluoroalcoxy, un groupe -NO₂ et un groupe -NR₁R₂,
R₁ et R₂ étant indépendamment un atome d'hydrogène ou un groupe (C₁-C₃) alkyle,
n étant 1 ou 2, et avantageusement 1,
n' étant 1 ou 2,
R" étant tel que défini dans la formule (I) dans la revendication 1 et étant avantageusement un atome d'hydrogène,
R' étant un atome d'hydrogène ou un groupe choisi parmi un groupe (C₁-C₃)alkyle, un groupe -NO₂, un groupe -NR₁R₂ et un groupe (C₁-C₃)alcoxy,
à la condition que lorsque R' est un atome d'hydrogène, R est différent d'un groupe -NO₂, d'un groupe -NH₂ ou d'un groupe -COOH,
ou l'un de son sel pharmaceutiquement acceptable,
(4) composé de formule (Ii') 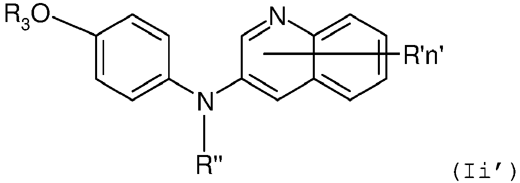
R₃ étant un groupe (C₁-C₃) fluoroalkyle ou un groupe (Ci-C₃)alkyle,
R' étant un atome d'hydrogène, un atome d'halogène ou un groupe choisi parmi un groupe (C₁-C₃) alkyle, un groupe hydroxyle, un groupe -COOR₁, un groupe -NO₂, un groupe - NR₁R₂, un groupe (C₁-C₃) alcoxy et un groupe -CN,
R₁ et R₂ étant indépendamment un atome d'hydrogène ou un groupe (C₁-C₃) alkyle,
R" étant tel que défini dans la revendication 1 et étant avantageusement un atome d'hydrogène,
n' étant tel que défini dans la revendication 1 et étant avantageusement 1,
à la condition que
lorsque R' est un atome d'hydrogène, R₃ n'est pas un groupe méthyle ou un groupe trifluorométhyle ou l'un de son sel pharmaceutiquement acceptable.

6. Composé de formule (I) selon l'une quelconque des revendications 1 à 3, qui est en variante choisi parmi,
(1) composé de formule (Ik) tel que défini dans la revendication 3
R représentant indépendamment un atome d'hydrogène, un atome d'halogène ou un groupe choisi parmi un groupe (Ci-C₃)alkyle, un groupe -CN, un groupe hydroxyle, un groupe -COOR₁, un groupe (C₁-C₃)fluoroalkyle, un groupe (C₁-C₃)fluoroalcoxy, un groupe -NO₂, un groupe -NR₁R₂ et un (C₁-C₃) alcoxy,
R₁ et R₂ étant indépendamment un atome d'hydrogène ou un groupe (C₁-C₃) alkyle,
n étant 1 ou 2, et étant avantageusement 1,
n' étant 1 ou 2,
R" étant tel que défini dans la formule (Ik) dans la revendication 3,
R' étant un atome d'hydrogène, un atome d'halogène ou un groupe choisi parmi un groupe (C₁-C₃)alkyle, un groupe hydroxyle, un groupe -COOR₁, un groupe -NO₂, un groupe - NR₁R₂, un groupe (C₁-C₃)alcoxy et un groupe -CN,
ou l'un de son sel pharmaceutiquement acceptable,
(2) composé de formule (Io) tel que défini dans la revendication 3
R représentant indépendamment un atome d'hydrogène, un atome d'halogène ou un groupe choisi parmi un groupe (Ci-C₃)alkyle, un groupe -CN, un groupe hydroxyle, un groupe -COOR₁, un groupe (C₁-C₃)fluoroalkyle, un groupe (C₁-C₃)fluoroalcoxy, un groupe -NO₂, un groupe -NR₁R₂ et un groupe (C₁-C₃) alcoxy,
R₁ et R₂ étant indépendamment un atome d'hydrogène ou un groupe (C₁-C₃) alkyle,
n étant 1, 2 ou 3,
n' étant 1 ou 2,
R' étant un atome d'hydrogène, un atome d'halogène ou un groupe choisi parmi un groupe (C₁-C₃)alkyle, un groupe hydroxyle, un groupe -COOR₁, un groupe -NO₂, un groupe - NR₁R₂, un groupe (C₁-C₃) alcoxy et un groupe -CN,
R" étant un atome d'hydrogène ou un groupe (C₁-C₄)alkyle, à la condition que
lorsque R est un atome d'hydrogène et n' est 1, R' n'est pas un groupe hydroxyle,
ou l'un de son sel pharmaceutiquement acceptable,
(3) composé de formule (Ij') 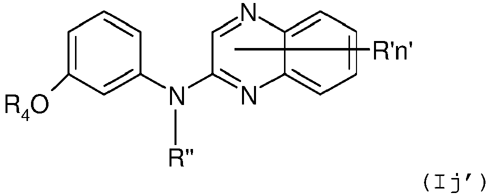
R₄ étant un groupe (C₁-C₃) fluoroalkyle ou un groupe (C₁-C₃)alkyle,
R' étant un atome d'hydrogène, un atome d'halogène ou un groupe choisi parmi un groupe (C₁-C₃) alkyle, un groupe hydroxyle, un groupe -COOR₁, un groupe -NO₂, un groupe - NR₁R₂, un groupe (C₁-C₃) alcoxy et un groupe -CN,
R₁ et R₂ étant indépendamment un atome d'hydrogène ou un groupe (C₁-C₃)alkyle,
R" étant tel que défini dans la revendication 1 et étant avantageusement un atome d'hydrogène,
n' étant tel que défini dans la revendication 1 et étant avantageusement 1,
à la condition que
lorsque R' est un atome d'hydrogène, R₄ n'est pas un groupe méthyle
ou l'un de son sel pharmaceutiquement acceptable,
(4) composé de formule (Ij") 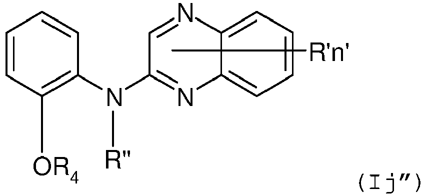
R₄ étant un groupe (C₁-C₃) fluoroalkyle ou un groupe (Ci-C₃)alkyle,
R' étant un atome d'hydrogène, un atome d'halogène ou un groupe choisi parmi un groupe (C₁-C₃) alkyle, un groupe hydroxyle, un groupe -COOR₁, un groupe -NO₂, un groupe - NR₁R₂, un groupe (C₁-C₃) alcoxy et un groupe -CN,
R₁ et R₂ étant indépendamment un atome d'hydrogène ou un groupe (C₁-C₃) alkyle,
R" étant tel que défini dans la revendication 1 et étant avantageusement un atome d'hydrogène,
n' étant tel que défini dans la revendication 1 et étant avantageusement 1,
ou l'un de son sel pharmaceutiquement acceptable,
(5) composé de formule (Ij'") 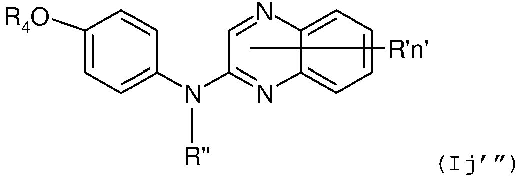
R₄ étant un groupe (C₁-C₃) fluoroalkyle ou un groupe (Ci-C₃)alkyle,
R' étant un atome d'hydrogène, un atome d'halogène ou un groupe choisi parmi un groupe (C₁-C₃) alkyle, un groupe hydroxyle, un groupe -NO₂, un groupe -NR₁R₂, un groupe (C₁-C₃) alcoxy et un groupe -CN,
R₁ et R₂ étant indépendamment un atome d'hydrogène ou un groupe (C₁-C₃) alkyle,
R" étant tel que défini dans la revendication 1 et étant avantageusement un atome d'hydrogène,
n' étant tel que défini dans la revendication 1 et étant avantageusement 1,
à la condition que
lorsque R' est un atome de chlore ou un atome d'hydrogène, R₄ n'est pas un groupe éthyle ou un groupe méthyle,
lorsque R' est un groupe méthyle ou un groupe tertiobutyle, R₄ n'est pas un groupe méthyle,
ou l'un de son sel pharmaceutiquement acceptable.

7. Composé de formule (I) selon l'une quelconque des revendications 1 à 3, qui est en variante choisi parmi,
(1) composé de formule (Ip) tel que défini dans la revendication 3
R représentant indépendamment un atome d'hydrogène, un atome d'halogène ou un groupe choisi parmi un groupe (C₁-C₃)alkyle, un groupe -CN, un groupe hydroxyle, un groupe -COOR₁, un groupe (C₁-C₃)fluoroalkyle, un groupe (C₁-C₃)fluoroalcoxy, un groupe -NO₂, un groupe -NR₁R₂ et un groupe (C₁-C₃) alcoxy,
R₁ et R₂ étant indépendamment un atome d'hydrogène ou un groupe (C₁-C₃) alkyle,
n étant 1 ou 2, et avantageusement 1,
n' étant 1 ou 2,
R' étant un atome d'hydrogène, un atome d'halogène ou un groupe choisi parmi un groupe (C₁-C₃)alkyle, un groupe hydroxyle, un groupe -COOR₁, un groupe -NO₂, un groupe - NR₁R₂, un groupe (C₁-C₃) alcoxy et un groupe -CN,
R" étant un atome d'hydrogène ou un groupe (C₁-C₄)alkyle, à la condition que
R et R' ne soient pas simultanément un atome d'hydrogène, lorsque n et n' sont 2 alors R et R' ne sont pas simultanément un groupe méthyle,
ou l'un de son sel pharmaceutiquement acceptable,
(2) composé de formule (Ir) tel que défini dans la revendication 3
R représentant indépendamment un atome d'hydrogène, un atome d'halogène ou un groupe choisi parmi un groupe (C₁-C₃)alkyle, un groupe -CN, un groupe hydroxyle, un groupe -COOR₁, un groupe (C₁-C₃)fluoroalkyle, un groupe (C₁-C₃)fluoroalcoxy, un groupe -NO₂, un groupe -NR₁R₂ et un groupe (C₁-C₃) alcoxy,
R₁ et R₂ étant indépendamment un atome d'hydrogène ou un groupe (C₁-C₃) alkyle,
n étant 1 ou 2, et avantageusement 1,
n' étant 1 ou 2,
R' étant un atome d'hydrogène, un atome d'halogène ou un groupe choisi parmi un groupe (C₁-C₃)alkyle, un groupe hydroxyle, un groupe -COOR₁, un groupe -NO₂, un groupe - NR₁R₂, un groupe (C₁-C₃) alcoxy et un groupe -CN,
R" étant un atome d'hydrogène ou un groupe (C₁-C₄)alkyle, ou l'un de son sel pharmaceutiquement acceptable.

8. Composé pour une utilisation selon l'une quelconque des revendications 1 à 4, ledit composé étant choisi parmi :
- (1) (8-Chloro-quinoléin-2-yl)-pyridin-2-yl-amine
- (2) Acide 2-(quinoléin-2-ylamino)-isonicotinique
- (3) (4-Méthyl-pyridin-2-yl)-quinoléin-2-yl-amine
- (4) Pyridin-2-yl-quinoléin-2-yl-amine
- (5) Acide 2-(8-chloro-quinoléin-2-ylamino)-isonicotinique
- (6) (8-Chloro-quinoléin-2-yl)-(4-méthyl-pyridin-2-yl)-amine
- (7) 6-(Quinoléin-2-ylamino)-nicotinonitrile
- (8) Quinoléin-2-yl-(4-trifluorométhoxy-phényl)-amine
- (9) Pyridin-2-yl-quinoléin-3-yl-amine
- (10) (3-Méthoxy-pyridin-2-yl)-quinoléin-3-yl-amine
- (11) Quinoléin-3-yl-(5-trifluorométhyl-pyridin-2-yl)-amine
- (12) (5-Nitro-pyridin-2-yl)-quinoléin-3-yl-amine
- (13) (5-Méthyl-pyridin-2-yl)-quinoléin-3-yl-amine
- (14) Acide 2-(quinoléin-3-ylamino)-isonicotinique
- (15) Quinoléin-6-yl-(5-trifluorométhyl-pyridin-2-yl)-amine
- (16) (6-Méthyl-pyridin-2-yl)-quinoléin-6-yl-amine
- (17) N-(6-méthylpyridin-2-yl)quinoléin-2-amine
- (18) 8-chloro-N-(6-méthylpyridin-2-yl)quinoléin-2-amine
- (19) 4-méthyl-N-(pyridin-2-yl)quinoléin-2-amine
- (20) 4-méthyl-N-(4-méthylpyridin-2-yl)quinoléin-2-amine
- (21) 3-méthyl-N-(4-méthylpyridin-2-yl)quinoléin-2-amine
- (22) 3-méthyl-N-(pyridin-2-yl)quinoléin-2-amine
- (23) 6-((4-méthylquinoléin-2-yl)amino)nicotinonitrile
- (24) 6-((3-méthylquinoléin-2-yl)amino)nicotinonitrile
- (25) 6-chloro-N-(4-méthylpyridin-2-yl)quinoléin-2-amine
- (26) 6-chloro-N-(6-méthylpyridin-2-yl)quinoléin-2-amine
- (27) 4-méthyl-N-(5-nitropyridin-2-yl)quinoléin-2-amine
- (28) N-(3-nitropyridin-2-yl)quinoléin-2-amine
- (29) 8-chloro-N-(3-nitropyridin-2-yl)quinoléin-2-amine
- (30) 2-((4-méthylquinoléin-2-yl)amino)nicotinonitrile
- (31) N-(3-méthylpyridin-2-yl)quinoléin-2-amine
- (32) N-(5-méthylpyridin-2-yl)quinoléin-2-amine
- (33) 2-(quinoléin-2-ylamino)isonicotinonitrile
- (34) N-(5-(trifluorométhyl)pyridin-2-yl)quinoléin-2-amine
- (35) 8-chloro-N-(3-méthylpyridin-2-yl)quinoléin-2-amine
- (36) 8-chloro-N-(5-méthylpyridin-2-yl)quinoléin-2-amine
- (37) 8-chloro-N-(5-(trifluorométhyl)pyridin-2-yl)quinoléin-2-amine
- (38) N-(3-méthoxypyridin-2-yl)quinoléin-2-amine
- (39) N-(5-nitropyridin-2-yl)quinoléin-2-amine
- (40) 6-((8-chloroquinoléin-2-yl)amino)nicotinonitrile
- (41) N-(5-fluoropyridin-2-yl)quinoléin-2-amine
- (42) N-(6-(trifluorométhyl)pyridin-2-yl)quinoléin-2-amine
- (43) 8-chloro-N-(5-fluoropyridin-2-yl)quinoléin-2-amine
- (44) acide 2-((8-chloroquinoléin-2-yl)amino)nicotinique
- (45) 4-méthyl-N-(6-méthylpyridin-2-yl)quinoléin-2-amine
- (46) 3-méthyl-N-(6-méthylpyridin-2-yl)quinoléin-2-amine
- (47) chlorure de 5-cyano-2-(quinoléin-2-ylamino)pyridin-1-ium
- (48) chlorure de 2-((8-chloroquinoléin-2-yl)amino)-4-méthylpyridin-1-ium
- (49) 8-chloro-N-(4-éthylpyridin-2-yl)quinoléin-2-amine
- (50) 8-chloro-N-(6-éthylpyridin-2-yl)quinoléin-2-amine
- (51) 8-chloro-N-(4,6-diméthylpyridin-2-yl)quinoléin-2-amine
- (52) 6-((8-chloroquinoléin-2-yl)amino)-2-méthylnicotinonitrile
- (53) 8-chloro-N-(4-chloropyridin-2-yl)quinoléin-2-amine
- (54) 8-méthyl-N-(4-méthylpyridin-2-yl)quinoléin-2-amine
- (55) N-(5-bromo-4-méthylpyridin-2-yl)-8-chloroquinoléin-2-amine
- (56) 8-chloro-N-(3-éthyl-6-méthylpyridin-2-yl)quinoléin-2-amine
- (57) 8-fluoro-N-(4-méthylpyridin-2-yl)quinoléin-2-amine
- (58) 8-bromo-N-(4-méthylpyridin-2-yl)quinoléin-2-amine
- (59) 6-(quinoléin-2-ylamino)nicotinate de méthyle
- (60) 6-[(8-chloroquinoléin-2-yl)amino]pyridine-3-carboxylate de méthyle
- (61) 6-[(3-méthylquinoléin-2-yl)amino]pyridine-3-carboxylate de méthyle
- (62) 2-[(8-chloroquinoléin-2-yl)amino]pyridine-3-carboxylate de méthyle
- (63) 8-méthoxy-N-(4-méthylpyridin-2-yl)quinoléin-2-amine
- (64) N-(4-méthylpyridin-2-yl)-5-nitroquinoléin-2-amine
- (65) 2-N-(4-méthylpyridin-2-yl)quinoléine-2,8-diamine
- (66) N-(4-méthylpyridin-2-yl)-5-aminoquinoléin-2-amine
- (67) 6-[(4-méthylquinoléin-2-yl)amino]pyridine-3-carboxylate de méthyle
- (68) 8-chloro-N-[4-(trifluorométhyl)pyridin-2-yl]quinoléin-2-amine
- (69) 2-[(8-chloroquinoléin-2-yl)amino]pyridin-3-ol
- (70) 8-chloro-N-[6-(trifluorométhyl)pyridin-2-yl]quinoléin-2-amine
- (71) 6-chloro-N-(5-fluoropyridin-2-yl)quinoléin-2-amine
- (72) N-(6-éthylpyridin-2-yl)-3-méthylquinoléin-2-amine
- (73) N-(5-fluoropyridin-2-yl)-3-méthylquinoléin-2-amine
- (74) 3-méthyl-N-[5-(trifluorométhyl)pyridin-2-yl]quinoléin-2-amine
- (75) 4-N-(8-chloroquinoléin-2-yl)-1-N, 1-N-diméthylbenzène-1,4-diamine
- (76) N-(4-méthoxyphényl)quinoléin-2-amine
- (77) 8-chloro-N-(4-méthoxyphényl)quinoléin-2-amine
- (78) 4-méthyl-N-[4-(trifluorométhoxy)phényl]quinoléin-2-amine
- (79) N-(4-méthoxyphényl)-3-méthylquinoléin-2-amine
- (80) 3-méthyl-N-[4-(trifluorométhoxy)phényl]quinoléin-2-amine
- (81) 1-N,1-N-diméthyl-4-N-(3-méthylquinoléin-2-yl)benzène-1,4-diamine
- (82) N-[2-méthyl-4-(trifluorométhoxy)phényl]quinoléin-2-amine
- (83) N-[3-(trifluorométhoxy)phényl]quinoléin-2-amine
- (84) N-[2-(trifluorométhoxy)phényl]quinoléin-2-amine
- (85) N-(4-nitrophényl)quinoléin-2-amine
- (86) N-(3-fluorophényl)quinoléin-2-amine
- (87) 8-chloro-N-[3-(trifluorométhoxy)phényl]quinoléin-2-amine
- (88) 8-chloro-N-(3-fluorophényl)quinoléin-2-amine
- (89) chlorure de 2-{[4-(trifluorométhoxy)phényl]amino}quinoléin-1-ium
- (90) 8-chloro-N-[4-(trifluorométhoxy)phényl]quinoléin-2-amine
- (91) 3-méthyl-N-[2-méthyl-4-(trifluorométhoxy)phényl]quinoléin-2-amine
- (92) 3-méthyl-N-[3-(trifluorométhoxy)phényl]quinoléin-2-amine
- (93) 3-méthyl-N-[2-(trifluorométhoxy)phényl]quinoléin-2-amine
- (94) 8-chloro-N-[2-méthyl-4-(trifluorométhoxy)phényl]quinoléin-2-amine
- (95) chlorure de 3-méthyl-2-{[4-(trifluorométhoxy)phényl]amino}quinoléin-1-ium
- (96) 6-chloro-N-(4-(trifluorométhoxy)phényl)quinoléin-2-amine
- (97) chlorure de 4-méthyl-2-{[4-(trifluorométhoxy)phényl]amino}quinoléin-1-ium
- (98) 8-bromo-N-[4-(trifluorométhoxy)phényl]quinoléin-2-amine
- (99) 8-fluoro-N-[4-(trifluorométhoxy)phényl]quinoléin-2-amine
- (100) 8-méthyl-N-[4-(trifluorométhoxy)phényl]quinoléin-2-amine
- (101) N-(4-butoxyphényl)-8-chloroquinoléin-2-amine
- (102) N-(4-phénoxyphényl)quinoléin-2-amine
- (103) 8-méthoxy-N-[4-(trifluorométhoxy)phényl]quinoléin-2-amine
- (104) 8-chloro-N-[3-chloro-4-(trifluorométhoxy)phényl]quinoléin-2-amine
- (105) N-(6-méthylpyridin-2-yl)quinoléin-3-amine
- (106) N-(3-nitropyridin-2-yl)quinoléin-3-amine
- (107) N-(5-méthylpyridin-2-yl)quinoléin-6-amine
- (108) N-(3-méthoxypyridin-2-yl)quinoléin-6-amine
- (109) 6-chloro-N-(pyrazin-2-yl)quinoléin-2-amine
- (110) 8-bromo-N-(pyrazin-2-yl)quinoléin-2-amine
- (111) 8-méthyl-N-(pyrazin-2-yl)quinoléin-2-amine
- (112) 8-chloro-N-(pyrazin-2-yl)quinoléin-2-amine
- (113) N-(pyrazin-2-yl)quinoléin-2-amine
- (114) 4-méthyl-N-(pyrazin-2-yl)quinoléin-2-amine
- (115) 3-méthyl-N-(pyrazin-2-yl)quinoléin-2-amine
- (116) 8-fluoro-N-(pyrazin-2-yl)quinoléin-2-amine
- (117) 8-méthoxy-N-(pyrazin-2-yl)quinoléin-2-amine
- (118) N-(pyridin-3-yl)quinoléin-3-amine
- (119) 8-chloro-N-(pyridin-4-yl)quinoléin-2-amine
- (120) N-(pyridin-4-yl)quinoléin-2-amine
- (121) N-(pyridin-4-yl)quinoléin-3-amine
- (122) N-[4-(trifluorométhoxy)phényl]quinoléin-3-amine
- (123) N-(4-méthoxyphényl)quinoléin-3-amine
- (124) N-[4-(trifluorométhoxy)phényl]quinoxalin-2-amine
- (125) N-[2-méthyl-4-(trifluorométhoxy)phényl]quinoxalin-2-amine
- (126) N-[3-(trifluorométhoxy)phényl]quinoxalin-2-amine
- (127) N-[2-(trifluorométhoxy)phényl]quinoxalin-2-amine
- (128) N-(pyrimidin-2-yl)quinoléin-2-amine
- (129) 8-chloro-N-(pyrimidin-2-yl)quinoléin-2-amine
- (130) 4-méthyl-N-(pyrimidin-2-yl)quinoléin-2-amine
- (131) N-(pyrazin-2-yl)quinoléin-6-amine
- (132) N-(pyrazin-2-yl)quinoléin-3-amine
- (133) 6-méthyl-N-(naphtalen-2-yl)pyridin-2-amine
- (134) N-(naphtalen-2-yl)pyridin-2-amine
- (135) N-(pyridin-2-yl)quinoxalin-2-amine
- (136) N-(4-méthylpyridin-2-yl)quinoxalin-2-amine
- (137) 6-(quinoxalin-2-ylamino)pyridine-3-carbonitrile
- (138) N-(6-méthylpyridin-2-yl)quinoxalin-2-amine
- (139) N-(4-méthylpyridin-2-yl)-3-(trifluorométhyl)quinoxalin-2-amine
- (140) N-(3,5-dichloro-4-méthylpyridin-2-yl)quinoxalin-2-amine
- (141) N-(4-méthyl-3-nitropyridin-2-yl)quinoxalin-2-amine
- (142) N-(pyrimidin-2-yl)quinoxalin-2-amine
- (143) 4-N,4-N-diméthyl-7-N-[4-(trifluorométhoxy)phényl]quinoléine-4,7-diamine
- (144) 4-(morpholin-4-yl)-N-[4-(trifluorométhoxy)phényl]quinoléin-7-amine
- (145) 4-méthoxy-N-(pyridin-2-yl)quinoléin-7-amine
- (146) 4-méthoxy-N-(4-méthylpyridin-2-yl)quinoléin-7-amine
- (147) 4-N,4-N-diméthyl-7-N-(4-méthylpyridin-2-yl)quinoléine-4,7-diamine
- (150) N-(4-méthylpyridin-2-yl)-8-nitroquinoléin-2-amine
- (151) 6-chloro-N-(6-éthylpyridin-2-yl)quinoléin-2-amine
- (152) 6-chloro-N-(5-méthylpyridin-2-yl)quinoléin-2-amine
- (153) 6-chloro-N-[5-(trifluorométhyl)pyridin-2-yl]quinoléin-2-amine
- (154) N2-(8-chloroquinoléin-2-yl)-4-méthylpyridine-2,3-diamine
- (155) N-(4-butoxyphényl)-3-méthylquinoléin-2-amine
- (156) 4-N-(6-chloroquinoléin-2-yl)-1-N,1-N-diméthylbenzène-1,4-diamine
- (157) 8-chloro-N-(3-chloro-4-méthoxyphényl)quinoléin-2-amine
- (158) N1-(8-chloroquinoléin-2-yl)-4-(trifluorométhoxy)benzène-1,2-diamine
- (159) N-(3-aminopyridin-2-yl)quinoléin-3-amine
- (160) 6-chloro-N-(4-méthylpyridin-2-yl)quinoxalin-2-amine
- (161) N-(4-éthylpyridin-2-yl)quinoxalin-2-amine
- (162) N-(5-bromo-4-méthylpyridin-2-yl)quinoxalin-2-amine
- (163) N-(4,6-diméthylpyridin-2-yl)quinoxalin-2-amine
- (164) [2-(quinoxalin-2-ylamino)pyridin-4-yl]méthanol
- (165) N-(4-méthyl-5-nitropyridin-2-yl)quinoxalin-2-amine
- (166) N-(4-méthoxyphényl)-4-(4-méthylpipérazin-1-yl)quinoléin-7-amine
- (167) 4-méthoxy-N-[4-(trifluorométhoxy)phényl]quinoléin-7-amine
- (168) N-(4-méthylpyridin-2-yl)-4-(morpholin-4-yl)quinoléin-7-amine
- et leurs sels pharmaceutiquement acceptables.

9. Composé selon l'une quelconque des revendications 5, 6, et 7, ledit composé étant choisi parmi les composés :
- (10) (3-Méthoxy-pyridin-2-yl)-quinoléin-3-yl-amine
- (11) Quinoléin-3-yl-(5-trifluorométhyl-pyridin-2-yl)-amine
- (12) (5-Nitro-pyridin-2-yl)-quinoléin-3-yl-amine
- (13) (5-Méthyl-pyridin-2-yl)-quinoléin-3-yl-amine
- (14) Acide 2-(quinoléin-3-ylamino)-isonicotinique
- (15) Quinoléin-6-yl-(5-trifluorométhyl-pyridin-2-yl)-amine
- (16) (6-Méthyl-pyridin-2-yl)-quinoléin-6-yl-amine
- (105) N-(6-méthylpyridin-2-yl)quinoléin-3-amine
- (106) N-(3-nitropyridin-2-yl)quinoléin-3-amine
- (107) N-(5-méthylpyridin-2-yl)quinoléin-6-amine
- (108) N-(3-méthoxypyridin-2-yl)quinoléin-6-amine
- (119) 8-chloro-N-(pyridin-4-yl)quinoléin-2-amine
- (124) N-[4-(trifluorométhoxy)phényl]quinoxalin-2-amine
- (125) N-[2-méthyl-4-(trifluorométhoxy)phényl]quinoxalin-2-amine
- (126) N-[3-(trifluorométhoxy)phényl]quinoxalin-2-amine
- (127) N-[2-(trifluorométhoxy)phényl]quinoxalin-2-amine
- (128) N-(pyrimidin-2-yl)quinoléin-2-amine
- (129) 8-chloro-N-(pyrimidin-2-yl)quinoléin-2-amine
- (130) 4-méthyl-N-(pyrimidin-2-yl)quinoléin-2-amine
- (135) N-(pyridin-2-yl)quinoxalin-2-amine
- (136) N-(4-méthylpyridin-2-yl)quinoxalin-2-amine
- (137) 6-(quinoxalin-2-ylamino)pyridine-3-carbonitrile
- (138) N-(6-méthylpyridin-2-yl)quinoxalin-2-amine
- (139) N-(4-méthylpyridin-2-yl)-3-(trifluorométhyl)quinoxalin-2-amine
- (140) N-(3,5-dichloro-4-méthylpyridin-2-yl)quinoxalin-2-amine
- (141) N-(4-méthyl-3-nitropyridin-2-yl)quinoxalin-2-amine
- (145) 4-méthoxy-N-(pyridin-2-yl)quinoléin-7-amine
- (146) 4-méthoxy-N-(4-méthylpyridin-2-yl)quinoléin-7-amine
- (147) 4-N,4-N-diméthyl-7-N-(4-méthylpyridin-2-yl)quinoléin-4,7-diamine
- (159) N-(3-aminopyridin-2-yl)quinoléin-3-amine
- (160) 6-chloro-N-(4-méthylpyridin-2-yl)quinoxalin-2-amine
- (161) N-(4-éthylpyridin-2-yl)quinoxalin-2-amine
- (162) N-(5-bromo-4-méthylpyridin-2-yl)quinoxalin-2-amine
- (163) N-(4,6-diméthylpyridin-2-yl)quinoxalin-2-amine
- (164) [2-(quinoxalin-2-ylamino)pyridin-4-yl]méthanol
- (165) N-(4-méthyl-5-nitropyridin-2-yl)quinoxalin-2-amine, et
- (168) N-(4-méthylpyridin-2-yl)-4-(morpholin-4-yl)quinoléin-7-amine
ainsi que leurs sels pharmaceutiquement acceptables, tels que chlorhydrate, bromhydrate, tartrate, fumarate, citrate, trifluoroacétate, ascorbate, triflate, mésylate, tosylate, formiate, acétate et malate.

10. Composition pharmaceutique comprenant au moins un composé tel que défini dans l'une quelconque des revendications 5, 6, 7 et 9.
